# EUROPEAN PATENT APPLICATION

(11) **EP 4 385 989 A1**
(43) Date of publication of application: **19.06.2024**
(21) Application number: 22855464.8
(22) Date of filing: 10.08.2022
(51) Int. Cl.: C07D 413/00, C07D 413/14, A61K 31/42, A61P 35/00

(54) **SULFONAMIDE DERIVATIVE, PREPARATION METHOD THEREFOR AND MEDICAL USE THEREOF**

(30) Priority: 10.08.2021 CN 202110913249; 28.09.2021 CN 202111142090; 09.12.2021 CN 202111500231
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: ZHANG, Xiaomin, Shanghai 200245 (CN); HU, Weimin, Shanghai 200245 (CN); HE, Feng, Shanghai 200245 (CN); TAO, Weikang, Shanghai 200245 (CN)
(74) Representative: Dragotti & Associati S.R.L.
(86) International application number: PCT/CN2022/111395
(87) International publication number: WO 2023/016484

(57) **Abstract**

Provided is a sulfonamide derivate represented by the general formula (I), a preparation method thereof, a pharmaceutical composition containing said derivative, and the use thereof as a therapeutic agent, especially as a Lysine Acetyl Transferase (KAT) inhibitor and the use of same in the preparation of a medicament for the treatment and/or prevention of cancer.

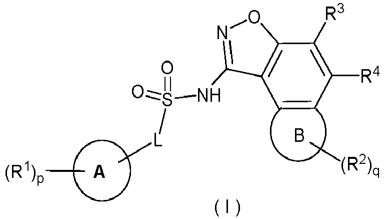

## Description

### TECHNICAL FIELD

The present disclosure pertains to the field of pharmaceutics and relates to a sulfamide derivative, a preparation method therefor, and pharmaceutical use thereof. In particular, the present disclosure relates to a sulfamide derivative represented by general formula (I), a preparation method therefor, a pharmaceutical composition comprising the derivative, and use thereof as a KAT inhibitor in the preparation of a medicament for treating and/or preventing cancer.

### BACKGROUND

Lysine acetyltransferases (KATs) are a class of enzymes that catalyze the transfer of acetyl groups from acetyl-CoA to lysine ε-amino groups of protein substrates. Lysine acetylation affects protein function and thus plays an important regulatory role in chromosome structures, gene transcription regulation, DNA-binding capacity, enzyme activity and stability, protein interactions, and intracellular localization. KATs are divided into several subfamilies, the biggest of which is MYST (MOZ, YBF2/SAS3, SAS2, TIP60), including KAT5 (TIP60), KAT6A (MOZ; MYST3), KAT6B (MORF; MYST4), KAT7 (HBO; MYST2), and KAT8 (MOF; MYST1). KAT6A/B, as a major member of the MYST family, plays a crucial role in development, stem cell maintenance in the hematopoietic and immune systems, tumor development and progression, and drug resistance.

TCGA database analysis shows that KAT6A and KAT6B are amplified in a variety of tumors. KAT6A is located in the chromosome 8p11-p12 amplicon region and is amplified in 10-15% of breast cancer cases. Its copy number is positively correlated with mRNA expression and is correlated with poor prognoses. KAT6A and KAT6B are both significantly highly expressed in breast cancer. Further subtype analysis shows that there is a certain correlation between the high KAT6A/B expression and the ERα expression level, which reveals that KAT6A/B may be a potential target for ER⁺/HER2⁻ breast cancer.

There is a report that when KAT6A was knocked down in the luminal breast cancer cell SUM-52, in which KAT6A is amplified, clonogenesis was significantly inhibited as compared to the non-tumorigenic cell MCF10A. RNAseq analysis shows that the expression of some genes was down-regulated following KAT6A knockdown, including ESR1 and hormone stress pathway-related genes. Further research shows that knockdown of KAT6A inhibits clonogenesis in the ER⁺ breast cancer cell lines T47D and CAMA1, which highly express KAT6A; however, this effect cannot be observed in the cell lines MCF7 and SKBR3, which lowly express KAT6A. Knockdown of KAT6A in T47D and CAMA1 down-regulates ERα expression, and overexpression of wild-type KAT6A in MCF7 and LY2 up-regulates ERα expression. However, these effects cannot be observed in mutants that lack KAT activity, which reveals the importance of KAT function. Overexpression of ERα in T47D reverses the inhibition of clonogenesis by KAT6A knockdown, which indicates that KAT6A function may be mediated by regulation of ERα expression. In line with this, KAT6A enrichment was found in the promoter region of the ESR1 gene. The inhibition of tumors and down-regulation of ERα by KAT6A knockdown were also observed in *in-vivo* efficacy experiments using T47D models. In T47D, knockdown of either KAT6A or KAT6B down-regulates ERα expression and inhibits clonogenesis, and KAT6A knockdown is more effective than KAT6B knockdown. If both are knocked down, the effect will be more significant, which indicates an additive effect. The KAT6A/B selective inhibitor CTx-648 shows anti-tumor activity in ER+ breast cancer both *in vitro* and *in vivo*, and there is a certain correlation between the KAT6A expression level and the sensitivity of CTx-648. In ER⁺ breast cancer cells highly expressing KAT6A, CTx-648 can down-regulate ERα expression, and H3K23Ac can be used as a pharmacodynamic biomarker for KAT6 inhibitors. In conclusion, it is of clinical development value to use KAT6A/B inhibitors as monotherapies or in combination with existing therapies for ER⁺/HER2⁻ breast cancer such as fulvestrant or CDK4/6 inhibitors, or even SERD or SERCA.

In addition to ER⁺/HER2⁻ breast cancer, KAT6A/B inhibitors also have the potential to be applied to the treatment of cerebral glioma, B cell lymphoma, liver cancer, ovarian cancer, etc., as an expansion of the indications for them.

Patent applications that disclose KAT6 inhibitors include WO2016198507A1, WO2019243491A1, WO2019043139A1, WO2019108824A1, WO2020216701A1, WO2020002587A1, WO2020254946A1, WO2020254989A1, etc.

### SUMMARY

The present disclosure aims to provide a compound represented by general formula (I) or a pharmaceutically acceptable salt thereof: 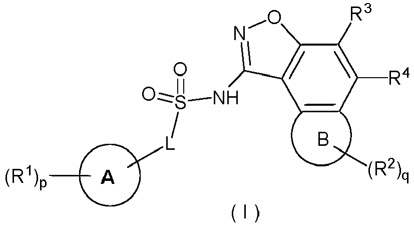 wherein:
ring A is selected from the group consisting of cycloalkyl, heterocyclyl, aryl and heteroaryl;
ring B is cycloalkyl or heterocyclyl;
L is a chemical bond, alkylene or heteroalkylene, wherein the alkylene or heteroalkylene is independently optionally substituted with one or more substituents selected from the group consisting of hydroxy, halogen, alkyl, haloalkyl, hydroxyalkyl, alkoxy and haloalkoxy;
each R¹, each R², and R³ are identical or different and are each independently selected from the group consisting of a hydrogen atom, halogen, cyano, nitro, oxo, alkenyl, alkynyl, alkyl, alkoxy, cycloalkyl, heterocyclyl, aryl, heteroaryl, -OR⁵, -C(O)R⁶, -C(O)OR⁶, -OC(O)R⁶, -NHC(O)OR⁶, -NR⁷R⁸, -C(O)NR⁷R⁸, -S(O)ᵣR⁶ and -S(O)ᵣNR⁷R⁸, wherein the alkenyl, alkynyl, alkyl, alkoxy, cycloalkyl, heterocyclyl, aryl and heteroaryl are each independently optionally substituted with one or more substituents selected from the group consisting of hydroxy, halogen, cyano, amino, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy and heterocyclyloxy;
R⁴ is a hydrogen atom or 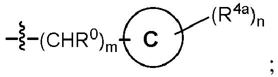 ring C is selected from the group consisting of cycloalkyl, heterocyclyl, aryl and heteroaryl;
R⁰ is selected from the group consisting of a hydrogen atom, hydroxy, halogen, alkyl, haloalkyl, hydroxyalkyl, alkoxy, haloalkoxy and cycloalkyl;
each R^{4a} is identical or different and is independently selected from the group consisting of a hydrogen atom, hydroxy, halogen, cyano, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy, heterocyclyloxy and -NR⁹R¹⁰;
R⁵ and R⁶ are identical or different and are each independently selected from the group consisting of a hydrogen atom, alkenyl, alkynyl, alkyl, cycloalkyl, heterocyclyl, aryl and heteroaryl, wherein the alkenyl, alkynyl, alkyl, cycloalkyl, heterocyclyl, aryl and heteroaryl are each independently optionally substituted with one or more substituents selected from the group consisting of hydroxy, halogen, cyano, amino, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy and heterocyclyloxy;
R⁷, R⁸, R⁹ and R¹⁰ are identical or different and are each independently selected from the group consisting of a hydrogen atom, alkyl, cycloalkyl, heterocyclyl, aryl and heteroaryl, wherein the alkyl, cycloalkyl, heterocyclyl, aryl and heteroaryl are each independently optionally substituted with one or more substituents selected from the group consisting of hydroxy, halogen, cyano, amino, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy and heterocyclyloxy;
or R⁷ and R⁸, together with the N atom to which they are attached, form one heterocyclyl group; or R⁹ and R¹⁰, together with the N atom to which they are attached, form one heterocyclyl group; the heterocyclyl group is optionally substituted with one or more substituents selected from the group consisting of hydroxy, halogen, cyano, amino, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy and heterocyclyloxy;
p is 0, 1, 2, 3 or 4;
q is 0, 1, 2, 3 or 4;
m is 0, 1, 2, 3 or 4;
n is 0, 1, 2, 3 or 4; and
r is 0, 1 or 2.

In some embodiments of the present disclosure, provided is the compound represented by general formula (I) or the pharmaceutically acceptable salt thereof,
wherein:
ring A is selected from the group consisting of cycloalkyl, heterocyclyl, aryl and heteroaryl;
ring B is selected from the group consisting of cycloalkyl and heterocyclyl;
L is a chemical bond, alkylene or heteroalkylene, wherein the alkylene or heteroalkylene is independently optionally substituted with one or more substituents selected from the group consisting of hydroxy, halogen, alkyl, haloalkyl, hydroxyalkyl, alkoxy and haloalkoxy;
each R¹, each R², and R³ are identical or different and are each independently selected from the group consisting of a hydrogen atom, halogen, cyano, nitro, oxo, alkenyl, alkynyl, alkyl, alkoxy, cycloalkyl, heterocyclyl, aryl, heteroaryl, -OR⁵, -C(O)R⁶, -C(O)OR⁶, -OC(O)R⁶, -NHC(O)OR⁶, -NR⁷R⁸, -C(O)NR⁷R⁸, -S(O)ᵣR⁶ and -S(O)ᵣNR⁷R⁸, wherein the alkenyl, alkynyl, alkyl, alkoxy, cycloalkyl, heterocyclyl, aryl and heteroaryl are each independently optionally substituted with one or more substituents selected from the group consisting of hydroxy, halogen, cyano, amino, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy and heterocyclyloxy;
R⁴ is a hydrogen atom or 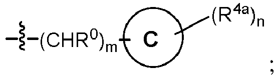
ring C is aryl or heteroaryl;
R⁰ is selected from the group consisting of a hydrogen atom, hydroxy, halogen, alkyl, haloalkyl, hydroxyalkyl, alkoxy, haloalkoxy and cycloalkyl;
each R^{4a} is identical or different and is independently selected from the group consisting of a hydrogen atom, hydroxy, halogen, cyano, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy, heterocyclyloxy and -NR⁹R¹⁰;
R⁵ and R⁶ are identical or different and are each independently selected from the group consisting of a hydrogen atom, alkenyl, alkynyl, alkyl, cycloalkyl, heterocyclyl, aryl and heteroaryl, wherein the alkenyl, alkynyl, alkyl, cycloalkyl, heterocyclyl, aryl and heteroaryl are each independently optionally substituted with one or more substituents selected from the group consisting of hydroxy, halogen, cyano, amino, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy and heterocyclyloxy;
R⁷, R⁸, R⁹ and R¹⁰ are identical or different and are each independently selected from the group consisting of a hydrogen atom, alkyl, cycloalkyl, heterocyclyl, aryl and heteroaryl, wherein the alkyl, cycloalkyl, heterocyclyl, aryl and heteroaryl are each independently optionally substituted with one or more substituents selected from the group consisting of hydroxy, halogen, cyano, amino, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy and heterocyclyloxy;
or R⁷ and R⁸, together with the N atom to which they are attached, form one heterocyclyl group; or R⁹ and R¹⁰, together with the N atom to which they are attached, form one heterocyclyl group; the heterocyclyl group is optionally substituted with one or more substituents selected from the group consisting of hydroxy, halogen, cyano, amino, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy and heterocyclyloxy;
p is 0, 1, 2, 3 or 4;
q is 0, 1, 2, 3 or 4;
m is 0, 1, 2, 3 or 4;
n is 0, 1, 2, 3 or 4; and
r is 0, 1 or 2.

In some embodiments of the present disclosure, provided is the compound represented by general formula (I) or the pharmaceutically acceptable salt thereof, wherein R³ is selected from the group consisting of a hydrogen atom, hydroxy, halogen, C₁₋₆ alkyl, C₁₋₆ haloalkyl, C₁₋₆ hydroxyalkyl, C₁₋₆ alkoxy, C₁₋₆ haloalkoxy and 3- to 8-membered cycloalkyl; preferably, R³ is a hydrogen atom.

In some embodiments of the present disclosure, provided is the compound represented by general formula (I) or the pharmaceutically acceptable salt thereof, wherein ring C is selected from the group consisting of 3- to 8-membered cycloalkyl, 3- to 8-membered heterocyclyl, 6- to 10-membered aryl, and 5- to 10-membered heteroaryl; preferably, ring C is 5- to 10-membered heteroaryl or 6- to 10-membered aryl; more preferably, ring C is 5- or 6-membered heteroaryl; most preferably, ring C is pyrazolyl.

In some embodiments of the present disclosure, provided is the compound represented by general formula (I) or the pharmaceutically acceptable salt thereof, wherein ring C is 5- or 6-membered heterocyclyl, or 5- or 6-membered heteroaryl; preferably, ring C is selected from the group consisting of pyrazolyl, pyridinyl, furanyl and tetrahydrofuranyl; more preferably, ring C is pyrazolyl.

In some embodiments of the present disclosure, provided is the compound represented by general formula (I) or the pharmaceutically acceptable salt thereof, wherein ring C is selected from the group consisting of

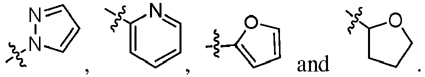

In some embodiments of the present disclosure, provided is the compound represented by general formula (I) or the pharmaceutically acceptable salt thereof, wherein R⁴ is 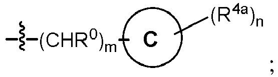 ring C is selected from the group consisting of 3- to 8-membered cycloalkyl, 3- to 8-membered heterocyclyl, 6- to 10-membered aryl, and 5-to 10-membered heteroaryl; R⁰, R^{4a}, m and n are as defined in general formula (I).

In some embodiments of the present disclosure, provided is the compound represented by general formula (I) or the pharmaceutically acceptable salt thereof, wherein R⁴ is 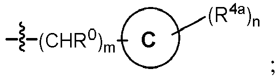 ring C is 5- to 10-membered heteroaryl or 6- to 10-membered aryl; R⁰, R^{4a}, m and n are as defined in general formula (I).

In some embodiments of the present disclosure, provided is the compound represented by general formula (I) or the pharmaceutically acceptable salt thereof, wherein R⁰ is selected from the group consisting of a hydrogen atom, hydroxy, halogen, C₁₋₆ alkyl, C₁₋₆ haloalkyl, C₁₋₆ hydroxyalkyl, C₁₋₆ alkoxy, C₁₋₆ haloalkoxy, and 3- to 8-membered cycloalkyl; preferably, R⁰ is selected from the group consisting of a hydrogen atom, hydroxy, halogen and C₁₋₆ alkyl; preferably, R⁰ is selected from the group consisting of a hydrogen atom, hydroxy and Cl; more preferably, R⁰ is a hydrogen atom.

In some embodiments of the present disclosure, provided is the compound represented by general formula (I) or the pharmaceutically acceptable salt thereof, wherein m is 0 or 1, preferably 1.

In some embodiments of the present disclosure, provided is the compound represented by general formula (I) or the pharmaceutically acceptable salt thereof, wherein R⁴ is 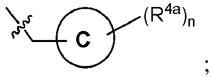 ring C is 5- to 10-membered heteroaryl, preferably 5- or 6-membered heteroaryl; R^{4a} and n are as defined in general formula (I);
preferably, R⁴ is 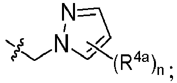 R^{4a} and n are as defined in general formula (I);
more preferably, R⁴ is

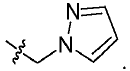

In some embodiments of the present disclosure, the compound represented by general formula (I) or the pharmaceutically acceptable salt thereof is a compound represented by general formula (II) or a pharmaceutically acceptable salt thereof: 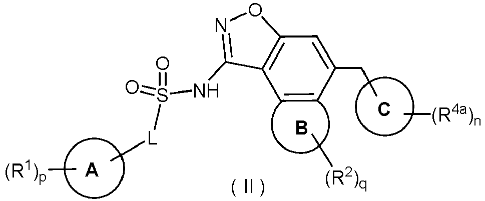 wherein:
ring C is 5- to 10-membered heteroaryl, preferably 5- or 6-membered heteroaryl;
ring A, ring B, L, R¹, R², R^{4a}, p, q and n are as defined in general formula (I).

In some embodiments of the present disclosure, provided is the compound represented by general formula (I) or the pharmaceutically acceptable salt thereof, wherein R⁴ is 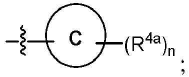 ring C is 3- to 8-membered heterocyclyl or 5- to 10-membered heteroaryl, preferably 5- or 6-membered heterocyclyl, or 5- or 6-membered heteroaryl; R^{4a} and n are as defined in general formula (I);
preferably, R⁴ is 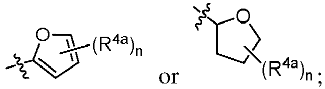 R^{4a} and n are as defined in general formula (I);
further preferably, R⁴ is 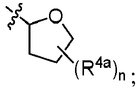 R^{4a} and n are as defined in general formula (I);
more preferably, R⁴ is 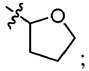
still more preferably, R⁴ is

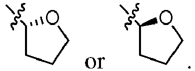

In some embodiments of the present disclosure, the compound represented by general formula (I) or the pharmaceutically acceptable salt thereof is a compound represented by general formula (Ii) or a pharmaceutically acceptable salt thereof: 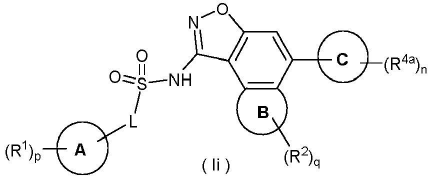 wherein:
ring C is 3- to 8-membered heterocyclyl or 5- to 10-membered heteroaryl, preferably 5- or 6-membered heterocyclyl, or 5- or 6-membered heteroaryl;
ring A, ring B, L, R¹, R², R^{4a}, p, q and n are as defined in general formula (I).

In some embodiments of the present disclosure, provided is the compound represented by general formula (I) or the pharmaceutically acceptable salt thereof, wherein R⁴ is 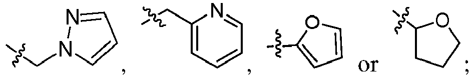 preferably, R⁴ is

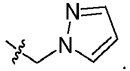

In some embodiments of the present disclosure, provided is the compound represented by general formula (I), general formula (II) or general formula (Ii) or the pharmaceutically acceptable salt thereof, wherein ring A is 6- to 10-membered aryl or 5-to 10-membered heteroaryl, and the 6- to 10-membered aryl is preferably phenyl or naphthyl; the 5- to 10-membered heteroaryl is preferably pyridinyl, quinolinyl and benzoxazolyl.

In some embodiments of the present disclosure, provided is the compound represented by general formula (I), general formula (II) or general formula (Ii) or the pharmaceutically acceptable salt thereof, wherein ring A is 6- to 10-membered aryl or 5-to 10-membered heteroaryl, and the 6- to 10-membered aryl is preferably selected from the group consisting of phenyl, naphthyl, 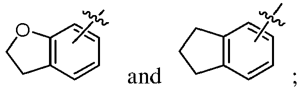 the 5- to 10-membered heteroaryl is preferably pyridinyl, quinolinyl or benzoxazolyl.

In some embodiments of the present disclosure, provided is the compound represented by general formula (I), general formula (II) or general formula (Ii) or the pharmaceutically acceptable salt thereof, wherein ring A is 6- to 10-membered aryl, and is preferably selected from the group consisting of phenyl, 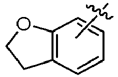 and 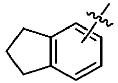 is phenyl; ring A is more preferably phenyl.

In some embodiments of the present disclosure, provided is the compound represented by general formula (I), general formula (II) or general formula (Ii) or the pharmaceutically acceptable salt thereof, wherein 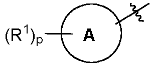 is selected from the group consisting of 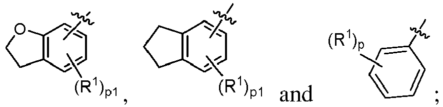 p1 is 0, 1, 2 or 3; R¹ and p are as defined in general formula (I).

In some embodiments of the present disclosure, provided is the compound represented by general formula (I), general formula (II) or general formula (Ii) or the pharmaceutically acceptable salt thereof, wherein 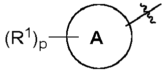 is 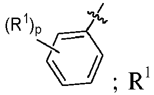 and p are as defined in general formula (I).

In some embodiments of the present disclosure, provided is the compound represented by general formula (I), general formula (II) or general formula (Ii) or the pharmaceutically acceptable salt thereof, wherein ring B is 3- to 8-membered heterocyclyl or 3- to 8-membered cycloalkyl;
preferably, ring B is 4- to 7-membered heterocyclyl or 4- to 7-membered cycloalkyl; further preferably, ring B is 4- to 7-membered heterocyclyl;
still more preferably, ring B is 4- to 7-membered heterocyclyl, wherein the 4- to 7-membered heterocyclyl contains 1-3 oxygen atoms;
most preferably, ring B is 5- or 6-membered heterocyclyl, wherein the 5- or 6-membered heterocyclyl contains 1 or 2 oxygen atoms.

In some embodiments of the present disclosure, provided is the compound represented by general formula (I), general formula (II) or general formula (Ii) or the pharmaceutically acceptable salt thereof, wherein ring B is selected from the group consisting of 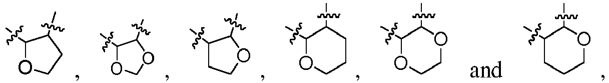 and is preferably 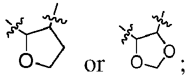 ring B may be substituted with R² at any substitutable position.

In some embodiments of the present disclosure, provided is the compound represented by general formula (I), general formula (II) or general formula (Ii) or the pharmaceutically acceptable salt thereof, wherein ring B is selected from the group consisting of 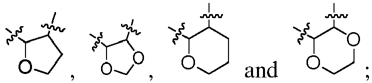 preferably, ring B is selected from the group consisting of 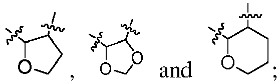 more preferably, ring B is 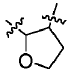 or 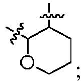 ring B may be substituted with R² at any substitutable position.

In some embodiments of the present disclosure, the compound represented by general formula (I) or general formula (II) or the pharmaceutically acceptable salt thereof is a compound represented by general formula (III) or a pharmaceutically acceptable salt thereof: 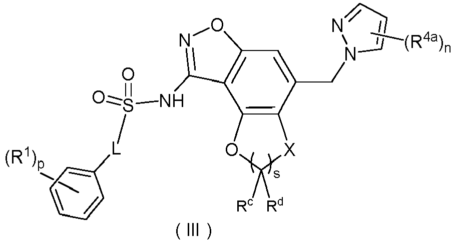 wherein:
X is selected from the group consisting of O, CR^{a}R^{b} and C=O;
each R^{a}, R^{b}, R^{c} and R^{d} is identical or different and is independently selected from the group consisting of a hydrogen atom, hydroxy, halogen, cyano, amino, alkenyl, alkynyl, alkyl, haloalkyl, hydroxyalkyl, alkoxy, haloalkoxy, cycloalkyl, heterocyclyl, aryl, heteroaryl, cycloalkyloxy and heterocyclyloxy;
or R^{c} and R^{d}, together with the carbon atom to which they are attached, form one C=O;
s is 0, 1, 2 or 3;
L, R¹, R^{4a}, p and n are as defined in general formula (I).

In some embodiments of the present disclosure, provided is the compound represented by general formula (III) or the pharmaceutically acceptable salt thereof, wherein each R^{c} and R^{d} is identical or different and is independently selected from the group consisting of a hydrogen atom, hydroxy, halogen, cyano, amino, C₁₋₆ alkyl, C₁₋₆ haloalkyl, C₁₋₆ hydroxyalkyl, C₁₋₆ alkoxy, C₁₋₆ haloalkoxy, 3- to 8-membered cycloalkyl, 3- to 8-membered heterocyclyl, 3- to 8-membered cycloalkyloxy, and 3- to 8-membered heterocyclyloxy; or R^{c} and R^{d}, together with the carbon atom to which they are attached, form one C=O;
preferably, each R^{c} and R^{d} is identical or different and is independently selected from the group consisting of a hydrogen atom, hydroxy, halogen, C₁₋₆ alkyl, C₁₋₆ haloalkyl, C₁₋₆ hydroxyalkyl and C₁₋₆ alkoxy; or R^{c} and R^{d}, together with the carbon atom to which they are attached, form one C=O;
further preferably, each R^{c} and R^{d} is identical or different and is independently selected from the group consisting of a hydrogen atom, halogen, C₁₋₆ alkyl and C₁₋₆ alkoxy;
still further preferably, each R^{c} and R^{d} is identical or different and is independently a hydrogen atom or halogen;
even further preferably, each R^{c} and R^{d} is identical or different and is independently a hydrogen atom or a fluorine atom;
most preferably, R^{c} and R^{d} are both hydrogen atoms.

In some embodiments of the present disclosure, the compound represented by general formula (I), general formula (II) or general formula (III) or the pharmaceutically acceptable salt thereof is a compound represented by general formula (IV) or a pharmaceutically acceptable salt thereof: 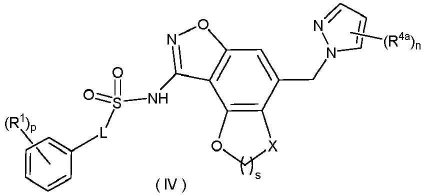 wherein:
L, X, R¹, R^{4a}, p, n and s are as defined in general formula (III).

In some embodiments of the present disclosure, provided is the compound represented by general formula (III) or general formula (IV) or the pharmaceutically acceptable salt thereof, wherein X is O or CR^{a}R^{b}; each R^{a} and R^{b} is identical or different and is independently selected from the group consisting of a hydrogen atom, hydroxy, halogen, cyano, amino, C₁₋₆ alkyl, C₁₋₆ haloalkyl, C₁₋₆ hydroxyalkyl, C₁₋₆ alkoxy, C₁₋₆ haloalkoxy, 3- to 8-membered cycloalkyl, 3- to 8-membered heterocyclyl, 3- to 8-membered cycloalkyloxy, and 3- to 8-membered heterocyclyloxy; preferably, each R^{a} and R^{b} is identical or different and is independently selected from the group consisting of a hydrogen atom, hydroxy, halogen, C₁₋₆ alkyl, C₁₋₆ haloalkyl, C₁₋₆ hydroxyalkyl and C₁₋₆ alkoxy; further preferably, each R^{a} and R^{b} is identical or different and is independently selected from the group consisting of a hydrogen atom, halogen, C₁₋₆ alkyl and C₁₋₆ alkoxy; most preferably, R^{a} and R^{b} are both hydrogen atoms.

In some embodiments of the present disclosure, provided is the compound represented by general formula (III) or general formula (IV) or the pharmaceutically acceptable salt thereof, wherein X is O or CH₂; preferably, X is CH₂.

In some embodiments of the present disclosure, provided is the compound represented by general formula (III) or general formula (IV) or the pharmaceutically acceptable salt thereof, wherein s is 1 or 2, preferably 1.

In some embodiments of the present disclosure, provided is the compound represented by general formula (III) or the pharmaceutically acceptable salt thereof, wherein R^{c} and R^{d} are identical or different and are each independently hydrogen atoms or halogens; and/or X is O or CH₂; and/or s is 1 or 2.

In some embodiments of the present disclosure, provided is the compound represented by general formula (IV) or the pharmaceutically acceptable salt thereof, wherein X is O or CH₂; and/or s is 1 or 2.

In some embodiments of the present disclosure, provided is the compound represented by general formula (I), general formula (II) or general formula (Ii) or the pharmaceutically acceptable salt thereof, wherein q is 0, 1 or 2; preferably, q is 0.

In some embodiments of the present disclosure, provided is the compound represented by general formula (I), general formula (II) or general formula (Ii) or the pharmaceutically acceptable salt thereof, wherein q is 1.

In some embodiments of the present disclosure, provided is the compound represented by general formula (I), general formula (II), general formula (Ii), general formula (III) or general formula (IV) or the pharmaceutically acceptable salt thereof, wherein each R¹ is identical or different and is independently selected from the group consisting of a hydrogen atom, hydroxy, halogen, cyano, amino, C₁₋₆ alkyl, C₁₋₆ haloalkyl, C₁₋₆ hydroxyalkyl, C₁₋₆ alkoxy, C₁₋₆ haloalkoxy, oxo, 3- to 8-membered cycloalkyl, C₁₋₆ alkoxy C₁₋₆ alkyl, -OR⁵, -C(O)R⁶, -C(O)OR⁶, -C(O)NR⁷R⁸ and -S(O)ᵣR⁶, and r, R⁵, R⁶, R⁷ and R⁸ are as defined in general formula (I); preferably, each R¹ is identical or different and is independently selected from the group consisting of a hydrogen atom, hydroxy, halogen, C₁₋₆ alkyl, C₁₋₆ haloalkyl, C₁₋₆ hydroxyalkyl, C₁₋₆ alkoxy, C₁₋₆ haloalkoxy, C₁₋₆ alkoxy C₁₋₆ alkyl and -C(O)OCH₃; more preferably, each R¹ is identical or different and is independently C₁₋₆ alkoxy; most preferably, R¹ is methoxy.

In some embodiments of the present disclosure, provided is the compound represented by general formula (I), general formula (II), general formula (Ii), general formula (III) or general formula (IV) or the pharmaceutically acceptable salt thereof, wherein each R¹ is identical or different and is independently selected from the group consisting of a hydrogen atom, hydroxy, halogen, cyano, C₁₋₆ alkyl, C₁₋₆ haloalkyl, C₁₋₆ hydroxyalkyl, C₁₋₆ alkoxy, C₁₋₆ haloalkoxy, oxo, 3- to 8-membered cycloalkyl, C₁₋₆ alkoxy C₁₋₆ alkyl, -NR⁷R⁸, -OR⁵, -C(O)R⁶, -C(O)OR⁶, -C(O)NR⁷R⁸ and -S(O)ᵣR⁶, and r, R⁵, R⁶, R⁷ and R⁸ are as defined in general formula (I);
preferably, each R¹ is identical or different and is independently selected from the group consisting of a hydrogen atom, hydroxy, halogen, C₁₋₆ alkyl, C₁₋₆ haloalkyl, C₁₋₆ hydroxyalkyl, C₁₋₆ alkoxy, C₁₋₆ haloalkoxy, C₁₋₆ alkoxy C₁₋₆ alkyl, -C(O)OCH₃ and -NR⁷R⁸; R⁷ and R⁸ are identical or different and are each independently hydrogen atoms or C₁₋₆ alkyl;
further preferably, each R¹ is identical or different and is independently selected from the group consisting of a hydrogen atom, halogen, C₁₋₆ alkyl, C₁₋₆ haloalkyl, C₁₋₆ alkoxy, C₁₋₆ haloalkoxy and -NR⁷R⁸; R⁷ and R⁸ are identical or different and are each independently hydrogen atoms or C₁₋₆ alkyl;
more preferably, each R¹ is identical or different and is independently C₁₋₆ alkoxy; most preferably, each R¹ is identical or different and is independently methoxy.

In some embodiments of the present disclosure, provided is the compound represented by general formula (I), general formula (II), general formula (Ii), general formula (III) or general formula (IV) or the pharmaceutically acceptable salt thereof, wherein each R¹ is identical or different and is independently selected from the group consisting of a hydrogen atom, a fluorine atom, a chlorine atom, methyl, ethyl, isopropyl, methoxy, ethoxy, trifluoromethoxy, monomethylamino, dimethylamino and 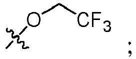 preferably, R¹ is methoxy.

In some embodiments of the present disclosure, provided is the compound represented by general formula (I), general formula (II) or general formula (Ii) or the pharmaceutically acceptable salt thereof, wherein each R² is identical or different and is independently selected from the group consisting of a hydrogen atom, hydroxy, halogen, cyano, amino, C₁₋₆ alkyl, C₁₋₆ haloalkyl, C₁₋₆ hydroxyalkyl, C₁₋₆ alkoxy, C₁₋₆ haloalkoxy, 3- to 8-membered cycloalkyl, 3- to 8-membered heterocyclyl, oxo, 3- to 8-membered cycloalkyloxy, and 3- to 8-membered heterocyclyloxy; preferably, each R² is identical or different and is independently selected from the group consisting of a hydrogen atom, hydroxy, halogen, C₁₋₆ alkyl, C₁₋₆ haloalkyl, C₁₋₆ hydroxyalkyl, C₁₋₆ alkoxy and oxo; further preferably, each R² is identical or different and is independently selected from the group consisting of a hydrogen atom, halogen, C₁₋₆ alkyl and C₁₋₆ alkoxy; more preferably, each R² is identical or different and is independently a hydrogen atom or halogen; even more preferably, each R² is identical or different and is independently a hydrogen atom or a fluorine atom; most preferably, R² is a hydrogen atom.

In some embodiments of the present disclosure, provided is the compound represented by general formula (I), general formula (II), general formula (Ii), general formula (III) or general formula (IV) or the pharmaceutically acceptable salt thereof, wherein each R^{4a} is identical or different and is independently selected from the group consisting of a hydrogen atom, hydroxy, halogen, cyano, C₁₋₆ alkyl, C₁₋₆ haloalkyl, C₁₋₆ hydroxyalkyl, C₁₋₆ alkoxy, C₁₋₆ haloalkoxy, -NR⁹R¹⁰, 3- to 8-membered cycloalkyl, 3- to 8-membered heterocyclyl, 3- to 8-membered cycloalkyloxy, and 3- to 8-membered heterocyclyloxy, and R⁹ and R¹⁰ are as defined in general formula (I); preferably, each R^{4a} is identical or different and is independently selected from the group consisting of a hydrogen atom, hydroxy, halogen, C₁₋₆ alkyl, C₁₋₆ hydroxyalkyl and C₁₋₆ alkoxy; more preferably, R^{4a} is a hydrogen atom.

In some embodiments of the present disclosure, provided is the compound represented by general formula (I), general formula (II), general formula (Ii), general formula (III) or general formula (IV) or the pharmaceutically acceptable salt thereof, wherein p is 0, 1, 2 or 3; preferably, p is 1, 2 or 3; more preferably, p is 2.

In some embodiments of the present disclosure, provided is the compound represented by general formula (I), general formula (II), general formula (Ii), general formula (III) or general formula (IV) or the pharmaceutically acceptable salt thereof, wherein p is 0, 1 or 2; preferably, p is 1 or 2.

In some embodiments of the present disclosure, provided is the compound represented by general formula (I), general formula (II), general formula (Ii), general formula (III) or general formula (IV) or the pharmaceutically acceptable salt thereof, wherein L is a chemical bond or C₁₋₆ alkylene; preferably, L is selected from the group consisting of a chemical bond, -CH₂- and -CH₂CH₂-; more preferably, L is a chemical bond or -CH₂-, most preferably, L is a chemical bond.

In some embodiments of the present disclosure, provided is the compound represented by general formula (I), general formula (II), general formula (Ii), general formula (III) or general formula (IV) or the pharmaceutically acceptable salt thereof, wherein n is 1 or 2.

In some embodiments of the present disclosure, provided is the compound represented by general formula (I), general formula (II), general formula (Ii), general formula (III) or general formula (IV) or the pharmaceutically acceptable salt thereof, wherein n is 0, 1, 2 or 3; preferably, n is 0.

In some embodiments of the present disclosure, provided is the compound represented by general formula (III) or general formula (IV) or the pharmaceutically acceptable salt thereof, wherein each R^{4a} is identical or different and is independently selected from the group consisting of halogen, hydroxy, C₁₋₆ alkyl, C₁₋₆ hydroxyalkyl and C₁₋₆ alkoxy, and n is 1 or 2; or each R^{4a} is a hydrogen atom, and n is 3.

In some embodiments of the present disclosure, provided is the compound represented by general formula (III) or general formula (IV) or the pharmaceutically acceptable salt thereof, wherein each R^{4a} is a hydrogen atom, and n is 3.

In some embodiments of the present disclosure, provided is the compound represented by general formula (I), general formula (II), general formula (Ii), general formula (III) or general formula (IV) or the pharmaceutically acceptable salt thereof, wherein R⁵ and R⁶ are identical or different and are each independently selected from the group consisting of a hydrogen atom, C₁₋₆ alkyl, 3- to 8-membered cycloalkyl, 3- to 12-membered heterocyclyl, 6- to 10-membered aryl, and 5- to 10-membered heteroaryl, wherein the C₁₋₆ alkyl, 3- to 8-membered cycloalkyl, 3- to 12-membered heterocyclyl, 6- to 10-membered aryl, and 5- to 10-membered heteroaryl are each independently optionally substituted with one or more substituents selected from the group consisting of hydroxy, halogen, cyano, amino, nitro, oxo, C₁₋₆ alkyl, C₁₋₆ haloalkyl, C₁₋₆ hydroxyalkyl, C₁₋₆ alkoxy, C₁₋₆ haloalkoxy and 3- to 8-membered cycloalkyl; preferably, R⁵ and R⁶ are identical or different and are each independently selected from the group consisting of C₁₋₆ alkyl, C₁₋₆ haloalkyl and 3- to 8-membered cycloalkyl.

In some embodiments of the present disclosure, provided is the compound represented by general formula (I), general formula (II), general formula (Ii), general formula (III) or general formula (IV) or the pharmaceutically acceptable salt thereof, wherein R⁷ and R⁸ are identical or different and are each independently selected from the group consisting of a hydrogen atom, C₁₋₆ alkyl, C₁₋₆ haloalkyl, C₁₋₆ hydroxyalkyl, 3- to 8-membered cycloalkyl, and 3- to 12-membered heterocyclyl; preferably, R⁷ and R⁸ are identical or different and are each independently hydrogen atoms or C₁₋₆ alkyl; more preferably, R⁷ and R⁸ are hydrogen atoms.

In some embodiments of the present disclosure, provided is the compound represented by general formula (I), general formula (II), general formula (Ii), general formula (III) or general formula (IV) or the pharmaceutically acceptable salt thereof, wherein R⁹ and R¹⁰ are identical or different and are each independently selected from the group consisting of a hydrogen atom, C₁₋₆ alkyl, C₁₋₆ haloalkyl, C₁₋₆ hydroxyalkyl, 3- to 8-membered cycloalkyl, and 3- to 12-membered heterocyclyl; preferably, R⁹ and R¹⁰ are hydrogen atoms.

In some embodiments of the present disclosure, provided are the compounds represented by general formula (I), general formula (II) and general formula (Ii) or the pharmaceutically acceptable salts thereof, wherein 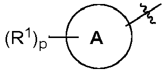 is 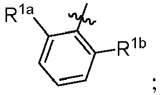 R^{1a} and R^{1b} are identical or different and are each independently selected from the group consisting of a hydrogen atom, hydroxy, halogen, cyano, amino, C₁₋₆ alkyl, C₁₋₆ haloalkyl, C₁₋₆ hydroxyalkyl, C₁₋₆ alkoxy, C₁₋₆ haloalkoxy, oxo, 3- to 8-membered cycloalkyl, C₁₋₆ alkoxy C₁₋₆ alkyl, -OR⁵, -C(O)R⁶, -C(O)OR⁶, -C(O)NR⁷R⁸ and -S(O)ᵣR⁶, and r, R⁵, R⁶, R⁷ and R⁸ are as defined in general formula (I); preferably, R^{1a} and R^{1b} are identical or different and are each independently selected from the group consisting of a hydrogen atom, hydroxy, halogen, C₁₋₆ alkyl, C₁₋₆ haloalkyl, C₁₋₆ hydroxyalkyl, C₁₋₆ alkoxy, C₁₋₆ haloalkoxy, C₁₋₆ alkoxy C₁₋₆ alkyl and -C(O)OCH₃; more preferably, R^{1a} is C₁₋₆ alkoxy, and/or R^{1b} is C₁₋₆ alkoxy; most preferably, R^{1a} and R^{1b} are both methoxy.

In some embodiments of the present disclosure, provided is the compound represented by general formula (III) or general formula (IV) or the pharmaceutically acceptable salt thereof, wherein 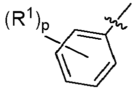 is 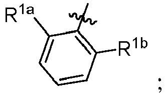 R^{1a} and R^{1b} are identical or different and are each independently selected from the group consisting of a hydrogen atom, hydroxy, halogen, cyano, amino, C₁₋₆ alkyl, C₁₋₆ haloalkyl, C₁₋₆ hydroxyalkyl, C₁₋₆ alkoxy, C₁₋₆ haloalkoxy, oxo, 3- to 8-membered cycloalkyl, C₁₋₆ alkoxy C₁₋₆ alkyl, -OR⁵, -C(O)R⁶, -C(O)OR⁶, -C(O)NR⁷R⁸ and -S(O)ᵣR⁶, and r, R⁵, R⁶, R⁷ and R⁸ are as defined in general formula (I); preferably, R^{1a} and R^{1b} are identical or different and are each independently selected from the group consisting of a hydrogen atom, hydroxy, halogen, C₁₋₆ alkyl, C₁₋₆ haloalkyl, C₁₋₆ hydroxyalkyl, C₁₋₆ alkoxy, C₁₋₆ haloalkoxy, C₁₋₆ alkoxy C₁₋₆ alkyl and -C(O)OCH₃; more preferably, R^{1a} is C₁₋₆ alkoxy, and/or R^{1b} is C₁₋₆ alkoxy; most preferably, R^{1a} and R^{1b} are both methoxy.

In some embodiments of the present disclosure, provided are the compounds represented by general formula (I), general formula (II) and general formula (Ii) or the pharmaceutically acceptable salts thereof, wherein 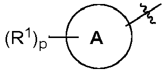 is 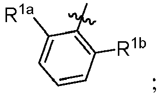 R^{1a} and R^{1b} are identical or different and are each independently selected from the group consisting of a hydrogen atom, hydroxy, halogen, cyano, amino, C₁₋₆ alkyl, C₁₋₆ haloalkyl, C₁₋₆ hydroxyalkyl, C₁₋₆ alkoxy, C₁₋₆ haloalkoxy, oxo, 3- to 8-membered cycloalkyl, C₁₋₆ alkoxy C₁₋₆ alkyl, -OR⁵, -C(O)R⁶, -C(O)OR⁶, -C(O)NR⁷R⁸ and -S(O)ᵣR⁶, and r, R⁵, R⁶, R⁷ and R⁸ are as defined in general formula (I); preferably, R^{1a} and R^{1b} are identical or different and are each independently selected from the group consisting of a hydrogen atom, hydroxy, halogen, C₁₋₆ alkyl, C₁₋₆ haloalkyl, C₁₋₆ hydroxyalkyl, C₁₋₆ alkoxy, C₁₋₆ haloalkoxy, C₁₋₆ alkoxy C₁₋₆ alkyl and -C(O)OCH₃; more preferably, R^{1a} is a hydrogen atom or C₁₋₆ alkoxy, and/or R^{1b} is a hydrogen atom or C₁₋₆ alkoxy; most preferably, R^{1a} and R^{1b} are both methoxy; or R^{1a} is a hydrogen atom, and R^{1b} is methoxy.

In some embodiments of the present disclosure, provided is the compound represented by general formula (III) or general formula (IV) or the pharmaceutically acceptable salt thereof, wherein 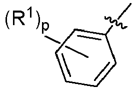 is 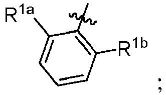 R^{1a} and R^{1b} are identical or different and are each independently selected from the group consisting of a hydrogen atom, hydroxy, halogen, cyano, amino, C₁₋₆ alkyl, C₁₋₆ haloalkyl, C₁₋₆ hydroxyalkyl, C₁₋₆ alkoxy, C₁₋₆ haloalkoxy, oxo, 3- to 8-membered cycloalkyl, C₁₋₆ alkoxy C₁₋₆ alkyl, -OR⁵, -C(O)R⁶, -C(O)OR⁶, -C(O)NR⁷R⁸ and -S(O)ᵣR⁶, and r, R⁵, R⁶, R⁷ and R⁸ are as defined in general formula (I); preferably, R^{1a} and R^{1b} are identical or different and are each independently selected from the group consisting of a hydrogen atom, hydroxy, halogen, C₁₋₆ alkyl, C₁₋₆ haloalkyl, C₁₋₆ hydroxyalkyl, C₁₋₆ alkoxy, C₁₋₆ haloalkoxy, C₁₋₆ alkoxy C₁₋₆ alkyl and -C(O)OCH₃; more preferably, R^{1a} is a hydrogen atom or C₁₋₆ alkoxy, and/or R^{1b} is a hydrogen atom or C₁₋₆ alkoxy; most preferably, R^{1a} and R^{1b} are both methoxy; or R^{1a} is a hydrogen atom, and R^{1b} is methoxy.

In some embodiments of the present disclosure, provided is the compound represented by general formula (III) or the pharmaceutically acceptable salt thereof, wherein 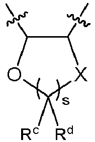 is 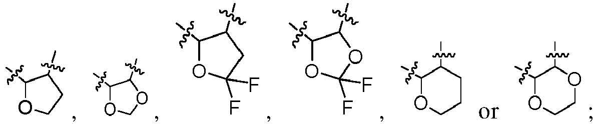 preferably, 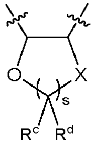 is selected from the group consisting of 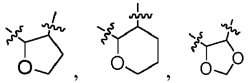 and 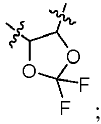 more preferably,

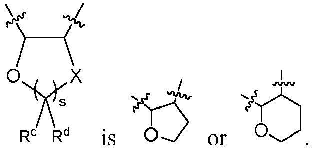

In some embodiments of the present disclosure, provided is the compound represented by general formula (IV) or the pharmaceutically acceptable salt thereof, wherein 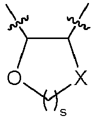 is selected from the group consisting of 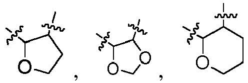 and 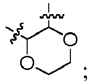 preferably, 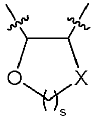 is selected from the group consisting of 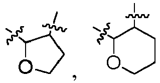 and 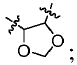 more preferably,

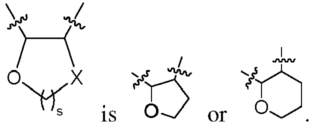

In some embodiments of the present disclosure, provided is the compound represented by general formula (I) or the pharmaceutically acceptable salt thereof, wherein 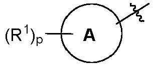 is selected from the group consisting of 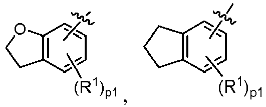 and 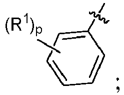 L is a chemical bond; ring B is 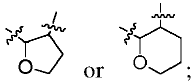 each R² is identical or different and is independently a hydrogen atom or a fluorine atom; q is 0, 1 or 2; R³ is a hydrogen atom; R⁴ is 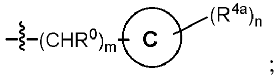 ring C is 5- or 6-membered heterocyclyl, or 5- or 6-membered heteroaryl; m is 0 or 1; n is 0; R⁰ is selected from the group consisting of a hydrogen atom, hydroxy and Cl; each R¹ is identical or different and is independently selected from the group consisting of a hydrogen atom, halogen, C₁₋₆ alkyl, C₁₋₆ haloalkyl, C₁₋₆ alkoxy, C₁₋₆ haloalkoxy and -NR⁷R⁸; R⁷ and R⁸ are identical or different and are each independently hydrogen atoms or C₁₋₆ alkyl; p is 0, 1 or 2; p1 is 0, 1, 2 or 3.

In some embodiments of the present disclosure, provided is the compound represented by general formula (I) or the pharmaceutically acceptable salt thereof, wherein each R¹ is identical or different and is independently selected from the group consisting of a hydrogen atom, hydroxy, halogen, C₁₋₆ alkyl, C₁₋₆ haloalkyl, C₁₋₆ hydroxyalkyl, C₁₋₆ alkoxy, C₁₋₆ haloalkoxy, C₁₋₆ alkoxy C₁₋₆ alkyl and -C(O)OCH₃; p is 1, 2 or 3; each R² is identical or different and is independently selected from the group consisting of a hydrogen atom, halogen, C₁₋₆ alkyl and C₁₋₆ alkoxy; q is 1; R³ is a hydrogen atom; R⁴ is 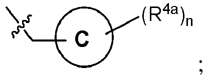 each R^{4a} is identical or different and is independently selected from the group consisting of a hydrogen atom, hydroxy, halogen, C₁₋₆ alkyl, C₁₋₆ hydroxyalkyl and C₁₋₆ alkoxy; n is 1 or 2; ring C is 5- to 10-membered heteroaryl; ring A is 6- to 10-membered aryl; ring B is 4- to 7-membered heterocyclyl; and L is a chemical bond or -CH₂-.

In some embodiments of the present disclosure, provided is the compound represented by general formula (I) or the pharmaceutically acceptable salt thereof, wherein each R¹ is identical or different and is independently selected from the group consisting of a hydrogen atom, hydroxy, halogen, C₁₋₆ alkyl, C₁₋₆ haloalkyl, C₁₋₆ hydroxyalkyl, C₁₋₆ alkoxy, C₁₋₆ haloalkoxy, C₁₋₆ alkoxy C₁₋₆ alkyl, -C(O)OCH₃ and -NR⁷R⁸; R⁷ and R⁸ are identical or different and are each independently hydrogen atoms or C₁₋₆ alkyl; p is 0, 1, 2, 3 or 4; each R² is identical or different and is independently selected from the group consisting of a hydrogen atom, halogen, C₁₋₆ alkyl and C₁₋₆ alkoxy; q is 0, 1, 2, 3 or 4; R³ is a hydrogen atom; R⁴ is 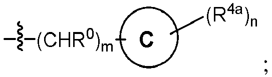 each R^{4a} is identical or different and is independently selected from the group consisting of a hydrogen atom, hydroxy, halogen, C₁₋₆ alkyl, C₁₋₆ hydroxyalkyl and C₁₋₆ alkoxy; n is 0, 1, 2, 3 or 4; m is 0 or 1; ring C is selected from the group consisting of 3- to 8-membered cycloalkyl, 3-to 8-membered heterocyclyl, 6- to 10-membered aryl, and 5- to 10-membered heteroaryl; R⁰ is selected from the group consisting of a hydrogen atom, hydroxy, halogen and C₁₋₆ alkyl; ring A is 6- to 10-membered aryl; ring B is 4- to 7-membered heterocyclyl; and L is a chemical bond or -CH₂-.

In some embodiments of the present disclosure, provided is the compound represented by general formula (II) or the pharmaceutically acceptable salt thereof, wherein each R¹ is identical or different and is independently selected from the group consisting of a hydrogen atom, hydroxy, halogen, C₁₋₆ alkyl, C₁₋₆ haloalkyl, C₁₋₆ hydroxyalkyl, C₁₋₆ alkoxy, C₁₋₆ haloalkoxy, C₁₋₆ alkoxy C₁₋₆ alkyl and -C(O)OCH₃; p is 1, 2 or 3; each R² is identical or different and is independently selected from the group consisting of a hydrogen atom, halogen, C₁₋₆ alkyl and C₁₋₆ alkoxy; q is 1; each R^{4a} is identical or different and is independently selected from the group consisting of a hydrogen atom, halogen, hydroxy, C₁₋₆ alkyl, C₁₋₆ hydroxyalkyl and C₁₋₆ alkoxy; n is 1 or 2; ring A is 6-to 10-membered aryl; ring B is 4- to 7-membered heterocyclyl; ring C is 5- to 10-membered heteroaryl; and L is a chemical bond or -CH₂-.

In some embodiments of the present disclosure, provided is the compound represented by general formula (II) or the pharmaceutically acceptable salt thereof, wherein each R¹ is identical or different and is independently selected from the group consisting of a hydrogen atom, halogen, C₁₋₆ alkyl, C₁₋₆ haloalkyl, C₁₋₆ alkoxy, C₁₋₆ haloalkoxy and -NR⁷R⁸; R⁷ and R⁸ are identical or different and are each independently hydrogen atoms or C₁₋₆ alkyl; p is 0, 1, 2 or 3; each R² is identical or different and is independently a hydrogen atom or halogen; q is 0, 1 or 2; each R^{4a} is identical or different and is independently selected from the group consisting of a hydrogen atom, hydroxy, halogen, C₁₋₆ alkyl, C₁₋₆ hydroxyalkyl and C₁₋₆ alkoxy; n is 0, 1, 2 or 3; ring A is 6- to 10-membered aryl; ring B is 4- to 7-membered heterocyclyl; ring C is 5- or 6-membered heteroaryl; and L is a chemical bond.

In some embodiments of the present disclosure, provided is the compound represented by general formula (Ii) or the pharmaceutically acceptable salt thereof, wherein each R¹ is identical or different and is independently selected from the group consisting of a hydrogen atom, halogen, C₁₋₆ alkyl, C₁₋₆ haloalkyl, C₁₋₆ alkoxy, C₁₋₆ haloalkoxy and -NR⁷R⁸; R⁷ and R⁸ are identical or different and are each independently hydrogen atoms or C₁₋₆ alkyl; p is 0, 1, 2 or 3; each R² is identical or different and is independently a hydrogen atom or halogen; q is 0, 1 or 2; each R^{4a} is identical or different and is independently selected from the group consisting of a hydrogen atom, hydroxy, halogen, C₁₋₆ alkyl, C₁₋₆ hydroxyalkyl and C₁₋₆ alkoxy; n is 0, 1, 2 or 3; ring A is 6- to 10-membered aryl; ring B is 4- to 7-membered heterocyclyl; ring C is 5- or 6-membered heterocyclyl, or 5- or 6-membered heteroaryl; and L is a chemical bond.

In some embodiments of the present disclosure, provided is the compound represented by general formula (III) or the pharmaceutically acceptable salt thereof, wherein each R¹ is identical or different and is independently selected from the group consisting of a hydrogen atom, hydroxy, halogen, C₁₋₆ alkyl, C₁₋₆ haloalkyl, C₁₋₆ hydroxyalkyl, C₁₋₆ alkoxy, C₁₋₆ haloalkoxy, C₁₋₆ alkoxy C₁₋₆ alkyl and -C(O)OCH₃; p is 1, 2 or 3; X is O or CH₂; R^{c} and R^{d} are both hydrogen atoms; each R^{4a} is identical or different and is independently selected from the group consisting of halogen, hydroxy, C₁₋₆ alkyl, C₁₋₆ hydroxyalkyl and C₁₋₆ alkoxy, and n is 1 or 2; or each R^{4a} is a hydrogen atom, and n is 3; s is 1 or 2; and L is a chemical bond or -CH₂-.

In some embodiments of the present disclosure, provided is the compound represented by general formula (III) or the pharmaceutically acceptable salt thereof, wherein each R¹ is identical or different and is independently selected from the group consisting of a hydrogen atom, halogen, C₁₋₆ alkyl, C₁₋₆ haloalkyl, C₁₋₆ alkoxy, C₁₋₆ haloalkoxy and -NR⁷R⁸; R⁷ and R⁸ are identical or different and are each independently hydrogen atoms or C₁₋₆ alkyl; p is 0, 1, 2 or 3; X is O or CH₂; each R^{c} and R^{d} is identical or different and is independently a hydrogen atom or halogen; each R^{4a} is a hydrogen atom; n is 3; s is 1 or 2; and L is a chemical bond.

In some embodiments of the present disclosure, provided is the compound represented by general formula (III) or the pharmaceutically acceptable salt thereof, wherein each R¹ is identical or different and is independently selected from C₁₋₆ alkoxy; p is 2; X is O or CH₂; each R^{c} and R^{d} is identical or different and is independently a hydrogen atom or a fluorine atom; each R^{4a} is a hydrogen atom; n is 3; s is 1 or 2; and L is a chemical bond.

In some embodiments of the present disclosure, provided is the compound represented by general formula (III) or the pharmaceutically acceptable salt thereof, wherein each R¹ is identical or different and is independently selected from C₁₋₆ alkoxy; p is 1 or 2; X is CH₂; each R^{c} and R^{d} is identical or different and is independently a hydrogen atom or a fluorine atom; n is 0; s is 1 or 2; and L is a chemical bond.

In some embodiments of the present disclosure, provided is the compound represented by general formula (IV) or the pharmaceutically acceptable salt thereof, wherein each R¹ is identical or different and is independently selected from the group consisting of a hydrogen atom, hydroxy, halogen, C₁₋₆ alkyl, C₁₋₆ haloalkyl, C₁₋₆ hydroxyalkyl, C₁₋₆ alkoxy, C₁₋₆ haloalkoxy, C₁₋₆ alkoxy C₁₋₆ alkyl and -C(O)OCH₃; p is 1, 2 or 3; X is O or CH₂; each R^{4a} is identical or different and is independently selected from the group consisting of halogen, hydroxy, C₁₋₆ alkyl, C₁₋₆ hydroxyalkyl and C₁₋₆ alkoxy, and n is 1 or 2; or each R^{4a} is a hydrogen atom, and n is 3; s is 1 or 2; and L is a chemical bond or -CH₂-.

In some embodiments of the present disclosure, provided is the compound represented by general formula (IV) or the pharmaceutically acceptable salt thereof, wherein each R¹ is identical or different and is independently selected from the group consisting of a hydrogen atom, halogen, C₁₋₆ alkyl, C₁₋₆ haloalkyl, C₁₋₆ alkoxy, C₁₋₆ haloalkoxy and -NR⁷R⁸; R⁷ and R⁸ are identical or different and are each independently hydrogen atoms or C₁₋₆ alkyl; p is 0, 1, 2 or 3; X is O or CH₂; each R^{4a} is a hydrogen atom; n is 3; s is 1 or 2; and L is a chemical bond.

In some embodiments of the present disclosure, provided is the compound represented by general formula (IV) or the pharmaceutically acceptable salt thereof, wherein each R¹ is identical or different and is independently C₁₋₆ alkoxy; p is 1 or 2; X is CH₂; each R^{4a} is a hydrogen atom; n is 3; s is 1 or 2; and L is a chemical bond.

In some embodiments of the present disclosure, provided is the compound represented by general formula (IV) or the pharmaceutically acceptable salt thereof, wherein each R¹ is identical or different and is independently C₁₋₆ alkoxy; p is 2; X is O or CH₂; each R^{4a} is a hydrogen atom; n is 3; s is 1 or 2; and L is a chemical bond.

In some embodiments of the present disclosure, provided is the compound represented by general formula (IV) or the pharmaceutically acceptable salt thereof, wherein each R¹ is identical or different and is independently C₁₋₆ alkoxy; p is 1 or 2; X is CH₂; n is 0; s is 1 or 2; and L is a chemical bond.

**Table A. Typical compounds of the present disclosure include, but are not limited to:**

| Exa mple No. | Structures and names of compounds |
|---|---|
| 1 | 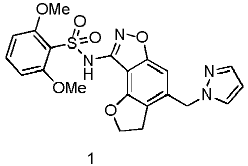 |
| | *N*-(4-((1*H*-pyrazol-1-yl)methyl)-2,3-dihydrobenzofuro[7,6-*d*]isoxazol-8-yl)-2,6-dimethoxybenzenesulfonamide **1** |
| 2 | 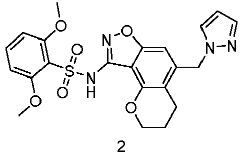 |
| | *N*-(5-((1*H*-pyrazol-1-yl)methyl)-3,4-dihydro-2*H*-chromeno[8,7-*d*]isoxazol-9-yl)-2,6-dimethoxybenzenesulfonamide **2** |
| 3 | 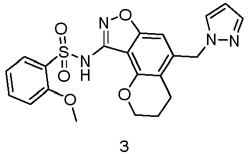 |
| | *N*-(5-((1*H*-pyrazol-1-yl)methyl)-3,4-dihydro-2*H*-chromeno[8,7-*d*]isoxazol-9-yl)-2-methoxybenzenesulfonamide **3** |
| 4 | 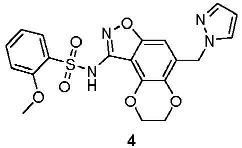 |
| | *N*-(5-((1*H*-pyrazol-1-yl)methyl)-2,3-dihydro-[1,4]dioxino[2',3':5,6]benzo[1,2-*d*]i soxazol-9-yl)-2-methoxybenzenesulfonamide **4** |
| 5 | 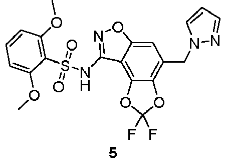 |
| | *N*-(4-((1*H*-pyrazol-1-yl)methyl)-2,2-difluoro-[1,3]dioxolo[4',5':5,6]benzo[1,2-*d*] isoxazol-8-yl)-2,6-dimethoxybenzenesulfonamide **5** |
| 6 | 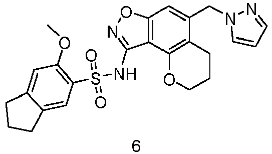 |
| | *N*-(5-((1*H*-pyrazol-1-yl)methyl)-3,4-dihydro-2*H*-chromeno[8,7-*d*]isoxazol-9-yl)-6-methoxy-2,3-dihydro-1*H*-indene-5-sulfonamide **6** |
| 7 | 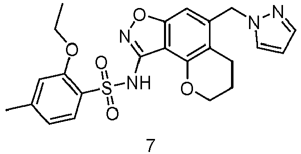 |
| | *N*-(5-((1*H*-pyrazol-1-yl)methyl)-3,4-dihydro-2*H*-chromeno[8,7-*d*]isoxazol-9-yl)-2-ethoxy-4-methylbenzenesulfonamide **7** |
| 8 | 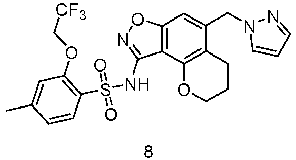 |
| | *N*-(5-((1*H*-pyrazol-1-yl)methyl)-3,4-dihydro-2*H*-chromeno[8,7-*d*]isoxazol-9-yl)-4-methyl-2-(2,2,2-trifluoroethoxy)benzenesulfonamide **8** |
| 9 | 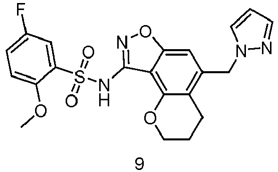 |
| | *N*-(5-((1*H*-pyrazol-1-yl)methyl)-3,4-dihydro-2*H*-chromeno[8,7-*d*]isoxazol-9-yl)-5-fluoro-2-methoxybenzenesulfonamide **9** |
| 10 | 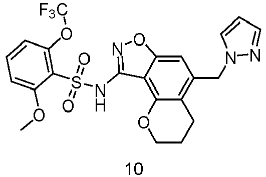 |
| | *N*-(5-((1*H*-pyrazol-1-yl)methyl)-3,4-dihydro-2*H*-chromeno[8,7-*d*]isoxazol-9-yl)-2-methoxy-6-(trifluoromethoxy)benzenesulfonamide **10** |
| 11 | 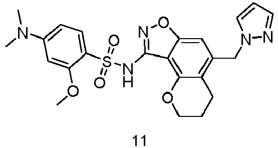 |
| | *N*-(5-((1*H*-pyrazol-1-yl)methyl)-3,4-dihydro-2*H*-chromeno[8,7-*d*]isoxazol-9-yl)-4-(dimethylamino)-2-methoxybenzenesulfonamide **11** |
| 12 | 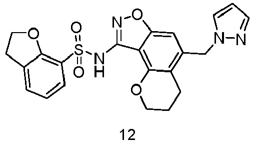 |
| | *N*-(5-((1*H*-pyrazol-1-yl)methyl)-3,4-dihydro-2*H*-chromeno[8,7-*d*]isoxazol-9-yl)-2,3-dihydrobenzofuran-7-sulfonamide **12** |
| 13 | 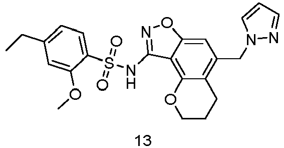 |
| | *N*-(5-((1H-pyrazol-1-yl)methyl)-3,4-dihydro-2*H*-chromeno[8,7-*d*]isoxazol-9-yl)-4-ethyl-2-methoxybenzenesulfonamide **13** |
| 14 | 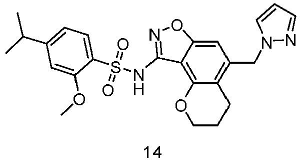 |
| | *N*-(5-((1*H*-pyrazol-1-yl)methyl)-3,4-dihydro-2*H*-chromeno[8,7-*d*]isoxazol-9-yl)-4-isopropyl-2-methoxybenzenesulfonamide **14** |
| 15 | 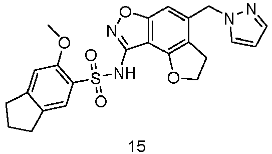 |
| | *N*-(4-((1*H*-pyrazol-1-yl)methyl)-2,3-dihydrobenzofuro[7,6-*d*]isoxazol-8-yl)-6-m ethoxy-2,3-dihydro-1*H*-indene-5-sulfonamide **15** |
| 16 | 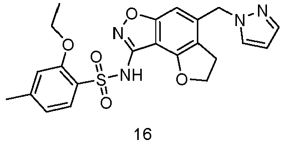 |
| | *N*-(4-((1*H*-pyrazol-1-yl)methyl)-2,3-dihydrobenzofuro[7,6-*d*]isoxazol-8-yl)-2-et hoxy-4-methylbenzenesulfonamide **16** |
| 17 | 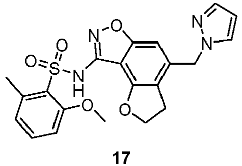 |
| | *N*-(4-((1*H*-pyrazol-1-yl)methyl)-2,3-dihydrobenzofuro[7,6-*d*]isoxazol-8-yl)-2-m ethoxy-6-methylbenzenesulfonamide **17** |
| 18 | 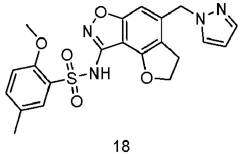 |
| | *N*-(4-((1*H*-pyrazol-1-yl)methyl)-2,3-dihydrobenzofuro[7,6-*d*]isoxazol-8-yl)-2-m ethoxy-5-methylbenzenesulfonamide 18 |
| 19 | 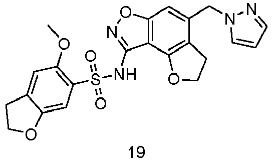 |
| | *N*-(4-((1*H*-pyrazol-1-yl)methyl)-2,3-dihydrobenzofuro[7,6-*d*]isoxazol-8-yl)-5-m ethoxy-2,3-dihydrobenzofuran-6-sulfonamide **19** |
| 20 | 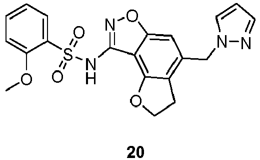 |
| | *N*-(4-((1*H*-pyrazol-1-yl)methyl)-2,3-dihydrobenzofuro[7,6-*d*]isoxazol-8-yl)-2-m ethoxybenzenesulfonamide **20** |
| 21 | 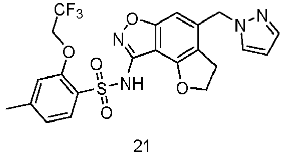 |
| | *N*-(4-((1*H*-pyrazol-1-yl)methyl)-2,3-dihydrobenzofuro[7,6-*d*]isoxazol-8-yl)-4-m ethyl-2-(2,2,2-trifluoroethoxy)benzenesulfonamide **21** |
| 22 | 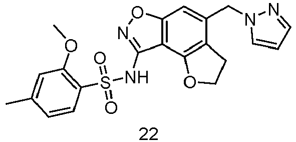 |
| | *N*-(4-((1*H*-pyrazol-1-yl)methyl)-2,3-dihydrobenzofuro[7,6-*d*]isoxazol-8-yl)-2-m ethoxy-4-methylbenzenesulfonamide **22** |
| 23 | 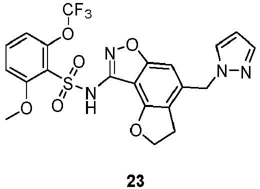 |
| | *N*-(4-((1*H*-pyrazol-1-yl)methyl)-2,3-dihydrobenzofuro[7,6-*d*]isoxazol-8-yl)-2-m ethoxy-6-(trifluoromethoxy)benzenesulfonamide **23** |
| 24 | 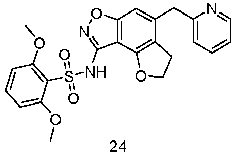 |
| | 2,6-dimethoxy-*N*-(4-(pyridin-2-ylmethyl)-2,3-dihydrobenzofuro[7,6-*d*]isoxazol-8-yl)benzenesulfonamide **24** |
| 24c | 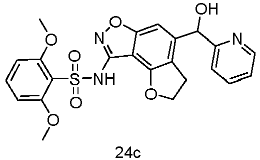 |
| | (±)-*N*-(4-(hydroxy(pyridin-2-yl)methyl)-2,3-dihydrobenzofuro[7,6-*d*]isoxazol-8-yl)-2,6-dimethoxybenzenesulfonamide **24c** |
| | 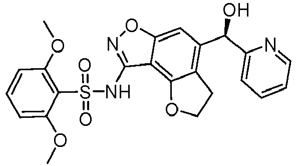 |
| | (*R*)-N-(4-(hydroxy(pyridin-2-yl)methyl)-2,3-dihydrobenzofuro[7,6-*d*]isoxazol-8-yl)-2,6-dimethoxybenzenesulfonamide |
| | 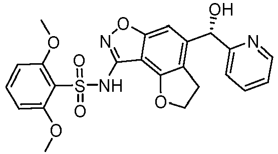 |
| | (*S*)-*N*-(4-(hydroxy(pyridin-2-yl)methyl)-2,3-dihydrobenzofuro[7,6-*d*]isoxazol-8-yl)-2,6-dimethoxybenzenesulfonamide |
| 24d | 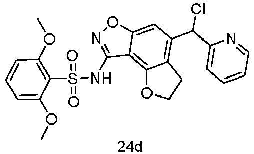 |
| | (±)-*N*-(4-(chloro(pyridin-2-yl)methyl)-2,3-dihydrobenzofuro[7,6-*d*]isoxazol-8-yl )-2,6-dimethoxybenzenesulfonamide **24d** |
| | 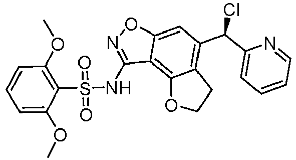 |
| | (*R*)-*N*-(4-(chloro(pyridin-2-yl)methyl)-2,3-dihydrobenzofuro[7,6-*d*]isoxazol-8-yl )-2,6-dimethoxybenzenesulfonamide |
| | 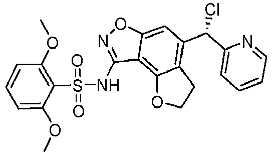 |
| | (*S*)-*N*-(4-(chloro(pyridin-2-yl)methyl)-2,3-dihydrobenzofuro[7,6-*d*]isoxazol-8-yl )-2,6-dimethoxybenzenesulfonamide |
| 25 | 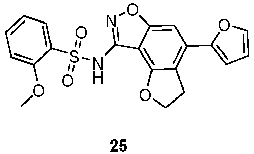 |
| | *N*-(4-(furan-2-yl)-2,3-dihydrobenzofuro[7,6-*d*]isoxazol-8-yl)-2-methoxybenzene sulfonamide **25** |
| 26 | 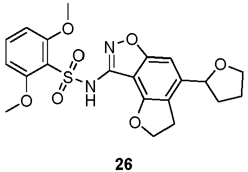 |
| | 2,6-dimethoxy-*N*-(4-(tetrahydrofuran-2-yl)-2,3-dihydrobenzofuro[7,6-*d*]isoxazol -8-yl)benzenesulfonamide **26** |
| 26-1 | 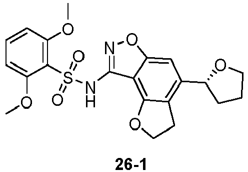 |
| | (*R*)-2,6-dimethoxy-*N*-(4-(tetrahydrofuran-2-yl)-2,3-dihydrobenzofuro[7,6-*d*]isox azol-8-yl)benzenesulfonamide **26-1** |
| 26-2 | 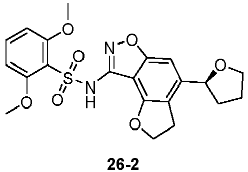 |
| | (*S*)-2,6-dimethoxy-*N*-(4-(tetrahydrofuran-2-yl)-2,3-dihydrobenzofuro[7,6-*d*]isox azol-8-yl)benzenesulfonamide **26-2** |

Another aspect of the present disclosure relates to a compound represented by general formula (IA) or a salt thereof: 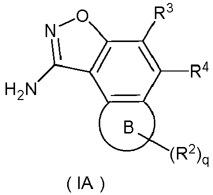 wherein:
ring B, R², R³, R⁴ and q are as defined in general formula (I).

Another aspect of the present disclosure relates to a compound represented by general formula (IIA) or a salt thereof: 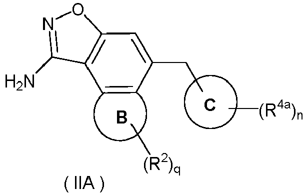 wherein:
ring B, ring C, R², R^{4a}, q and n are as defined in general formula (II).

Another aspect of the present disclosure relates to a compound represented by general formula (IiA) or a salt thereof: 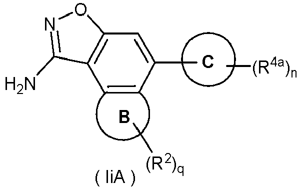 wherein:
ring B, ring C, R², R^{4a}, q and n are as defined in general formula (Ii).

Another aspect of the present disclosure relates to a compound represented by general formula (IIIA) or a salt thereof: 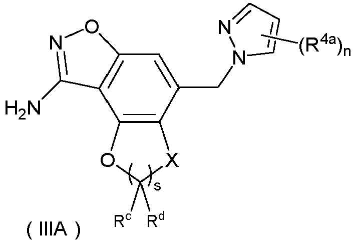 wherein:
X, R^{4a}, R^{c}, R^{d}, n and s are as defined in general formula (III).

Another aspect of the present disclosure relates to a compound represented by general formula (IVA) or a salt thereof: 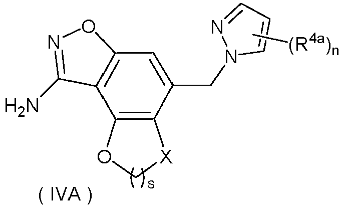 wherein:
X, R^{4a}, n and s are as defined in general formula (IV).

Another aspect of the present disclosure relates to a compound represented by general formula (IA') or a salt thereof: 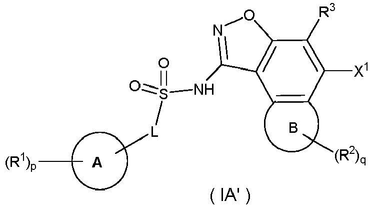 wherein:
X¹ is a halogen, preferably bromine;
ring A, ring B, R¹, R², R³, L, p and q are as defined in general formula (I).

Another aspect of the present disclosure relates to a compound represented by general formula (IIA') or a salt thereof: 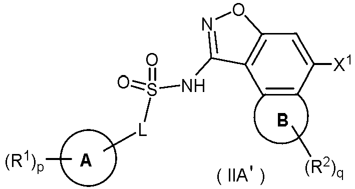 wherein:
X¹ is a halogen, preferably bromine;
ring A, ring B, R¹, R², L, p and q are as defined in general formula (II).

Another aspect of the present disclosure relates to a compound represented by general formula (IIIA') or a salt thereof: 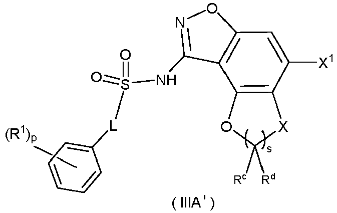 wherein:
X¹ is a halogen, preferably bromine;
R¹, R^{c}, R^{d}, s, p, X and L are as defined in general formula (III).

Another aspect of the present disclosure relates to a compound represented by general formula (IVA') or a salt thereof: 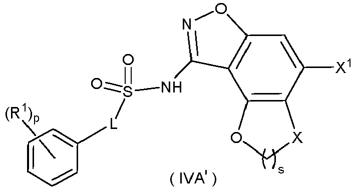 wherein:
X¹ is a halogen, preferably bromine;
R¹, s, p, X and L are as defined in general formula (IV).

**Table B. Typical intermediate compounds of the present disclosure include, but are not limited to:**

| Compou nd No. | Structures and names of compounds |
|---|---|
| 1n | 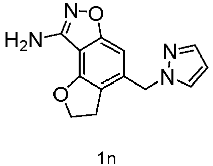 |
| | 4-((1*H*-pyrazol-1-yl)methyl)-2,3-dihydrobenzofuro[7,6-*d*]isoxazol-8-amine **In** |
| 2g | 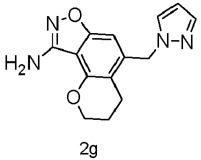 |
| | 5-((1*H*-pyrazol-1-yl)methyl)-3 ,4-dihydro-2*H*-chromeno[8,7-*d*]isoxazol-9-am ine **2g** |
| 4h | 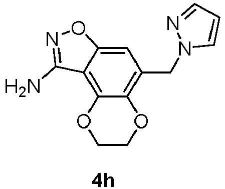 |
| | 5-((1*H*-pyrazol-1-yl)methyl)-2,3-dihydro-[1,4]dioxino[2',3':5,6]benzo[1,2-*d*] isoxazol-9-amine **4h** |
| Sf | 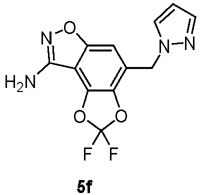 |
| | 4-((1*H*-pyrazol-1-yl)methyl)-2,2-difluoro-[1,3]dioxolo[4',5':5,6]benzo[1,2-*d*] isoxazol-8-amine **Sf** |
| 24a | 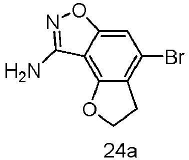 |
| | 4-bromo-2,3-dihydrobenzofuro[7,6-*d*]isoxazol-8-amine **24a** |
| 24b | 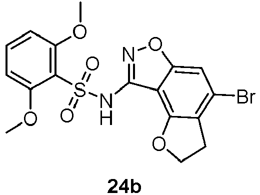 |
| | *N*-(4-bromo-2,3-dihydrobenzofuro[7,6-*d*]isoxazol-8-yl)-2,6-dimethoxybenze nesulfonamide **24b** |
| 25c | 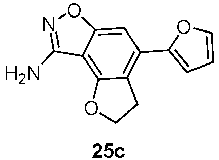 |
| | 4-(furan-2-yl)-2,3-dihydrobenzofuro[7,6-*d*]isoxazol-8-amine **25c** |

Another aspect of the present disclosure relates to a method for preparing a compound represented by general formula (I) or a pharmaceutically acceptable salt thereof, the method comprising the following step: 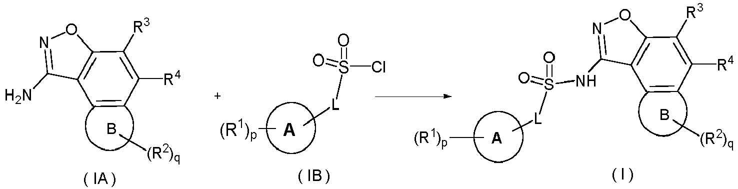 reacting a compound represented by general formula (IA) or a salt thereof with a compound represented by general formula (IB) or a salt thereof to give the compound represented by general formula (I) or the pharmaceutically acceptable salt thereof, wherein:
ring A, ring B, L, R¹ to R⁴, p and q are as defined in general formula (I).

Another aspect of the present disclosure relates to a method for preparing a compound represented by general formula (II) or a pharmaceutically acceptable salt thereof, the method comprising the following step: 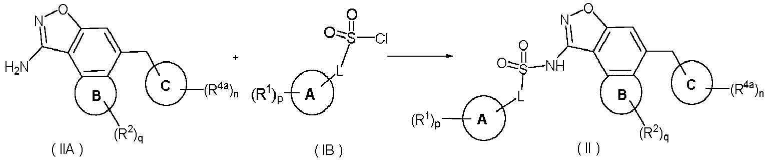 reacting a compound represented by general formula (IIA) or a salt thereof with a compound represented by general formula (IB) or a salt thereof to give the compound represented by general formula (II) or the pharmaceutically acceptable salt thereof, wherein:
ring A, ring B, ring C, L, R¹, R², R^{4a}, p, q and n are as defined in general formula (II).

Another aspect of the present disclosure relates to a method for preparing a compound represented by general formula (Ii) or a pharmaceutically acceptable salt thereof, the method comprising the following step: 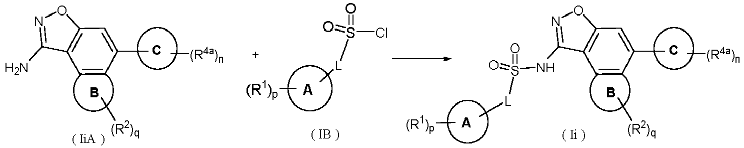 reacting a compound represented by general formula (IiA) or a salt thereof with a compound represented by general formula (IB) or a salt thereof to give the compound represented by general formula (Ii) or the pharmaceutically acceptable salt thereof, wherein:
ring A, ring B, ring C, L, R¹, R², R^{4a}, p, q and n are as defined in general formula (Ii).

Another aspect of the present disclosure relates to a method for preparing a compound represented by general formula (III) or a pharmaceutically acceptable salt thereof, the method comprising the following step: 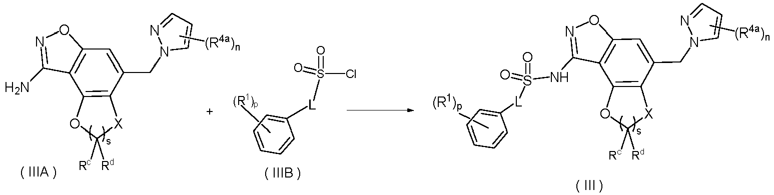 reacting a compound represented by general formula (IIIA) or a salt thereof with a compound represented by general formula (IIIB) or a salt thereof to give the compound represented by general formula (III) or the pharmaceutically acceptable salt thereof, wherein:
L, X, R¹, R^{4a}, R^{c}, R^{d}, p, n and s are as defined in general formula (III).

Another aspect of the present disclosure relates to a method for preparing a compound represented by general formula (IV) or a pharmaceutically acceptable salt thereof, the method comprising the following step: 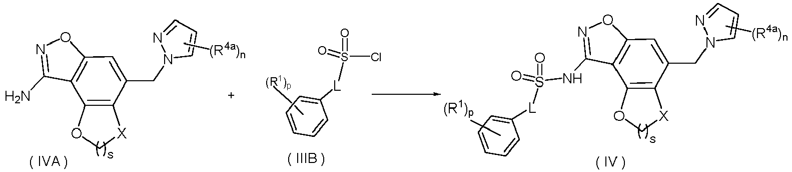 reacting a compound represented by general formula (IVA) or a salt thereof with a compound represented by general formula (IIIB) or a salt thereof to give the compound represented by general formula (IV) or the pharmaceutically acceptable salt thereof, wherein:
X, L, R¹, R^{4a}, p, n and s are as defined in general formula (IV).

Another aspect of the present disclosure relates to a method for preparing a compound represented by general formula (Ii) or a pharmaceutically acceptable salt thereof, the method comprising the following step: 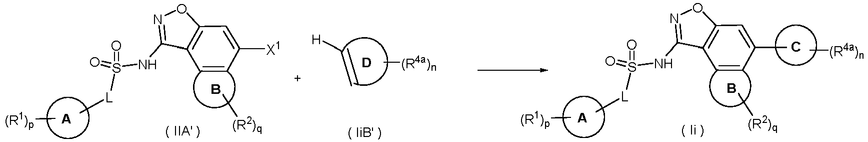 conducting a coupling reaction of a compound represented by general formula (IIA') or a salt thereof with a compound represented by general formula (IiB') or a salt thereof to give the compound represented by general formula (Ii) or the pharmaceutically acceptable salt thereof, wherein:
ring D is 3- to 8-membered heterocyclyl containing at least one intra-ring double bond, preferably 5- or 6-membered heterocyclyl containing at least one intra-ring double bond, and more preferably 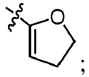
ring C is 3- to 8-membered heterocyclyl, preferably 5- or 6-membered heterocyclyl, and
more preferably 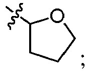
X¹ is a halogen, preferably bromine;
ring A, ring B, L, R¹, R², R^{4a}, p, q and n are as defined in general formula (Ii).

Another aspect of the present disclosure relates to a pharmaceutical composition comprising the compound represented by general formula (I), general formula (II), general formula (Ii), general formula (III) or general formula (IV) of the present disclosure or a compound from Table A or a pharmaceutically acceptable salt thereof, and one or more pharmaceutically acceptable carriers, diluents or excipients.

The present disclosure further relates to use of the compound represented by general formula (I), general formula (II), general formula (Ii), general formula (III) or general formula (IV) or the compound from Table A or the pharmaceutically acceptable salt thereof or the pharmaceutical composition comprising same in the preparation of a medicament for inhibiting a KAT, wherein the KAT is preferably KAT6, and is more preferably KAT6A and/or KAT6B.

The present disclosure further relates to use of the compound represented by general formula (I), general formula (II), general formula (Ii), general formula (III) or general formula (IV) or the compound from Table A or the pharmaceutically acceptable salt thereof or the pharmaceutical composition comprising same in the preparation of a medicament for treating and/or preventing a KAT-mediated disease, wherein the KAT is preferably KAT6, and is more preferably KAT6A and/or KAT6B.

The present disclosure further relates to use of the compound represented by general formula (I), general formula (II), general formula (Ii), general formula (III) or general formula (IV) or the compound from Table A or the pharmaceutically acceptable salt thereof or the pharmaceutical composition comprising same in the preparation of a medicament for treating and/or preventing a cancer, wherein the cancer is preferably selected from the group consisting of lung cancer (e.g., NCSLC or SCLC), mesothelioma, bone cancer, pancreatic cancer, skin cancer, head and neck cancer, brain cancer, melanoma, anal cancer, liver cancer, breast cancer, fallopian tube cancer, endometrial cancer, cervical cancer, ovarian cancer, vaginal cancer, vulvar cancer, Hodgkin's lymphoma, esophageal cancer, colorectal cancer, small intestine cancer, stomach cancer, thyroid cancer, parathyroid cancer, adrenal cancer, soft tissue sarcoma, penile cancer, testicular cancer, prostate cancer, leukemia, B-cell lymphoma, bladder cancer, urethral cancer, ureter cancer, renal cell carcinoma, renal pelvis cancer, central nervous system tumor (CNS), primary CNS lymphoma, spinal cord tumor, glioblastoma, cerebral glioma, pituitary adenoma and squamous cell carcinoma; preferably breast cancer, prostate cancer, lung cancer (e.g., NCSLC or SCLC), pancreatic cancer, ovarian cancer, cervical cancer, endometrial cancer, bladder cancer, cerebral glioma, B-cell lymphoma, liver cancer and leukemia, wherein the breast cancer is preferably ER⁺ breast cancer or ER⁺/HER2⁻ breast cancer; the lung cancer (e.g., NCSLC or SCLC) is preferably non-small cell lung cancer; the prostate cancer is preferably castration-resistant prostate cancer.

The present disclosure further relates to a method for inhibiting a KAT, comprising administering to a patient in need thereof an inhibitory effective amount of the compound represented by general formula (I), general formula (II), general formula (Ii), general formula (III) or general formula (IV) or the compound from Table A or the pharmaceutically acceptable salt thereof, or the pharmaceutical composition comprising same, wherein the KAT is preferably KAT6, and is more preferably KAT6A and/or KAT6B.

The present disclosure also relates to a method for treating and/or preventing a KAT-mediated disease, comprising administering to a patient in need thereof a therapeutically and/or prophylactically effective amount of the compound represented by general formula (I), general formula (II), general formula (Ii), general formula (III) or general formula (IV) or the compound from Table A or the pharmaceutically acceptable salt thereof or the pharmaceutical composition comprising same, wherein the KAT is preferably KAT6, and is more preferably KAT6A and/or KAT6B.

The present disclosure further relates to a method for treating and/or preventing a cancer, comprising administering to a patient in need thereof a therapeutically and/or prophylactically effective amount of the compound represented by general formula (I), general formula (II), general formula (Ii), general formula (III) or general formula (IV) or the compound from Table A or the pharmaceutically acceptable salt thereof, or the pharmaceutical composition comprising same, wherein the cancer is preferably selected from the group consisting of lung cancer (e.g., NCSLC or SCLC), mesothelioma, bone cancer, pancreatic cancer, skin cancer, head and neck cancer, brain cancer, melanoma, anal cancer, liver cancer, breast cancer, fallopian tube cancer, endometrial cancer, cervical cancer, ovarian cancer, vaginal cancer, vulvar cancer, Hodgkin's lymphoma, esophageal cancer, colorectal cancer, small intestine cancer, stomach cancer, thyroid cancer, parathyroid cancer, adrenal cancer, soft tissue sarcoma, penile cancer, testicular cancer, prostate cancer, leukemia, B-cell lymphoma, bladder cancer, urethral cancer, ureter cancer, renal cell carcinoma, renal pelvis cancer, central nervous system tumor (CNS), primary CNS lymphoma, spinal cord tumor, glioblastoma, cerebral glioma, pituitary adenoma and squamous cell carcinoma; preferably breast cancer, prostate cancer, lung cancer (e.g., NCSLC or SCLC), pancreatic cancer, ovarian cancer, cervical cancer, endometrial cancer, bladder cancer, cerebral glioma, B-cell lymphoma, liver cancer and leukemia, wherein the breast cancer is preferably ER⁺ breast cancer or ER⁺/HER2⁻ breast cancer; the lung cancer (e.g., NCSLC or SCLC) is preferably non-small cell lung cancer; the prostate cancer is preferably castration-resistant prostate cancer.

The present disclosure further relates to a compound represented by general formula (I), general formula (II), general formula (Ii), general formula (III) or general formula (IV) or a compound from Table A or a pharmaceutically acceptable salt thereof, or a pharmaceutical composition comprising same, for use as a medicament.

The present disclosure further relates to a compound represented by general formula (I), general formula (II), general formula (Ii), general formula (III) or general formula (IV) or a compound from Table A or a pharmaceutically acceptable salt thereof, or a pharmaceutical composition comprising same, for use as a medicament for inhibiting a KAT, wherein the KAT is preferably KAT6, and is more preferably KAT6A and/or KAT6B.

The present disclosure further relates to a compound represented by general formula (I), general formula (II), general formula (Ii), general formula (III) or general formula (IV) or a compound from Table A or a pharmaceutically acceptable salt thereof, or a pharmaceutical composition comprising same, for use in inhibiting a KAT, wherein the KAT is preferably KAT6, and is more preferably KAT6A and/or KAT6B.

The present disclosure further relates to a compound represented by general formula (I), general formula (II), general formula (Ii), general formula (III) or general formula (IV) or a compound from Table A or a pharmaceutically acceptable salt thereof, or a pharmaceutical composition comprising same, for use as a KAT inhibitor, wherein the KAT is preferably KAT6, and is more preferably KAT6A and/or KAT6B.

The present disclosure also relates to a compound represented by general formula (I), general formula (II), general formula (Ii), general formula (III) or general formula (IV) or a compound from Table A or a pharmaceutically acceptable salt thereof or a pharmaceutical composition comprising same for use in treating and/or preventing a KAT-mediated disease, wherein the KAT is preferably KAT6, and is more preferably KAT6A and/or KAT6B.

The present disclosure further relates to a compound represented by general formula (I), general formula (II), general formula (Ii), general formula (III) or general formula (IV) or a compound from Table A or a pharmaceutically acceptable salt thereof, or a pharmaceutical composition comprising same, for use in treating and/or preventing a cancer, wherein the cancer is preferably selected from the group consisting of lung cancer (e.g., NCSLC or SCLC), mesothelioma, bone cancer, pancreatic cancer, skin cancer, head and neck cancer, brain cancer, melanoma, anal cancer, liver cancer, breast cancer, fallopian tube cancer, endometrial cancer, cervical cancer, ovarian cancer, vaginal cancer, vulvar cancer, Hodgkin's lymphoma, esophageal cancer, colorectal cancer, small intestine cancer, stomach cancer, thyroid cancer, parathyroid cancer, adrenal cancer, soft tissue sarcoma, penile cancer, testicular cancer, prostate cancer, leukemia, B-cell lymphoma, bladder cancer, urethral cancer, ureter cancer, renal cell carcinoma, renal pelvis cancer, central nervous system tumor (CNS), primary CNS lymphoma, spinal cord tumor, glioblastoma, cerebral glioma, pituitary adenoma and squamous cell carcinoma; preferably breast cancer, prostate cancer, lung cancer (e.g., NCSLC or SCLC), pancreatic cancer, ovarian cancer, cervical cancer, endometrial cancer, bladder cancer, cerebral glioma, B-cell lymphoma, liver cancer and leukemia, wherein the breast cancer is preferably ER⁺ breast cancer or ER⁺/HER2⁻ breast cancer; the lung cancer (e.g., NCSLC or SCLC) is preferably non-small cell lung cancer; the prostate cancer is preferably castration-resistant prostate cancer.

In some embodiments of the present disclosure, the KAT6 is KAT6A and/or KAT6B.

In some embodiments of the present disclosure, the cancer is breast cancer.

In some embodiments of the present disclosure, the breast cancer is ER⁺ breast cancer.

In some embodiments of the present disclosure, the breast cancer is ER⁺/HER2⁻ breast cancer.

In some embodiments of the present disclosure, the breast cancer is locally advanced or metastatic ER⁺/HER2⁻ breast cancer.

In some embodiments of the present disclosure, the lung cancer (e.g., NCSLC or SCLC) is non-small cell lung cancer.

In some embodiments of the present disclosure, the lung cancer (e.g., NCSLC or SCLC) is locally advanced or metastatic non-small cell lung cancer.

In some embodiments of the present disclosure, the prostate cancer is castration-resistant prostate cancer.

In some embodiments of the present disclosure, the prostate cancer is locally advanced or metastatic castration-resistant prostate cancer.

For convenience, certain well-known abbreviations may be used herein, including: estrogen receptor positive (ER⁺), human epidermal growth factor receptor 2 negative (HER2⁻), non-small cell lung cancer (NSCLC), and castration-resistant prostate cancer (CRPC).

The active compound may be formulated into a form suitable for administration by any suitable route, and one or more pharmaceutically acceptable carriers are used to formulate the composition of the present disclosure by conventional methods. Thus, the active compound of the present disclosure may be formulated into a variety of dosage forms for oral administration, administration by injection (e.g., intravenous, intramuscular or subcutaneous), or administration by inhalation or insufflation. The compounds of the present disclosure may also be formulated into a dosage form, such as tablets, hard or soft capsules, aqueous or oily suspensions, emulsions, injections, dispersible powders or granules, suppositories, lozenges or syrups.

As a general guide, the active compound is preferably in the form of a unit dose, or in the form of a single dose that can be self-administered by a patient. The unit dose of the compound or composition of the present disclosure may be in a tablet, capsule, cachet, vial, powder, granule, lozenge, suppository, regenerating powder, or liquid formulation. A suitable unit dose may be 0.1-1000 mg.

The pharmaceutical composition of the present disclosure may comprise, in addition to the active compound, one or more auxiliary materials selected from the group consisting of a filler (diluent), a binder, a wetting agent, a disintegrant, an excipient, and the like. Depending on the method of administration, the composition may comprise 0.1 wt.% to 99 wt.% of the active compound.

The tablet comprises the active ingredient and a non-toxic pharmaceutically acceptable excipient that is used for mixing and is suitable for the preparation of the tablet. Such an excipient may be an inert excipient, a granulating agent, a disintegrant, a binder, and a lubricant. Such a tablet may be uncoated or may be coated by known techniques for masking the taste of the drug or delaying the disintegration and absorption of the drug in the gastrointestinal tract and thus enabling a sustained release of the drug over a longer period.

An oral formulation in a soft gelatin capsule where the active ingredient is mixed with an inert solid diluent or with a water-soluble carrier or oil vehicle may also be provided. An aqueous suspension comprises the active substance and an excipient that is used for mixing and is suitable for the preparation of the aqueous suspension. Such an excipient is a suspending agent, a dispersant, or a wetting agent. The aqueous suspension may also comprise one or more preservatives, one or more colorants, one or more corrigents, and one or more sweeteners.

An oil suspension may be formulated by suspending the active ingredient in a vegetable oil, or in a mineral oil. The oil suspension may comprise a thickening agent. The sweeteners and corrigents described above may be added to provide a palatable formulation. Antioxidants may also be added to preserve the compositions.

The pharmaceutical composition of the present disclosure may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a corrigent, a preservative, and an antioxidant. Such a formulation may also comprise a palliative, a preservative, a colorant, and an antioxidant.

The pharmaceutical composition of the present disclosure may be in the form of a sterile injectable aqueous solution. Acceptable vehicles or solvents that can be used include water, Ringer's solution, and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition of the present disclosure may be in the form of a sterile injectable aqueous or oil suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be used. In addition, fatty acids may also be used to prepare injections.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

The compound of the present disclosure can be administered in the form of dispersible powders and granules that are formulated into aqueous suspensions by adding water. Such a pharmaceutical composition can be prepared by mixing the active ingredient with a dispersant or a wetting agent, a suspending agent, or one or more preservatives. As is well known to those skilled in the art, the dose of the drug administered depends on a variety of factors, including, but not limited to, the activity of the particular compound used, the age of the patient, the body weight of the patient, the health condition of the patient, the behavior of the patient, the diet of the patient, the time of administration, the route of administration, the rate of excretion, the combination of drugs, the severity of the disease, and the like. In addition, the optimal treatment regimen, such as the mode of administration, the daily dose of the compound, or the type of pharmaceutically acceptable salts, can be verified according to conventional treatment regimens.

### Description of the terms

Unless otherwise stated, the terms used in the specification and claims have the following meanings.

The term "alkyl" refers to a saturated aliphatic hydrocarbon group, which is a straight-chain or branched-chain alkyl group containing 1 to 20 (e.g., 1, 2, 3, 4, 5, 6, 7, 8, 9, 10, 11, 12, 13, 14, 15, 16, 17, 18, 19 or 20) carbon atoms (i.e., C₁₋₂₀ alkyl), preferably 1 to 12 (e.g., 1, 2, 3, 4, 5, 6, 7, 8, 9, 10, 11 and 12) carbon atoms (i.e., C₁₋₂₀ alkyl), and more preferably 1 to 6 carbon atoms (i.e., C₁₋₆ alkyl). Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, and the like. Alkyl may be substituted or unsubstituted, and when it is substituted, it may be substituted at any accessible point of attachment, and the substituent is preferably selected from one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkylene" refers to a saturated straight-chain or branched-chain aliphatic hydrocarbon group, which is a residue derived from its parent alkane by removal of two hydrogen atoms from the same carbon atom or two different carbon atoms and has 1 to 20 (e.g., 1, 2, 3, 4, 5, 6, 7, 8, 9, 10, 11, 12, 13, 14, 15, 16, 17, 18, 19 or 20) carbon atoms (i.e., C₁₋₂₀ alkylene), preferably 1 to 12 carbon atoms (i.e., C₁₋₁₂ alkylene), and more preferably 1 to 6 carbon atoms (i.e., C₁₋₆ alkylene). Non-limiting examples include methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), and the like. Alkylene may be substituted or unsubstituted, and when it is substituted, it may be substituted at any accessible point of attachment, and the substituent is preferably selected from one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "heteroalkylene" means that one or more -CH₂- in alkylene are replaced by one or more selected from the group consisting of N, O, S, S(O) and S(O)₂, wherein the alkyl is as defined above; heteroalkylene may be substituted or unsubstituted, and when it is substituted, the substituent may be substituted at any accessible point of attachment, and the substituent is preferably selected from one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkenyl" refers to an alkyl compound containing at least one carbon-carbon double bond in the molecule, wherein the alkyl is as defined above, and it has 2 to 12 (e.g., 2, 3, 4, 5, 6, 7, 8, 9, 10, 11 or 12) carbon atoms (i.e., C₂₋₁₂ alkenyl). The alkenyl is preferably an alkenyl group having 2 to 6 carbon atoms (i.e., C₂₋₆ alkenyl). Alkenyl may be substituted or unsubstituted, and when it is substituted, the substituent is preferably selected from one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkynyl" refers to an alkyl compound containing at least one carbon-carbon triple bond in the molecule, wherein the alkyl is as defined above, and it has 2 to 12 (e.g., 2, 3, 4, 5, 6, 7, 8, 9, 10, 11 or 12) carbon atoms (i.e., C₂₋₁₂ alkynyl). The alkynyl is preferably an alkynyl group having 2 to 6 carbon atoms (i.e., C₂₋₆ alkynyl). Alkynyl may be substituted or unsubstituted, and when it is substituted, the substituent is preferably selected from one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "cycloalkyl" refers to a saturated or partially unsaturated monocyclic or polycyclic hydrocarbon substituent, wherein the cycloalkyl ring contains 3 to 20 (e.g., 3, 4, 5, 6, 7, 8, 9, 10, 11, 12, 13, 14, 15, 16, 17, 18, 19 or 20) carbon atoms (i.e., 3- to 20-membered cycloalkyl), preferably 3 to 12 (e.g., 3, 4, 5, 6, 7, 8, 9, 10, 11 or 12) carbon atoms (i.e., 3- to 12-membered cycloalkyl), preferably 3 to 8 (e.g., 3, 4, 5, 6, 7 and 8) carbon atoms (i.e., 3- to 8-membered cycloalkyl), further preferably 4 to 7 (e.g., 4, 5, 6 and 7) carbon atoms (i.e., 4- to 7-membered cycloalkyl), and more preferably 3 to 6 (e., 3, 4, 5 and 6) carbon atoms (i.e., 3- to 6-membered cycloalkyl). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spirocycloalkyl, fused cycloalkyl, and bridged cycloalkyl.

The term "spirocycloalkyl" refers to a 5- to 20-membered (i.e., 5, 6, 7, 8, 9, 10, 11, 12, 13, 14, 15, 16, 17, 18, 19 or 20 ring atoms, i.e., 5- to 20-membered spirocycloalkyl) polycyclic group in which one carbon atom (referred to as a spiro atom) is shared between monocyclic rings, and it may contain one or more double bonds. It is preferably 6- to 14-membered (i.e., 6- to 14-membered spirocycloalkyl), and is more preferably 7- to 10-membered (e.g., 7-, 8-, 9- or 10-membered, i.e., 7- to 10-membered spirocycloalkyl). According to the number of spiro atoms shared among rings, spirocycloalkyl may be monospirocycloalkyl, bispirocycloalkyl or polyspirocycloalkyl, preferably monospirocycloalkyl and bispirocycloalkyl, and more preferably 3 -membered/5 -membered, 3-membered/6-membered, 4-membered/4-membered, 4-membered/5-membered, 4-membered/6-membered, 5-membered/5-membered or 5-membered/6-membered monospirocycloalkyl. Non-limiting examples of spirocycloalkyl include:

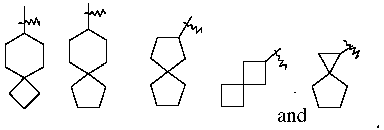

The term "fused cycloalkyl" refers to a 5- to 20-membered (e.g., 5, 6, 7, 8, 9, 10, 11, 12, 13, 14, 15, 16, 17, 18, 19 or 20 ring atoms, i.e., 5- to 20-membered fused cycloalkyl) all-carbon polycyclic group in which each of the rings in the system shares a pair of adjacent carbon atoms with the other rings, wherein one or more of the rings may contain one or more double bonds. It is preferably 6- to 14-membered (i.e., 6- to 14-membered fused cycloalkyl), and is more preferably 7- to 10-membered (e.g., 7-, 8-, 9- or 10-membered, i.e., 7- to 10-membered fused cycloalkyl). According to the number of constituent rings, it may be bicyclic, tricyclic, tetracyclic or polycyclic fused cycloalkyl, preferably bicyclic or tricyclic, and more preferably 3 -membered/4-membered, 3 -membered/5 -membered, 3-membered/6-membered, 4-membered/4-membered, 4-membered/5-membered, 4-membered/6-membered, 5 -membered/4-membered, 5 -membered/5 -membered, 5-membered/6-membered, 6-membered/3-membered, 6-membered/4-membered, 6-membered/5-membered and 6-membered/6-membered bicyclic fused cycloalkyl. Non-limiting examples of fused cycloalkyl include:

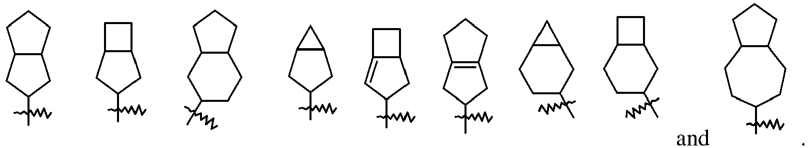

The term "bridged cycloalkyl" refers to a 5- to 20-membered (e.g., 5, 6, 7, 8, 9, 10, 11, 12, 13, 14, 15, 16, 17, 18, 19 or 20 carbon atoms, i.e., 5- to 20-membered bridged cycloalkyl) all-carbon polycyclic group in which any two of the rings share two carbon atoms that are not directly connected, and it may contain one or more double bonds. It is preferably 6- to 14-membered (i.e., 6- to 14-membered bridged cycloalkyl), and is more preferably 7- to 10-membered (e.g., 7-, 8-, 9- or 10-membered, i.e., 7- to 10-membered bridged cycloalkyl). According to the number of constituent rings, it may be bicyclic, tricyclic, tetracyclic or polycyclic bridged cycloalkyl, preferably bicyclic, tricyclic or tetracyclic, and more preferably bicyclic or tricyclic. Non-limiting examples of bridged cycloalkyl include:

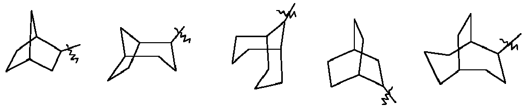

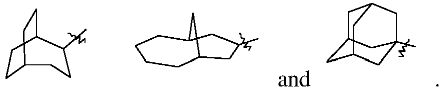

The cycloalkyl ring includes those in which the cycloalkyl described above (including monocyclic cycloalkyl, spirocycloalkyl, fused cycloalkyl and bridged cycloalkyl) is fused to an aryl, heteroaryl or heterocycloalkyl ring, wherein the ring attached to the parent structure is cycloalkyl; non-limiting examples include 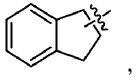 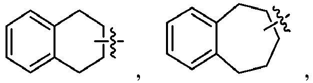 and the like; it is preferably 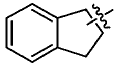 or

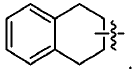

Cycloalkyl may be substituted or unsubstituted, and when it is substituted, it may be substituted at any accessible point of attachment, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkoxy" refers to -O-(alkyl), wherein the alkyl is as defined above. Non-limiting examples of alkoxy include methoxy, ethoxy, propoxy, and butoxy. Alkoxy may be optionally substituted or unsubstituted, and when it is substituted, the substituent is preferably one or more of the following groups; it is independently selected from the group consisting of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heterocyclyl" refers to a saturated or partially unsaturated monocyclic or polycyclic substituent containing 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl), wherein one or more ring atoms are heteroatoms selected from the group consisting of nitrogen, oxygen and sulfur; the sulfur may optionally be substituted with oxo (i.e., to form sulfoxide or sulfone), but does not include a cyclic moiety of -O-O-, -O-S- or -S-S-, and the other ring atoms are carbon. Preferably, it contains 3 to 12 (e.g., 3, 4, 5, 6, 7, 8, 9, 10, 11 and 12) ring atoms (i.e., 3- to 12-membered heterocyclyl), 1-4 (e.g., 1, 2, 3 and 4) of which are heteroatoms; more preferably, it contains 3 to 8 (e.g., 3, 4, 5, 6, 7 and 8) ring atoms (i.e., 3- to 8-membered heterocyclyl), 1-3 (e.g., 1, 2 and 3) of which are heteroatoms; further preferably, it contains 4 to 7 (e.g., 4, 5, 6 and 7) ring atoms (i.e., 4- to 7-membered heterocyclyl), 1-3 (e.g., 1, 2 and 3) of which are heteroatoms; more preferably, it contains 3 to 6 (e.g., 3, 4, 5 and 6) ring atoms (i.e., 3- to 6-membered heterocyclyl), 1-3 (e.g., 1, 2 and 3) of which are heteroatoms; most preferably, it contains 5 or 6 ring atoms (i.e., 5- or 6-membered heterocyclyl), 1-2 (e.g., 1 or 2) of which are heteroatoms. Non-limiting examples of monocyclic heterocyclyl include pyrrolidinyl, tetrahydropyranyl, 1,2,3,6-tetrahydropyridinyl, piperidinyl, piperazinyl, morpholinyl, thiomorpholinyl, homopiperazinyl, and the like. Polycyclic heterocyclyl includes spiroheterocyclyl, fused heterocyclyl, and bridged heterocyclyl.

The term "spiroheterocyclyl" refers to a 5- to 20-membered (e.g., 5, 6, 7, 8, 9, 10, 11, 12, 13, 14, 15, 16, 17, 18, 19 or 20 ring atoms, i.e., 5- to 20-membered spiroheterocyclyl) polycyclic heterocyclyl group in which one atom (referred to as a spiro atom) is shared between monocyclic rings, wherein one or more of the ring atoms are heteroatoms selected from the group consisting of nitrogen, oxygen and sulfur; the sulfur may optionally be substituted with oxo (i.e., to form sulfoxide or sulfone), and the other ring atoms are carbon. It may contain one or more double bonds. It is preferably 6- to 14-membered (i.e., 6- to 14-membered spiroheterocyclyl), and is more preferably 7- to 10-membered (e.g., 7-, 8-, 9- or 10-membered, i.e., 7- to 10-membered spiroheterocyclyl). According to the number of spiro atoms shared among rings, spiroheterocyclyl may be monospiroheterocyclyl, bispiroheterocyclyl or polyspiroheterocyclyl, preferably, monospiroheterocyclyl and bispiroheterocyclyl, and more preferably 3-membered/5-membered, 3-membered/6-membered, 4-membered/4-membered, 4-membered/5-membered, 4-membered/6-membered, 5-membered/5-membered or 5-membered/6-membered monospiroheterocyclyl. Non-limiting examples of spiroheterocyclyl include:

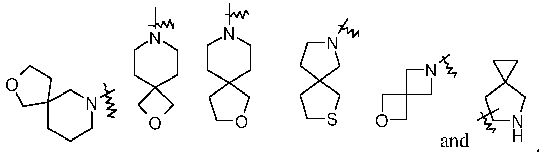

The term "fused heterocyclyl" refers to a 5- to 20-membered (e.g., 5, 6, 7, 8, 9, 10, 11, 12, 13, 14, 15, 16, 17, 18, 19 or 20 ring atoms, i.e., 5- to 20-membered fused heterocyclyl) polycyclic heterocyclyl group in which each of the rings in the system shares a pair of adjacent atoms with the other rings, wherein one or more of the rings may contain one or more double bonds, and one or more of the ring atoms are heteroatoms selected from the group consisting of nitrogen, oxygen and sulfur; the sulfur may optionally be substituted with oxo (i.e., to form sulfoxide or sulfone), and the other ring atoms are carbon. It is preferably 6- to 14-membered (i.e., 6- to 14-membered fused heterocyclyl), and is more preferably 7- to 10-membered (e.g., 7-, 8-, 9- or 10-membered, i.e., 7- to 10-membered fused heterocyclyl). According to the number of constituent rings, it may be bicyclic, tricyclic, tetracyclic or polycyclic fused heterocyclyl, preferably bicyclic or tricyclic, and more preferably 3 -membered/4-membered, 3 -membered/5 -membered, 3-membered/6-membered, 4-membered/4-membered, 4-membered/5-membered, 4-membered/6-membered, 5 -membered/4-membered, 5 -membered/5 -membered, 5-membered/6-membered, 6-membered/3-membered, 6-membered/4-membered, 6-membered/5-membered and 6-membered/6-membered bicyclic fused heterocyclyl. Non-limiting examples of fused heterocyclyl include:

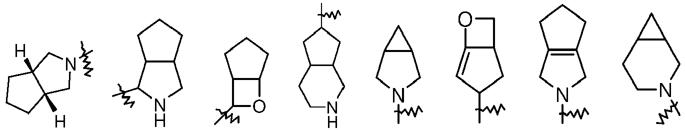

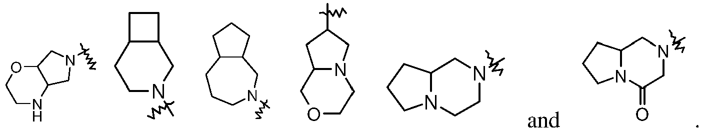

The term "bridged heterocyclyl" refers to a 5- to 14-membered (e.g., 5, 6, 7, 8, 9, 10, 11, 12, 13 or 14 ring atoms, i.e., 5- to 14-membered bridged heterocyclyl) polycyclic heterocyclyl group in which any two of the rings share two atoms that are not directly connected, and it may contain one or more double bonds, wherein one or more ring atoms are heteroatoms selected from the group consisting of nitrogen, oxygen and sulfur; the sulfur may optionally be substituted with oxo (i.e., to form sulfoxide or sulfone), and the other ring atoms are carbon. It is preferably 6- to 14-membered (i.e., 6-to 14-membered bridged heterocyclyl), and is more preferably 7- to 10-membered (e.g., 7-, 8-, 9- or 10-membered, i.e., 7- to 10-membered bridged heterocyclyl). According to the number of constituent rings, it may be bicyclic, tricyclic, tetracyclic or polycyclic bridged heterocyclyl, preferably bicyclic, tricyclic or tetracyclic, and more preferably bicyclic or tricyclic. Non-limiting examples of bridged heterocyclyl include:

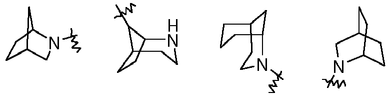

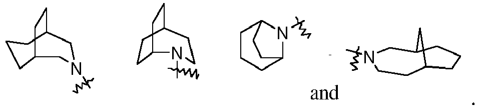

The heterocyclyl ring includes those in which the heterocyclyl described above (including monocyclic heterocyclyl, spiroheterocyclyl, fused heterocyclyl and bridged heterocyclyl) is fused to an aryl, heteroaryl or cycloalkyl ring, wherein the ring attached to the parent structure is heterocyclyl; its non-limiting examples include: 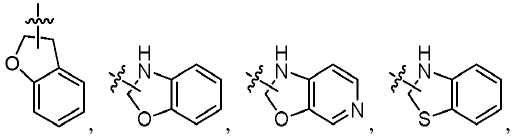 and the like. Heterocyclyl may be substituted or unsubstituted, and when it is substituted, it may be substituted at any accessible point of attachment, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "aryl" refers to a 6- to 14-membered (e.g., 6, 7, 8, 9, 10, 11, 12, 13 or 14 ring atoms, i.e., 6- to 14-membered aryl) all-carbon monocyclic or fused polycyclic (fused polycyclic describes rings that share adjacent pairs of carbon atoms) group having a conjugated π-electron system. Aryl is preferably 6- to 10-membered (i.e., 6- to 10-membered aryl), such as phenyl and naphthyl. The aryl ring includes those in which the aryl ring described above is fused to a heteroaryl, heterocyclyl or cycloalkyl ring, wherein the ring attached to the parent structure is an aryl ring; its non-limiting examples include: 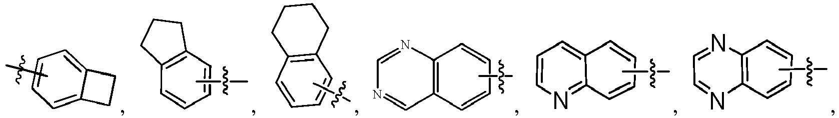 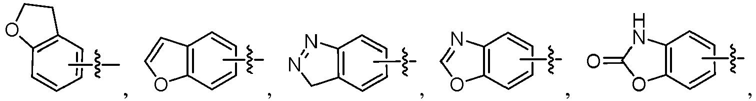 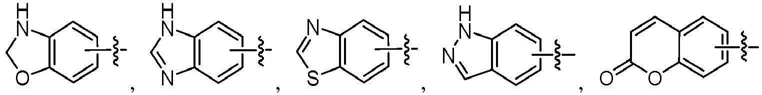 and

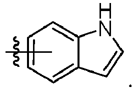

Aryl may be substituted or unsubstituted, and when it is substituted, it may be substituted at any accessible point of attachment, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heteroaryl" refers to a heteroaromatic system containing 1 to 4 (e.g., 1, 2, 3 and 4) heteroatoms, and 5 to 14 ring atoms (e.g., 5, 6, 7, 8, 9, 10, 11, 12, 13 or 14 ring atoms, i.e., 5- to 14-membered heteroaryl), wherein the heteroatoms are selected from the group consisting of oxygen, sulfur and nitrogen. Heteroaryl is preferably 5- to 10-membered (e.g., 5-, 6-, 7-, 8-, 9- or 10-membered, i.e., 5- to 10-membered heteroaryl), and is more preferably 5-membered or 6-membered (i.e., 5- or 6-membered heteroaryl), e.g., furanyl, thienyl, pyridinyl, pyrrolyl, N-alkylpyrrolyl, pyrimidinyl, pyrazinyl, pyridazinyl, imidazolyl, pyrazolyl, triazolyl or tetrazolyl. The heteroaryl ring includes those in which the heteroaryl described above is fused to an aryl, heterocyclyl or cycloalkyl ring, wherein the ring attached to the parent structure is a heteroaryl ring; its non-limiting examples include: 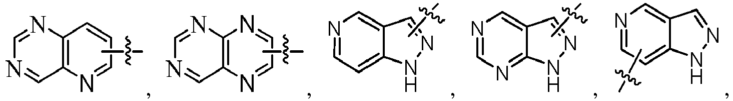 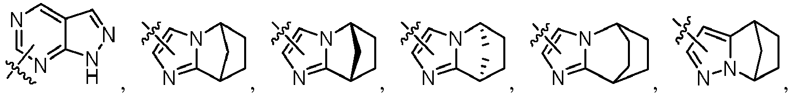 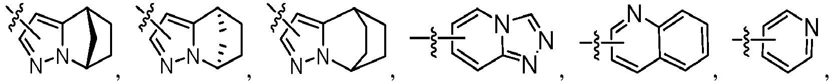 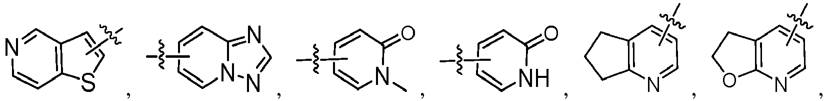 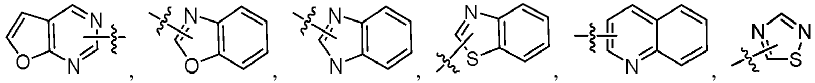 and

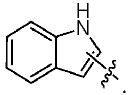

Heteroaryl may be substituted or unsubstituted, and when it is substituted, it may be substituted at any accessible point of attachment, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The cycloalkyl, heterocyclyl, aryl and heteroaryl described above include residues derived from their parent structures by removal of one hydrogen atom from a ring atom, or residues derived from their parent structures by removal of two hydrogen atoms from the same ring atom or two different ring atoms, i.e., "divalent cycloalkyl", "divalent heterocyclyl" (e.g., 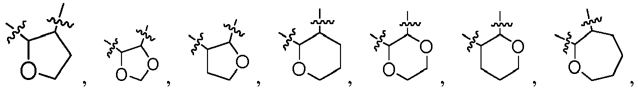 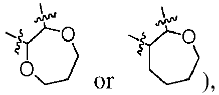 "arylene" and "heteroarylene".

The term "cycloalkylalkyl" refers to an alkyl group substituted with one or more cycloalkyl groups, wherein the cycloalkyl and alkyl are as defined above.

The term "heterocyclylalkyl" refers to an alkyl group substituted with one or more heterocyclyl groups, wherein the heterocyclyl and alkyl are as defined above.

The term "heteroarylalkyl" refers to an alkyl group substituted with one or more heteroaryl groups, wherein the heteroaryl and alkyl are as defined above.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "alkylthio" refers to alkyl-S-, wherein the alkyl is as defined above.

The term "haloalkyl" refers to an alkyl group substituted with one or more halogens, wherein the alkyl is as defined above.

The term "haloalkoxy" refers to an alkoxy group substituted with one or more halogens, wherein the alkoxy is as defined above.

The term "alkoxyalkyl" refers to an alkyl group substituted with one or more alkoxy groups, wherein the alkyl and alkoxy are as defined above.

The term "hydroxyalkyl" refers to an alkyl group substituted with one or more hydroxy groups, wherein the alkyl is as defined above.

The term "halogen" refers to fluorine, chlorine, bromine or iodine.

The term "hydroxy" refers to -OH.

The term "sulfhydryl" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "aldehyde" refers to -C(O)H.

The term "carboxyl" refers to -C(O)OH.

The term "carboxylate group" refers to -C(O)O(alkyl), -C(O)O(cycloalkyl)(alkyl)C(O)O- or (cycloalkyl)C(O)O-, wherein the alkyl and cycloalkyl are as defined above.

In another aspect, the compounds of the present disclosure may exist in specific geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof are included within the scope of the present disclosure. Optically active (R)- and (S)-enantiomers and D- and L-isomers can be prepared by chiral synthesis, chiral reagents or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines).

In the chemical structure of the compound of the present disclosure, a bond " 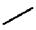 " represents an unspecified configuration; that is, if chiral isomers exist in the chemical structure, the bond " 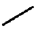 " may be " 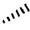 " or " 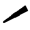 ", or contains both the configurations of " 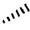 " and " 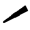 ". In the chemical structure of the compound described herein, a bond " 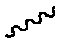 " is not specified with a configuration; that is, it may be in a Z configuration or an E configuration, or contains both configurations.

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, proton tautomers (also known as proton transfer tautomers) include interconversion via proton migration, such as keto-enol and imine-enamine isomerization. An example of a lactam-lactim equilibrium is present between A and B as shown below.

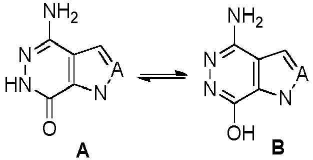

All compounds in the present disclosure can be drawn as form A or form B. All tautomeric forms are within the scope of the present disclosure. The names of the compounds do not exclude any tautomers.

The present disclosure further includes isotopically labeled compounds that are identical to those recited herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl.

The compound of the present disclosure may contain an unnatural proportion of atomic isotopes at one or more of the atoms that constitute the compound. For example, the compound may be labeled with a radioisotope such as tritium (³H). Hydrogen may be substituted with deuterium to form a deuterated drug, and the bond formed by deuterium and carbon is firmer than a bond formed by common hydrogen and carbon. The deuterated drug has the advantages of reduced toxic and side effects, increased drug stability, enhanced efficacy, prolonged drug biological half-life period and the like compared with an undeuterized drug. All isotopic variations of the compounds of the present disclosure, whether radioactive or not, are intended to be included within the scope of the present disclosure.

Furthermore, substitution with heavier isotopes such as deuterium (i.e., ²H) may provide certain therapeutic advantages (e.g., increased *in vivo* half-life or reduced dose requirement) resulting from greater metabolic stability and hence may be preferred in some circumstances in which deuterium substitution may be partial or complete, wherein partial deuterium substitution refers to substitution of at least one hydrogen with at least one deuterium.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure further includes various deuterated forms of the compound of formula (I). Each available hydrogen atom connected to a carbon atom may be independently replaced by a deuterium atom. Those skilled in the art are able to synthesize the deuterated forms of the compound of formula (I) according to the relevant literature. Commercially available deuterated starting materials can be used in preparing the deuterated forms of the compound of formula (I), or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like.

"Optional" or "optionally" means that the event or circumstance subsequently described may, but does not necessarily, occur, and that the description includes instances where the event or circumstance occurs or does not occur. For example, "a heterocyclyl group optionally substituted with alkyl" means that the alkyl may, but does not necessarily, exist, and that the description includes instances where the heterocyclyl group is or is not substituted with the alkyl.

"Substituted" means that one or more, preferably 1-5, more preferably 1-3 hydrogen atoms in the group are independently substituted with a corresponding number of substituents. Those skilled in the art are able to determine (experimentally or theoretically) possible or impossible substitution without undue effort. For example, it may be unstable when an amino or hydroxy having free hydrogen is bound to a carbon atom having an unsaturated (e.g., olefinic) bond.

"Pharmaceutical composition" refers to a mixture containing one or more of the compounds described herein or a physiologically/pharmaceutically acceptable salt or pro-drug thereof, and other chemical components, and other components, for example, physiologically/pharmaceutically acceptable carriers and excipients. The pharmaceutical composition is intended to promote the administration to an organism, which facilitates the absorption of the active ingredient, thereby exerting biological activity.

"Pharmaceutically acceptable salt" refers to the salts of the compound of the present disclosure, which are safe and effective for use in the body of a mammal and possess the requisite biological activities. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs or pharmacologically active agents, the term "therapeutically effective amount", "inhibitory effective amount" or "prophylactically effective amount" refers to an amount of the drug or agent sufficient to achieve, or partially achieve, the desired effect. The determination of the effective amount varies from person to person. It depends on the age and general condition of a subject, as well as the particular active substance used. The appropriate effective amount in a case may be determined by those skilled in the art in the light of routine tests.

The term "pharmaceutically acceptable" used herein means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use. As used herein, the singular forms "a", "an" and "the" include plural references and vice versa, unless otherwise clearly defined in the context.

When the term "about" is applied to parameters such as pH, concentration and temperature, it means that the parameter may vary by ±10%, and sometimes more preferably within ±5%. As will be appreciated by those skilled in the art, when the parameters are not critical, the numbers are generally given for illustrative purposes only and are not intended to be limiting.

### Synthetic methods of the compounds of the present disclosure

To achieve the purpose of the present disclosure, the following technical schemes are adopted in the present disclosure:

### Scheme 1

A method for preparing the compound represented by general formula (I) or the pharmaceutically acceptable salt thereof of the present disclosure, comprising the following step: 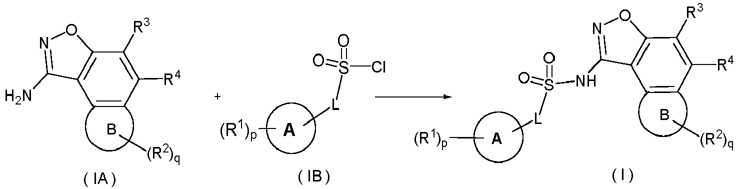 conducting a nucleophilic substitution reaction of a compound represented by general formula (IA) or a salt thereof with a compound represented by general formula (IB) or a salt thereof in the presence of an alkali, optionally under microwave conditions, to give the compound represented by general formula (I) or the pharmaceutically acceptable salt thereof,
wherein:
L, ring A, ring B, R¹ to R⁴, p and q are as defined in general formula (I).

### Scheme 2

A method for preparing the compound represented by general formula (II) or the pharmaceutically acceptable salt thereof of the present disclosure, comprising the following step: 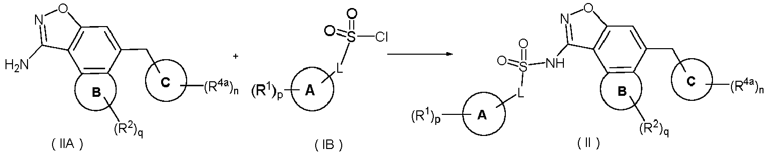 conducting a nucleophilic substitution reaction of a compound represented by general formula (IIA) or a salt thereof with a compound represented by general formula (IB) or a salt thereof in the presence of an alkali, optionally under microwave conditions, to give the compound represented by general formula (II) or the pharmaceutically acceptable salt thereof,
wherein:
ring A, ring B, ring C, L, R¹, R², R^{4a}, p, q and n are as defined in general formula (II).

### Scheme 2-1

A method for preparing the compound represented by general formula (Ii) or the pharmaceutically acceptable salt thereof of the present disclosure, comprising the following step: 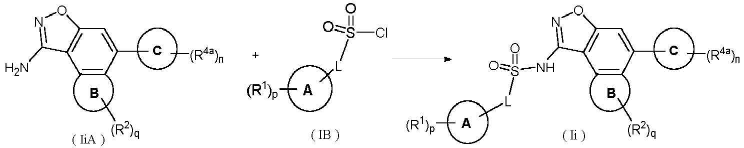 conducting a nucleophilic substitution reaction of a compound represented by general formula (IiA) or a salt thereof with a compound represented by general formula (IB) or a salt thereof in the presence of an alkali, optionally under microwave conditions, to give the compound represented by general formula (Ii) or the pharmaceutically acceptable salt thereof,
wherein:
ring A, ring B, ring C, L, R¹, R², R^{4a}, p, q and n are as defined in general formula (Ii).

### Scheme 3

A method for preparing the compound represented by general formula (III) or the pharmaceutically acceptable salt thereof of the present disclosure, comprising the following step: 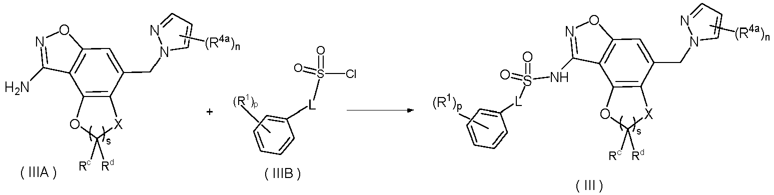 conducting a nucleophilic substitution reaction of a compound represented by general formula (IIIA) or a salt thereof with a compound represented by general formula (IIIB) or a salt thereof in the presence of an alkali, optionally under microwave conditions, to give the compound represented by general formula (III) or the pharmaceutically acceptable salt thereof,
wherein:
L, X, R¹, R^{4a}, R^{c}, R^{d}, p, n and s are as defined in general formula (III).

### Scheme 4

A method for preparing the compound represented by general formula (IV) or the pharmaceutically acceptable salt thereof of the present disclosure, comprising the following step: 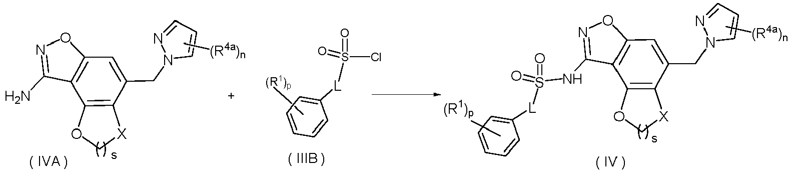 conducting a nucleophilic substitution reaction of a compound represented by general formula (IVA) or a salt thereof with a compound represented by general formula (IIIB) or a salt thereof in the presence of an alkali, optionally under microwave conditions, to give the compound represented by general formula (IV) or the pharmaceutically acceptable salt thereof,
wherein:
X, L, R¹, R^{4a}, p, n and s are as defined in general formula (IV).

### Scheme 5

A method for preparing the compound represented by general formula (II) or the pharmaceutically acceptable salt thereof of the present disclosure, comprising the following steps: 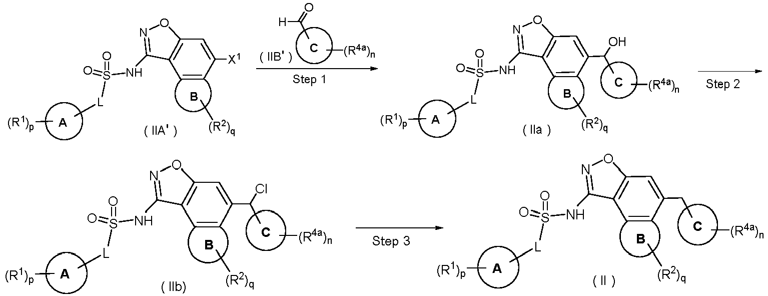
step 1: conducting a nucleophilic addition reaction of a compound represented by general formula (IIA') or a salt thereof with a compound represented by general formula (IIB') or a salt thereof in the presence of an alkali (e.g., n-butyllithium) to give a compound represented by general formula (IIa) or a pharmaceutically acceptable salt thereof;
step 2: conducting a chlorination reaction of the compound represented by general formula (IIa) or the salt thereof (for example, in the presence of PCl₃ or SOCl₂) to give a compound represented by general formula (IIb) or a pharmaceutically acceptable salt thereof; and
step 3: conducting a reduction reaction of the compound represented by general formula (IIb) or the pharmaceutically acceptable salt thereof in the presence of a metal (e.g., zinc powder or iron powder) to give the compound represented by general formula (II) or the pharmaceutically acceptable salt thereof;
wherein:
   X¹ is a halogen, preferably bromine;
   ring A, ring B, ring C, L, R¹, R², R^{4a}, p, q and n are as defined in general formula (II).

### Scheme 6

A method for preparing the compound represented by general formula (Ii) or the pharmaceutically acceptable salt thereof of the present disclosure, comprising the following step: 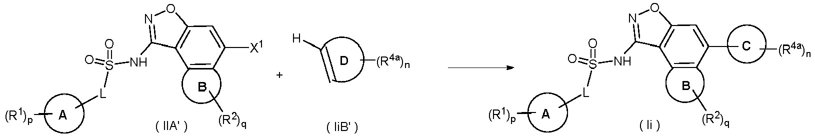 conducting a coupling reaction of a compound represented by general formula (IIA') or a salt thereof with a compound represented by general formula (IiB') or a salt thereof in the presence of an alkali and a metal catalyst and optionally a ligand to give the compound represented by general formula (Ii) or the pharmaceutically acceptable salt thereof,
wherein:
ring D is 3- to 8-membered heterocyclyl containing at least one intra-ring double bond, preferably 5- or 6-membered heterocyclyl containing at least one intra-ring double bond, and more preferably 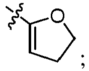
ring C is 3- to 8-membered heterocyclyl, preferably 5- or 6-membered heterocyclyl, and
more preferably 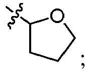
X¹ is a halogen, preferably bromine;
ring A, ring B, L, R¹, R², R^{4a}, p, q and n are as defined in general formula (Ii).

In the reactions of schemes 1 to 6, the alkalis include organic alkalis and inorganic alkalis; the organic alkalis include, but are not limited to, triethylamine, pyridine, 3,5-dimethylpyridine, N,N-diisopropylethylamine, n-butyllithium, lithium diisopropylamide, lithium bis(trimethylsilyl)amide, sodium acetate, potassium acetate, sodium tert-butoxide, potassium tert-butoxide or 1,8-diazabicycloundec-7-ene; the inorganic alkalis include, but are not limited to, sodium hydride, potassium phosphate, sodium carbonate, potassium carbonate, cesium carbonate, sodium hydroxide, lithium hydroxide and potassium hydroxide; the alkalis in schemes 1 to 4 are preferably selected from the group consisting of pyridine, lithium bis(trimethylsilyl)amide and 3,5-dimethylpyridine; the alkali in scheme 5 is preferably n-butyllithium; the alkali in scheme 6 is preferably potassium carbonate.

In scheme 6, the metal catalyst includes, but is not limited to, palladium acetate, tetrakis(triphenylphosphine)palladium, tris(dibenzylideneacetone)dipalladium, [1,1 '-bis(diphenylphosphino)ferrocene]palladium(II) dichloride, bis(acetonitrile)palladium(II) chloride, and palladium/carbon, and is preferably palladium acetate.

In scheme 6, the ligand includes, but is not limited to, triphenylphosphine, tri(o-tolyl)phosphine and 2,2'-bis(diphenylphosphino)-1,1'-binaphthyl (BINAP), and is preferably triphenylphosphine.

The reactions of schemes 1 to 4 are optionally conducted in the presence of catalysts; the catalysts include, but are not limited to, 4-dimethylaminopyridine and dimethyl sulfoxide (DMSO).

When the reactions of schemes 1 to 6 are conducted under microwave conditions, the reaction temperature is 100-150 °C, preferably 120 °C.

When the reactions of schemes 1 to 6 are conducted under microwave conditions, the reaction time is 0.5-6 h, preferably 2-3 h, and more preferably 3 h.

The reactions of schemes 1 to 6 are preferably conducted in solvents; the solvents include, but are not limited to, ethylene glycol dimethyl ether, acetic acid, methanol, ethanol, acetonitrile, n-butanol, toluene, tetrahydrofuran, dichloromethane, petroleum ether, ethyl acetate, n-hexane, dimethyl sulfoxide, 1,4-dioxane, water, N,N-dimethylformamide, N,N-dimethylacetamide, 1,2-dibromoethane, pyridine, and mixtures thereof.

### DETAILED DESCRIPTION

The present disclosure is further described below with reference to examples, but these examples are not intended to limit the scope of the present disclosure.

### Examples

The structures of compounds were determined by nuclear magnetic resonance (NMR) spectroscopy and/or mass spectrometry (MS). The NMR shifts (δ) are given in 10⁻⁶ (ppm). The NMR analyses were performed on a Bruker AVANCE NEO 500M nuclear magnetic resonance instrument, with dimethyl sulfoxide-D6 (DMSO-*d₆*), chloroform-D (CDCl₃) and methanol-D4 (CD₃OD) as solvents and tetramethylsilane (TMS) as an internal standard.

The MS analyses were performed on an Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters; MS model: Waters ACQuity Qda Detector/Waters SQ Detector) and THERMO Ultimate 3000-Q Exactive (manufacturer: THERMO; MS model: THERMO Q Exactive).

The high performance liquid chromatography (HPLC) analyses were performed on Agilent HPLC 1200DAD, Agilent HPLC 1200VWD, and Waters HPLC e2695-2489 high performance liquid chromatographs.

The chiral HPLC analyses were performed on an Agilent 1260 DAD high performance liquid chromatograph.

The preparative high-performance liquid chromatography purification was performed using Waters 2767, Waters 2767-SQ Detecor2, Shimadzu LC-20AP and a Gilson-281 preparative chromatograph.

The preparative chiral chromatography purification was performed on a Shimadzu LC-20AP preparative chromatograph.

The CombiFlash preparative flash chromatograph used was Combiflash Rf200 (TELEDYNE ISCO).

Yantai Huanghai HSGF254 or Qingdao GF254 silica gel plates, 0.15 mm-0.2 mm layer thickness, were used in thin-layer chromatography (TLC) analyses and 0.4 mm-0.5 mm layer thickness in TLC separation and purification.

In the silica gel column chromatography purification, 200- to 300-mesh silica gel (Huanghai, Yantai) was generally used as the carrier.

The kinase mean inhibition rates and the IC₅₀ values were measured on a NovoStar microplate reader (BMG, Germany).

The known starting materials of the present disclosure may be synthesized using or according to methods known in the art, or may be purchased from ABCR GmbH & Co. KG; Acros Organics; Aldrich Chemical Company; Accela ChemBio (Shanghai) Inc.; Darui Chemicals; Shanghai Titan Scientific; Aladdin; Energy Chemical, China National Pharmaceutical Group Corporation; Adamas Reagent Co., Ltd.; Sigma-Aldrich (Shanghai) Trading Co., Ltd.; Bide Pharmatech Ltd.; Shanghai Haohong Biomedical Technology Co., Ltd.; Thermo Fisher Scientific (China) Co., Ltd.; and the like.

In the examples, the reactions can all be performed in an argon atmosphere or a nitrogen atmosphere unless otherwise specified.

The argon atmosphere or nitrogen atmosphere means that the reaction flask is connected to a balloon containing about 1 L of argon or nitrogen gas.

The hydrogen atmosphere means that the reaction flask is connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 cycles of vacuumization/hydrogen filling.

The microwave reactions were performed using a CEM Discover-S 908860 microwave reactor.

In the examples, the solutions were aqueous solutions unless otherwise specified.

In the examples, the reaction temperature was room temperature, i.e., 20 °C-30 °C, unless otherwise specified.

The reaction process monitoring in the examples was performed using thin-layer chromatography (TLC). The developing solvents used in the reactions, the eluent systems used in the column chromatography purification and the developing solvent systems used in the thin-layer chromatography analyses include: A: n-hexane/ethyl acetate system, and B: dichloromethane/methanol system. The volume ratios of the solvents were adjusted depending on the polarity of the compounds, or by adding a small amount of basic or acidic reagents such as triethylamine and acetic acid.

### Example 1

### N-(4-((1H-pyrazol-1-yl)methyl)-2,3-dihydrobenzofuro[7,6-d]isoxazol-8-yl)-2,6-dimetho xybenzenesulfonamide 1

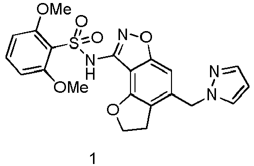

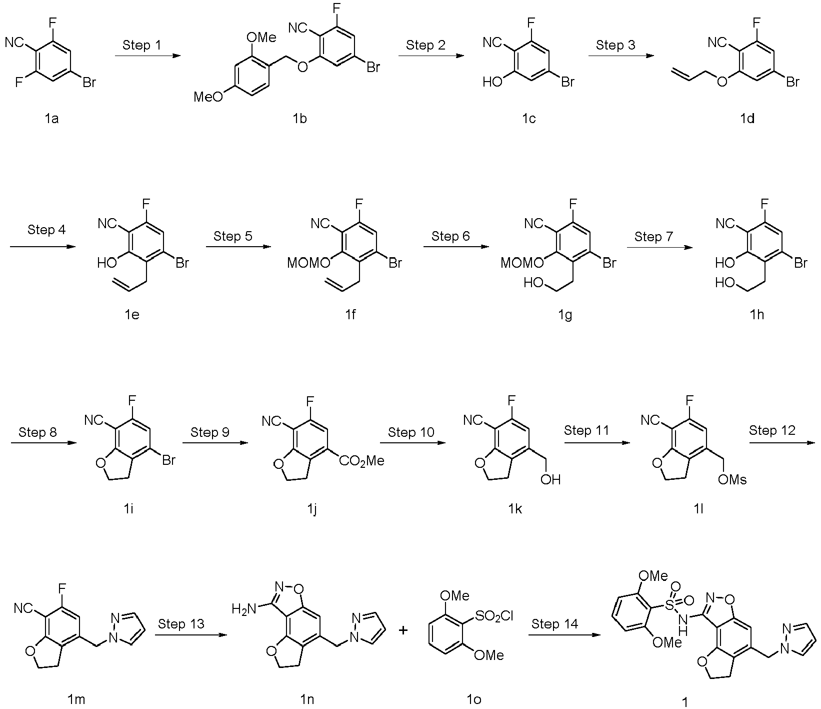

### Step 1

### 4-bromo-2-((2,4-dimethoxybenzyl)oxy)-6-fluorobenzonitrile 1b

4-Bromo-2,6-difluorobenzonitrile **1a** (22 g, 101 mmol) and 2,4-dimethoxybenzyl alcohol (18.5 g, 110 mmol) were dissolved in *N*,*N*-dimethylformamide (200 mL), and cesium carbonate (49 g, 150 mmol) was added. The reaction mixture was stirred at 60 °C for 16 h. The reaction mixture was cooled to room temperature and filtered under reduced pressure. The filtrate was diluted with ethyl acetate (500 mL) and washed with a saturated sodium chloride solution (30 mL × 5). The resulting organic phase was dried over anhydrous sodium sulfate and filtered, and the filtrate was concentrated under reduced pressure to give the title product **1b** (36.9 g, yield: 100%). The product was directly used in the next step without purification.

### Step 2

### 4-bromo-2-fluoro-6-hydroxybenzonitrile 1c

Compound **1b** (36.9 g, 100.7 mmol) was dissolved in dichloromethane (250 mL), and the solution was cooled to 0 °C. Trifluoroacetic acid (39 g, 342 mmol) was added dropwise, and the reaction mixture was warmed to room temperature and stirred for 1 h. The reaction mixture was concentrated under reduced pressure, and the resulting residue was purified by silica gel column chromatography with eluent system A to give the title product **1c** (9.7 g, yield: 44.5%).

### Step 3

### 2-(allyloxy)-4-bromo-6-fluorobenzonitrile 1d

Compound **1c** (10.7 g, 49.5 mmol) was dissolved in *N,N*-dimethylformamide (120 mL), and the reaction mixture was cooled to 0 °C. Cesium carbonate (24 g, 73.7 mmol) and allyl bromide (11.2 g, 92.6 mmol) were added, and the reaction mixture was warmed to room temperature and stirred for 4 h. The reaction mixture was filtered under reduced pressure. The filtrate was diluted with ethyl acetate (400 mL) and washed with a saturated sodium chloride solution (30 mL × 3). The resulting organic phase was dried over anhydrous sodium sulfate and filtered, and the filtrate was concentrated under reduced pressure. The resulting residue was purified by silica gel column chromatography with eluent system A to give the title product **1d** (11.7 g, yield: 92%). ¹H NMR (500 MHz, CDCl₃) δ 7.02 (dt, 1H), 6.95 (t, 1H), 6.04 (m, 1H), 5.57-5.47 (m, 1H), 5.41 (dt, 1H), 4.75-4.64 (m, 2H).

### Step 4

### 3-allyl-4-bromo-6-fluoro-2-hydroxybenzonitrile 1e

Compound **1d** (3.35 g, 13.1 mmol) was dissolved in 1,2-dichlorobenzene (80 mL), and the reaction mixture was purged with nitrogen three times and stirred at 180 °C for 13 h. The reaction mixture was cooled to room temperature, and the resulting residue was purified by silica gel column chromatography (wet loading) with eluent system A to give the title product **1e** (2.77 g, yield: 82.7%).

¹H NMR (500 MHz, CDCl₃) δ 7.07 (dd, 1H), 6.45 (s, 1H), 5.90 (dddd, 1H), 5.24-5.10 (m, 2H), 3.68-3.55 (m, 2H).

### Step 5

### 3-allyl-4-bromo-6-fluoro-2-(methoxymethoxy)benzonitrile 1f

Compound **1e** (1 g, 3.91 mmol) was dissolved in acetonitrile (15 mL), and potassium carbonate (1.07 g, 7.74 mmol) and bromo(bromomethoxy)methane (MOMBr, 634 mg, 5.08 mmol) were added. The reaction was stirred for 2 h. The reaction mixture was quenched with water (10 mL) and extracted with ethyl acetate (50 mL × 3). The organic phases were combined, dried over anhydrous sodium sulfate, and filtered. The filtrate was concentrated under reduced pressure, and the resulting residue was purified by silica gel column chromatography with eluent system A to give the title product **1f** (1.11 g, yield: 94.7%).

¹H NMR (500 MHz, CDCl₃) δ 7.28 (d, 1H), 5.89 (ddt, 1H), 5.26 (d, 2H), 5.08-4.98 (m, 2H), 3.66 (s, 3H), 3.58 (s, 2H).

### Step 6

### 4-bromo-6-fluoro-3-(2-hydroxyethyl)-2-(methoxymethoxy)benzonitrile 1g

Compound **1f** (1.1 g, 3.66 mmol) was dissolved in 50 mL of a mixed solvent of methanol and tetrahydrofuran (V:V = 1:1), and the reaction mixture was cooled to -78 °C. Dry ozone was bubbled into the mixture for 1 h. The reaction mixture was quenched with triphenylphosphine (1.05 g, 4.0 mmol), slowly warmed to room temperature, and stirred for 0.5 h. Then the reaction mixture was cooled to 0 °C, and sodium borohydride (560 mg, 14.8 mmol) was added portionwise. The reaction was stirred for 1 h. The reaction mixture was quenched with water (5 mL), concentrated under reduced pressure, diluted with ethyl acetate (150 mL), and washed with a saturated sodium chloride solution (10 mL × 2). The resulting organic phase was dried over anhydrous sodium sulfate and filtered. The filtrate was concentrated under reduced pressure, and the resulting residue was purified by silica gel column chromatography with eluent system A to give the title product **1g** (800 mg, yield: 71.8%).

¹H NMR (500 MHz, CDCl₃) δ 7.28 (d, 1H), 5.32 (s, 2H), 3.82 (t, 2H), 3.67 (s, 3H), 3.10 (t, 2H), 2.39 (s, 1H).

### Step 7

### 4-bromo-6-fluoro-2-hydroxy-3-(2-hydroxyethyl)benzonitrile 1h

Compound **1g** (800 mg, 2.63 mmol) was dissolved in methanol (25 mL), and the reaction mixture was cooled to 0 °C. A solution of hydrochloride solution in dioxane (4 M, 24 mmol, 6 mL) was added, and the reaction mixture was warmed to room temperature and stirred for 2 h. The reaction mixture was concentrated under reduced pressure, and the resulting residue was purified by silica gel column chromatography with eluent system A to give the title product **1h** (680 mg, yield: 99%).

¹H NMR (500 MHz, CDCl₃) δ 7.07-6.95 (m, 1H), 4.01 (t, 2H), 3.14 (t, 2H).

### Step 8

### 4-bromo-6-fluoro-2,3-dihydrobenzofuran-7-carbonitrile 1i

Compound **1h** (680 mg, 2.61 mmol) was dissolved in tetrahydrofuran (100 mL), and the reaction mixture was cooled to 0 °C. Triphenylphosphine (2.05 g, 7.81 mmol) and diisopropyl azodicarboxylate (1.58 g, 7.81 mmol) were added, and the reaction mixture was warmed to room temperature and stirred for 2 h. The reaction mixture was concentrated under reduced pressure, and the resulting residue was purified by silica gel column chromatography with eluent system A to give the title product **1i** (570 mg, yield: 90%).

¹H NMR (500 MHz, CDCl₃) δ 6.84 (dd, 1H), 4.84 (td, 2H), 3.24 (tt, 2H).

### Step 9

### methyl 7-cyano-6-fluoro-2,3-dihydrobenzofuran-4-carboxylate 1j

Compound **1i** (615 mg, 2.54 mmol) was dissolved in 20 mL of a mixed solvent of methanol and *N*,*N*-dimethylformamide (V:V = 1:3), and [1,1'-bis(diphenylphosphino)ferrocene]palladium dichloride (185 mg, 252 mmmol) and triethylamine (771 mg, 7.62 mmol) were sequentially added. The reaction mixture was purged with carbon monoxide 3 times and stirred at 80 °C for 12 h in a carbon monoxide atmosphere. The reaction mixture was cooled to room temperature, concentrated under reduced pressure, diluted with ethyl acetate (150 mL), and washed with a saturated sodium chloride solution (20 mL × 3). The resulting organic phase was dried over anhydrous sodium sulfate and filtered, and the filtrate was concentrated under reduced pressure. The resulting residue was purified by silica gel column chromatography with eluent system A to give the title product **1j** (289 mg, yield: 51.4%).

¹H NMR (500 MHz, CDCl₃) δ 7.24 (d, 1H), 4.84 (td, 2H), 3.94 (s, 3H), 3.57 (td, 2H).

### Step 10

### 6-fluoro-4-(hydroxymethyl)-2,3-dihydrobenzofuran-7-carbonitrile 1k

Compound **1j** (436 mg, 1.97 mmol) was dissolved in dry tetrahydrofuran (10 mL), and the reaction mixture was purged with nitrogen 3 times and cooled to 0 °C. Lithium borohydride (2 M, 5 mmol, 2.5 mL) was added. The reaction mixture was heated to 70 °C and stirred for 2 h. The reaction mixture was cooled to room temperature, quenched with water (1 mL), diluted with ethyl acetate (100 mL), and washed with a saturated sodium chloride solution (20 mL × 2). The resulting organic phase was dried over anhydrous sodium sulfate and filtered, and the filtrate was concentrated under reduced pressure. The resulting residue was purified by silica gel column chromatography with eluent system A to give the title product **1k** (350 mg, yield: 91.9%).

¹H NMR (500 MHz, CDCl₃) δ 6.76 (dd, 1H), 4.81 (td, 2H), 4.66 (s, 2H), 3.20 (t, 2H).

### Step 11

### (7-cyano-6-fluoro-2,3-dihydrobenzofuran-4-yl)methyl methanesulfonate 1l

Compound **1k** (350 mg, 1.81 mmol) was dissolved in dichloromethane (20 mL), and the solution was cooled to 0 °C. Triethylamine (2.2 g, 21.74 mmol) and methanesulfonyl chloride (1.24 g, 10.82 mmol) were added. The reaction mixture was warmed to room temperature and stirred for 2 h. The reaction mixture was quenched with a saturated sodium bicarbonate solution (10 mL), diluted with ethyl acetate (100 mL), and washed with a saturated sodium chloride solution (20 mL × 2). The resulting organic phase was dried over anhydrous sodium sulfate and filtered, and the filtrate was concentrated under reduced pressure to give the title product **1l** (491 mg, yield: 99%). The product was directly used in the next step without purification.

### Step 12

### 4-((1H-pyrazol-1-yl)methyl)-6-fluoro-2,3-dihydrobenzofuran-7-carbonitrile 1m

Compound **1l** (491 mg, 1.81 mmol) was dissolved in *N,N*-dimethylformamide (15 mL), and potassium carbonate (1.25 g, 9.04 mmol) and pyrazole (369 mg, 5.42 mmol) were added. The reaction mixture was stirred at 60 °C for 12 h. The reaction mixture was filtered, and the resulting filtrate was diluted with ethyl acetate (100 mL) and washed with a saturated sodium chloride solution (20 mL × 3). The resulting organic phase was dried over anhydrous sodium sulfate and filtered, and the filtrate was concentrated under reduced pressure. The resulting residue was purified by silica gel column chromatography with eluent system A to give the title product **1m** (361 mg, yield: 82%).

MS m/z (ESI): 244.0 [M+1].

### Step 13

### 4-((1H-pyrazol-1-yl)methyl)-2,3-dihydrobenzofuro[7,6-d]isoxazol-8-amine 1n

Compound **1m** (361 mg, 1.48 mmol) and acetohydroxamic acid (334 mg, 4.45 mmol) were dissolved in *N*,*N*-dimethylformamide (15 mL), and potassium carbonate (1.0 g, 7.23 mmol) was added. The reaction mixture was stirred at 60 °C for 12 h. The reaction mixture was filtered, diluted with ethyl acetate (100 mL), and washed with a saturated sodium chloride solution (20 mL × 3). The resulting organic phase was dried over anhydrous sodium sulfate and filtered, and the filtrate was concentrated under reduced pressure. The resulting residue was purified by silica gel column chromatography with eluent system A to give the title product **1n** (189 mg, yield: 49.7%).

MS m/z (ESI): 257.0 [M+1].

### Step 14

### N-(4-((1H-pyrazol-1-yl)methyl)-2,3-dihydrobenzofuro[7,6-d]isoxazol-8-yl)-2,6-dimetho xybenzenesulfonamide 1

Compound **1n** (189 mg, 737 µmol) and 2,6-dimethoxybenzenesulfonyl chloride **1o** (265 mg, 1.12 mmol, prepared by the method disclosed in "scheme 15 on page 70 of the specification of the patent application WO2020254946A1") were dissolved in pyridine (8 mL), and the solution was purged with nitrogen 3 times. The reaction mixture was microwaved at 120 °C for 2 h. The reaction mixture was cooled to room temperature and concentrated under reduced pressure. The resulting residue was purified by high performance liquid chromatography (Welch Xtimate C18 5 µm 30 × 150 mm; mobile phases: A-aqueous phase (0.1% formic acid): B-acetonitrile = 30%-45% (15 min), flow rate: 30 mL/min) to give the title product **1** (44 mg, yield: 13%).

MS m/z (ESI): 457.0 [M+1].

¹H NMR (500 MHz, CD₃OD) δ 7.71 (s, 1H), 7.55 (s, 1H), 7.47 (td, 1H), 6.74 (dd, 2H), 6.58 (s, 1H), 6.36 (q, 1H), 5.42 (s, 2H), 4.80 (td, 2H), 3.83 (s, 6H), 3.10 (t, 2H).

### Example 2

### N-(5-((1H-pyrazol-1-yl)methyl)-3,4-dihydro-2H-chromeno[8,7-d]isoxazol-9-yl)-2,6-di methoxybenzenesulfonamide 2

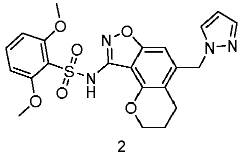

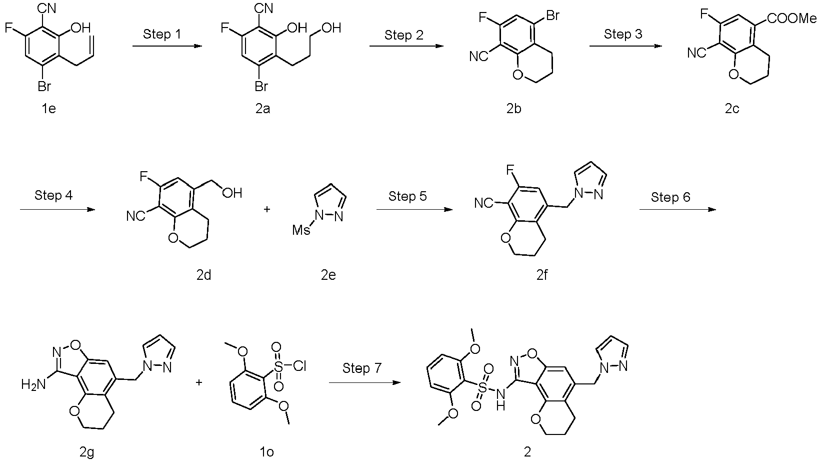

### Step 1

### 4-bromo-6-fluoro-2-hydroxy-3-(3-hydroxypropyl)benzonitrile 2a

Compound **1e** (4.8 g, 18.7 mmol) was dissolved in anhydrous tetrahydrofuran (100 mL), and a solution of borane in tetrahydrofuran (1.0 M, 22 mL, 22 mmol) was added dropwise at 0 °C. The reaction mixture was stirred in an ice bath for 2 h. A 3 M aqueous solution of sodium hydroxide (13 mL, 39 mmol) and 30% hydrogen peroxide (3.0 mL) were sequentially added under ice-bath conditions. After the addition, the mixture was stirred for 10 min. The pH of the reaction mixture was adjusted to 2 with 2 M hydrochloric acid, and the reaction mixture was extracted with ethyl acetate (100 mL × 2). The organic phase was collected, dried over anhydrous sodium sulfate, and filtered. The filtrate was concentrated under reduced pressure, and the resulting residue was purified by silica gel column chromatography with eluent system A to give the title product **2a** (3.5 g, yield: 68.1%).

MS m/z (ESI): 275.8 [M+1].

¹H NMR (500 MHz, CDCl₃) δ 7.04 (d, 1H), 3.71 (t, 2H), 3.00-2.98 (m, 2H), 2.01-1.96 (m, 2H).

### Step 2

### 5-bromo-7-fluorochromane-8-carbonitrile 2b

Compound **2a** (3.8 g, 13.9 mmol) was dissolved in anhydrous tetrahydrofuran (80 mL), and the reaction was cooled to 0 °C. Triphenylphosphine (4.4 g, 16.8 mmol) and diisopropyl azodicarboxylate (3.4 g, 16.8 mmol) were added, and the reaction mixture was warmed to room temperature and stirred for 2 h. The reaction mixture was concentrated under reduced pressure, and the resulting residue was purified by silica gel column chromatography with eluent system A to give the title product **2b** (3.0 g, yield: 84.5%).

MS m/z (ESI): 257.8 [M+1].

¹H NMR (500 MHz, CDCl₃) δ 7.03 (d, 1H), 4.33 (t, 2H), 2.77-2.74 (m, 2H), 2.12-2.08 (m, 2H).

### Step 3

### methyl 8-cyano-7-fluorochromane-5-carboxylate 2e

Compound **2b** (2.6 g, 10.2 mmol) was dissolved in 40 mL of a mixed solvent of methanol and *N,N*-dimethylformamide (V:V = 1:3), and [1,1'-bis(diphenylphosphino)ferrocene]palladium dichloride (800 mg, 1.09 mmol) and triethylamine (3.0 g, 2.93 mmol) were sequentially added. The mixture was purged with carbon monoxide 3 times and stirred at 90 °C under 10 bar pressure for 16 h. The reaction mixture was cooled to room temperature, concentrated under reduced pressure, diluted with ethyl acetate (150 mL), and washed with a saturated sodium chloride solution (50 mL × 3). The resulting organic phase was dried over anhydrous sodium sulfate and filtered, and the filtrate was concentrated under reduced pressure. The resulting residue was purified by silica gel column chromatography with eluent system A to give the title product **2c** (2.1 g, yield: 87.9%).

MS m/z (ESI): 235.9 [M+1].

¹H NMR (500 MHz, CDCl₃) δ 7.26 (d, 1H), 4.38-4.36 (m, 2H), 3.93 (s, 3H), 3.10-3.07 (m, 2H), 2.08-2.03 (m, 2H).

### Step 4

### 7-fluoro-5-(hydroxymethyl)chromane-8-carbonitrile 2d

Compound **2c** (2.1 g, 8.93 mmol) was dissolved in dry tetrahydrofuran (40 mL), and the reaction mixture was purged with nitrogen 3 times and cooled to 0 °C. Lithium borohydride (2 M, 18 mmol, 9.0 mL) was added. The reaction mixture was heated to 70 °C and stirred for 2 h. The reaction mixture was cooled to room temperature, quenched with water (1 mL), diluted with ethyl acetate (100 mL), and washed with a saturated sodium chloride solution (50 mL × 2). The resulting organic phase was dried over anhydrous sodium sulfate and filtered, and the filtrate was concentrated under reduced pressure. The resulting residue was purified by silica gel column chromatography with eluent system A to give the title product **2d** (1.84 g, yield: 99.5%). MS m/z (ESI): 207.9 [M+1].

### Step 5

### 5-((1H-pyrazol-1-yl)methyl)-7-fluorochromane-8-carbonitrile 2f

Compound **2d** (1.8 g, 8.69 mmol) and 1-(methylsulfonyl)-1*H*-pyrazole **2e** (1.5 g, 10.3 mmol, prepared by the method for "intermediate 13 disclosed in scheme 8 on page 63 of the specification of the patent application WO2020254946A1") were dissolved in acetonitrile (30 mL), and cesium carbonate (4.2 g, 12.9 mmol) was added. The mixture was left to react at 70 °C for 1 h. The reaction mixture was filtered, and the filtrate was concentrated under reduced pressure. The resulting residue was purified by silica gel column chromatography with eluent system A to give the title product **2f** (1.9 g, yield: 85.0%).

MS m/z (ESI): 258.0 [M+1].

### Step 6

### 5-((1H-pyrazol-1-yl)methyl)-3,4-dihydro-2H-chromeno[8,7-d]isoxazol-9-amine 2g

Compound **2f** (1.9 g, 7.39 mmol) and acetohydroxamic acid (1.7 g, 22.2 mmol, Adamas) were dissolved in *N,N*-dimethylformamide (30 mL) and water (4.0 mL), and potassium carbonate (6.2 g, 44.9 mmol) was added. The reaction mixture was stirred at 70 °C for 24 h. The reaction mixture was cooled to room temperature, and water (100 mL) was added. The mixture was filtered, and the filter cake was collected and dried to give the title product **2g** (1.65 g, yield: 82.7%).

MS m/z (ESI): 271.0 [M+1].

¹H NMR (500 MHz, DMSO-*d*₆) δ 7.78 (d, 1H), 7.52 (d, 1H), 6.34 (s, 1H), 6.32 (t, 1H), 5.85 (s, 2H), 5.39 (s, 2H), 4.25-4.23 (m, 2H), 2.68 (t, 2H), 2.03-1.98 (m, 2H).

### Step 7

### N-(5-((1H-pyrazol-1-yl)methyl)-3,4-dihydro-2H-chromeno[8,7-d]isoxazol-9-yl)-2,6-di methoxybenzenesulfonamide 2

Compound **2g** (200 mg, 0.740 mmol) and compound **1o** (300 mg, 1.27 mmol) were dissolved in pyridine (5.0 mL), and the solution was purged with nitrogen 3 times. The reaction mixture was microwaved at 120 °C for 3 h. The reaction mixture was cooled to room temperature and concentrated under reduced pressure. The resulting residue was purified by high performance liquid chromatography (Xtimate phenyl-hexyl Prep C18 5 µm, 30 × 150 mm; mobile phases: A-aqueous phase (0.1% ammonia water): B-acetonitrile = 5%-45% (20 min), flow rate: 30 mL/min) to give the title product **2** (40 mg, yield: 11.5%).

MS m/z (ESI): 470.8 [M+1].

¹H NMR (500 MHz, DMSO-*d*₆) δ 9.40 (s, 1H), 7.79 (d, 1H), 7.52 (d, 1H), 7.48 (t, 1H), 6.77 (d, 2H), 6.43 (s, 1H), 6.32 (t, 1H), 5.42 (s, 2H), 4.25 (t, 2H), 3.78 (s, 6H), 2.70 (t, 2H), 2.04-1.99 (m, 2H).

### Example 3

### N-(5-((1H-pyrazol-1-yl)methyl)-3,4-dihydro-2H-chromeno[8,7-d]isoxazol-9-yl)-2-meth oxybenzenesulfonamide 3

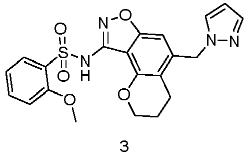

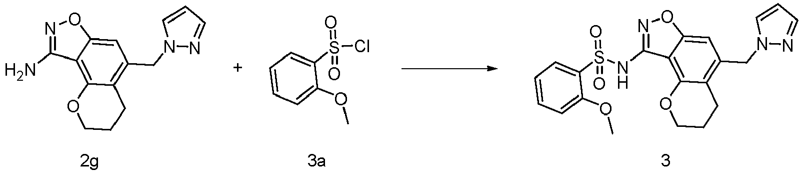

Compound **2g** (300 mg, 1.11 mmol) and 2-methoxybenzenesulfonyl chloride **3a** (460 mg, 2.22 mmol) were dissolved in pyridine (6.0 mL), and the solution was purged with nitrogen 3 times. The reaction mixture was microwaved at 120 °C for 3 h. The reaction mixture was cooled to room temperature and concentrated under reduced pressure. The resulting residue was purified by high performance liquid chromatography (Sharpsil-T Prep C18 5 µm 30 × 150 mm; mobile phases: A-aqueous phase (10 mM ammonium bicarbonate): B-acetonitrile = 20%-45% (15 min), flow rate: 30 mL/min) to give the title product **3** (100 mg, yield: 20.5%).

MS m/z (ESI): 440.8 [M+1].

¹H NMR (500 MHz, DMSO-*d*₆) δ 9.96 (s, 1H), 7.81-7.78 (m, 2H), 7.62 (d, 1H), 7.51 (d, 1H), 7.20 (d, 1H), 7.09 (d, 1H), 6.45 (s, 1H), 6.32 (t, 1H), 5.41 (s, 2H), 4.19 (t, 2H), 3.81 (s, 3H), 2.69 (t, 2H), 2.01-1.96 (m, 2H).

### Example 4

### N-(5-((1H-pyrazol-1-yl)methyl)-2,3-dihydro-[1,4]dioxino[2',3':5,6]benzo[1,2-d]isoxazol -9-yl)-2-methoxybenzenesulfonamide 4

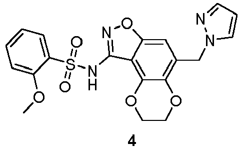

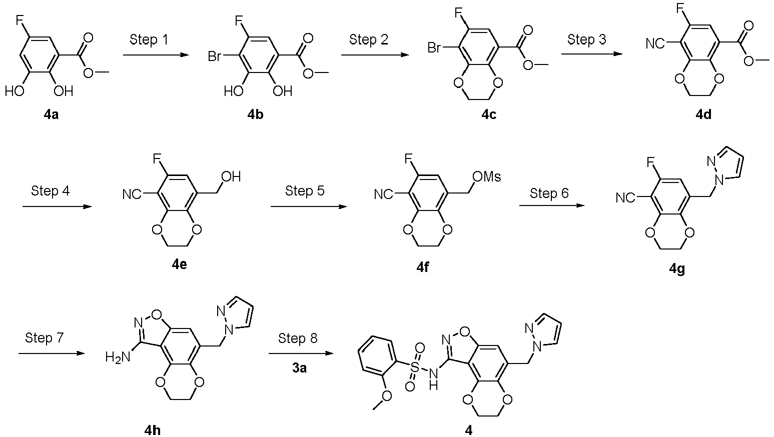

### Step 1

### methyl 4-bromo-5-fluoro-2,3-dihydroxybenzoate 4b

The compound methyl 5-fluoro-2,3-dihydroxybenzoate **4a** (2.26 g, 12.1 mmol, prepared by the method disclosed in "J. Med. Chem. 2010, 53, 7035-7047") was dissolved in dichloromethane (60 mL), and the reaction mixture was cooled to 0 °C. A-Bromosuccinimide (2.6 g, 14.6 mmol) was added portionwise, and the reaction mixture was stirred at room temperature for 3 d. The reaction mixture was washed with a saturated sodium bisulfite solution (30 mL × 2). The organic phases were combined, dried over anhydrous sodium sulfate, and filtered. The filtrate was concentrated under reduced pressure, and the resulting residue was purified by silica gel column chromatography with eluent system A to give the title product **4b** (1.8 g, yield: 55.9%). ¹H NMR (500 MHz, CDCl₃) δ 10.84 (s, 1H), 7.14 (d, 1H), 3.97 (s, 3H).

### Step 2

### methyl 8-bromo-7-fluoro-2,3-dihydrobenzo[b][1,4]dioxine-5-carboxylate 4e

Compound **4b** (1.0 g, 3.77 mmol) and 1,2-dibromoethane (1.1 g, 5.85 mmol) were dissolved in *N,N*-dimethylformamide (10 mL), and cesium carbonate (2.46 g, 7.55 mmol) was added. The reaction mixture was stirred at 80 °C for 2 h. The reaction mixture was filtered, and the filtrate was concentrated under reduced pressure. The resulting residue was purified by silica gel column chromatography with eluent system A to give the title product **4e** (873 mg, yield: 79.5%).

MS m/z (ESI): 292.9 [M+1].

¹H NMR (500 MHz, CDCl₃) δ 7.26 (d, 1H), 4.45-4.43 (m, 2H), 4.39-4.36 (m, 2H), 3.91 (s, 3H).

### Step 3

### methyl 8-cyano-7-fluoro-2,3-dihydrobenzo[b][1,4]dioxine-5-carboxylate 4d

Compound **4e** (708 mg, 2.43 mmol), zinc cyanide (714 mg, 6.08 mmol) and methanesulfonato(2-dicyclohexylphosphino-2',6'-diisopropoxy-1,1'-biphenyl)(2-amino-1,1'-biphenyl-2-yl)palladium (306 mg, 0.365 mmol, Bide) were dissolved in *N*,*N*-dimethylformamide (15 mL), and the solution was purged with nitrogen 3 times. The reaction mixture was left to react at 110 °C for 16 h. The reaction mixture was cooled to room temperature and filtered, and the filtrate was concentrated under reduced pressure. The resulting residue was purified by silica gel column chromatography with eluent system A to give the title product **4d** (503 mg, yield: 87.2%).

MS m/z (ESI): 238.0 [M+1].

¹H NMR (500 MHz, CDCl₃) δ 7.20 (d, 1H), 4.51-4.48 (m, 2H), 4.42-4.40 (m, 2H), 3.94 (s, 3H).

### Step 4

### 6-fluoro-8-(hydroxymethyl)-2,3-dihydrobenzo[b][1,4]dioxine-5-carbonitrile 4e

Compound **4d** (500 mg, 2.1 mmol) was dissolved in dry tetrahydrofuran (15 mL), and the reaction mixture was purged with nitrogen 3 times and cooled to 0 °C. Lithium borohydride (2 M, 4 mmol, 2.0 mL) was added. The reaction mixture was heated to 70 °C and stirred for 2 h. The reaction mixture was cooled to room temperature, quenched with water (1 mL), diluted with ethyl acetate (30 mL), and washed with a saturated sodium chloride solution (30 mL × 2). The organic phases were combined, dried over anhydrous sodium sulfate, and filtered. The filtrate was concentrated under reduced pressure, and the resulting residue was purified by silica gel column chromatography with eluent system A to give the title product **4e** (323 mg, yield: 73.2%).

¹H NMR (500 MHz, CDCl₃) δ 6.87 (dd, 1H), 4.72 (s, 2H), 4.46-4.42 (m, 2H), 4.36-4.32 (m, 2H).

### Step 5

### (8-cyano-7-fluoro-2,3-dihydrobenzo[b][1,4]dioxin-5-yl)methyl methanesulfonate 4f

Compound **4e** (323 mg, 1.54 mmol) was dissolved in dichloromethane (10 mL), and the solution was cooled to 0 °C. Triethylamine (780 mg, 7.71 mmol) and methanesulfonyl chloride (355 mg, 3.1 mmol) were added. The reaction mixture was warmed to room temperature and stirred for 2 h. The reaction mixture was quenched with a saturated sodium bicarbonate solution (5 mL), diluted with ethyl acetate (30 mL), and washed with a saturated sodium chloride solution (20 mL × 2). The organic phases were combined, dried over anhydrous sodium sulfate, and filtered. The filtrate was concentrated under reduced pressure to give the title product **4f** (200 mg, yield: 45.1%). The product was directly used in the next step without purification.

### Step 6

### 8-((1H-pyrazol-1-yl)methyl)-6-fluoro-2,3-dihydrobenzo[b][1,4]dioxine-5-carbonitrile 4g

Compound **4f** (200 mg, 0.69 mmol) was dissolved in *N*,*N*-dimethylformamide (7 mL), and potassium carbonate (240 mg, 1.73 mmol) and pyrazole (120 mg, 1.76 mmol) were added. The reaction mixture was stirred at 60 °C for 12 h. The reaction mixture was filtered, and the resulting filtrate was diluted with ethyl acetate (30 mL) and washed with a saturated sodium chloride solution (20 mL × 3). The organic phases were combined, dried over anhydrous sodium sulfate, and filtered. The filtrate was concentrated under reduced pressure. The resulting residue was purified by silica gel column chromatography with eluent system A to give the title product **4g** (155 mg, yield: 85.8%).

MS m/z (ESI): 260.0 [M+1].

¹H NMR (500 MHz, CDCl₃) δ 7.63 (d, 1H), 7.48 (dd, 1H), 6.34 (t, 1H), 6.22 (d, 1H), 5.34 (s, 2H), 4.47-4.42 (m, 2H), 4.38-4.35 (m, 2H).

### Step 7

### 5-((1H-pyrazol-1-yl)methyl)-2,3-dihydro-[1,4]dioxino[2',3':5,6]benzo[1,2-d]isoxazol-9-amine 4h

Compound **4g** (155 mg, 0.6 mmol) and acetohydroxamic acid (135 mg, 1.8 mmol) were dissolved in 4 mL of a mixed solvent of *N,N*-dimethylformamide and water (V:V = 7:1), and potassium carbonate (495 mg, 3.58 mmol) was added. The reaction mixture was stirred at 70 °C for 24 h. The reaction mixture was cooled to room temperature and filtered, and the filtrate was concentrated under reduced pressure. The resulting residue was purified by silica gel column chromatography with eluent system A to give the title product **4h** (110 mg, yield: 67.5%).

MS m/z (ESI): 273.0 [M+1].

¹H NMR (500 MHz, CDCl₃) δ 7.61-7.55 (m, 1H), 7.49 (d, 1H), 6.51 (s, 1H), 6.32 (t, 1H), 5.39 (s, 2H), 4.60 (s, 2H), 4.42 (dd, 2H), 4.37 (dd, 2H).

### Step 8

### N-(5-((1H-pyrazol-1-yl)methyl)-2,3-dihydro-[1,4]dioxino[2',3':5,6]benzo[1,2-d]isoxazol -9-yl)-2-methoxybenzenesulfonamide 4

Compound **4h** (40 mg, 0.147 mmol) and compound **3a** (152 mg, 0.735 mmol) were dissolved in pyridine (3.0 mL), and the solution was purged with nitrogen 3 times. The reaction mixture was microwaved at 120 °C for 3 h. The reaction mixture was cooled to room temperature and concentrated under reduced pressure. The resulting residue was purified by high performance liquid chromatography (Sharpsil-T Prep C18 5 µm 30 × 150 mm; mobile phases: A-aqueous phase (10 mM ammonium bicarbonate): B-acetonitrile = 15%-35% (20 min), flow rate: 30 mL/min) to give the title product **4** (25 mg, yield: 38.4%).

MS m/z (ESI): 443.0 [M+1].

¹H NMR (500 MHz, DMSO-*d*₆) δ 10.33 (s, 1H), 7.80 (d, 1H), 7.77 (dd, 1H), 7.58 (s, 1H), 7.50 (d, 1H), 7.18 (s, 1H), 7.05 (s, 1H), 6.41 (s, 1H), 6.30 (t, 1H), 5.37 (s, 2H), 4.33 (q, 4H), 3.80 (s, 3H).

### Example 5

### N-(4-((1H-pyrazol-1-yl)methyl)-2,2-difluoro-[1,3]dioxolo[4',5':5,6]benzo[1,2-d]isoxazo l-8-yl)-2,6-dimethoxybenzenesulfonamide 5

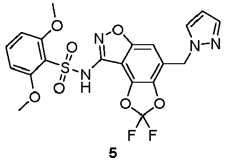

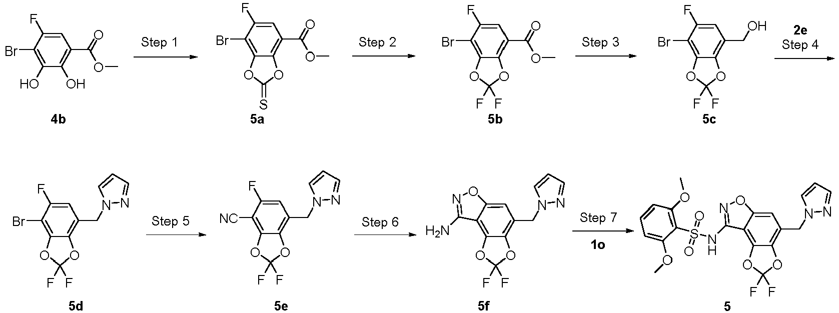

### Step 1

### methyl 7-bromo-6-fluoro-2-thioxobenzo[d][1,3]dioxole-4-carboxylate 5a

Compound **4b** (16.7 g, 63 mmol) was dissolved in tetrahydrofuran (200 mL), and the reaction mixture was cooled to 0 °C. *N*,*N*'-Thiocarbonyldiimidazole (18 g, 101 mmol) was added portionwise, and the reaction mixture was stirred at room temperature for 3 h. The reaction mixture was concentrated under reduced pressure, and the resulting residue was purified by silica gel column chromatography with eluent system A to give the title product **5a** (9.1 g, yield: 47.0%).

¹H NMR (500 MHz, CDCl₃) δ 7.71 (d, 1H), 4.05 (s, 3H).

### Step 2

### methyl 7-bromo-2,2,6-trifluorobenzo[d][1,3]dioxole-4-carboxylate 5b

Compound **5a** (9.1 g, 29.6 mmol) was dissolved in dichloromethane (160 mL) at -40 °C, and a pyridine hydrogen fluoride solution (42.3 g, 427 mmol) was added in a nitrogen atmosphere. The reaction mixture was left to react at -40 °C for 5 min. Then *N*-iodosuccinimide (20 g, 88.9 mmol) was added portionwise, and the reaction mixture was left to react at -40 °C for another 30 min. The reaction mixture was quenched with a saturated sodium bisulfite solution (20 mL) and washed with a saturated sodium chloride solution (60 mL × 2). The organic phases were combined, dried over anhydrous sodium sulfate, and filtered. The filtrate was concentrated under reduced pressure, and the resulting residue was purified by silica gel column chromatography with eluent system A to give the title product **5b** (4.8 g, yield: 51.7%).

¹H NMR (500 MHz, CDCl₃) δ 7.49 (d, 1H), 4.00 (s, 3H).

### Step 3

### (7-bromo-2,2,6-trifluorobenzo[d][1,3]dioxol-4-yl)methanol 5c

Compound **5b** (4.8 g, 15.3 mmol) was dissolved in dry tetrahydrofuran (150 mL), and the reaction mixture was purged with nitrogen 3 times and cooled to 0 °C. Lithium borohydride (2 M, 27.6 mmol, 13.8 mL) was added. The reaction mixture was heated to 70 °C and stirred for 2 h. The reaction mixture was cooled to room temperature, quenched with water (5 mL), diluted with ethyl acetate (60 mL), and washed with a saturated sodium chloride solution (30 mL × 2). The organic phases were combined, dried over anhydrous sodium sulfate, and filtered. The filtrate was concentrated under reduced pressure, and the resulting residue was purified by silica gel column chromatography with eluent system A to give the title product **5c** (3.2 g, yield: 73.2%).

¹H NMR (500 MHz, CDCl₃) δ 7.04 (d, 1H), 4.76 (d, 2H).

### Step 4

### 1-((7-bromo-2,2,6-trifluorobenzo[d][1,3]dioxol-4-yl)methyl)-1H-pyrazole 5d

Compound **5c** (3.2 g, 11.2 mmol) and compound **2e** (1.8 g, 12.3 mmol) were dissolved in acetonitrile (55 mL), and cesium carbonate (5.5 g, 16.9 mmol) was added. The mixture was left to react at 70 °C for 1 h. The reaction mixture was filtered, and the filtrate was concentrated under reduced pressure. The resulting residue was purified by silica gel column chromatography with eluent system A to give the title product **5d** (2.61 g, yield: 69.3%).

MS m/z (ESI): 336.9 [M+1].

¹H NMR (500 MHz, CDCl₃) δ 7.59 (d, 1H), 7.51 (d, 1H), 6.67 (d, 1H), 6.35 (t, 1H), 5.33 (s, 2H).

### Step 5

### 7-((1H-pyrazol-1-yl)methyl)-2,2,5-trifluorobenzo[d][1,3]dioxole-4-carbonitrile 5e

Compound **5d** (1.6 g, 4.77 mmol) and cuprous cyanide (3.2 g, 11.2 mmol) were dissolved in *N*-methylpyrrolidinone (50 mL), and the reaction mixture was left to react at 200 °C for 1 h. The reaction mixture was cooled to room temperature, diluted with dichloromethane (60 mL), and washed with a saturated sodium chloride solution (60 mL × 2). The organic phases were combined, dried over anhydrous sodium sulfate, and filtered. The filtrate was concentrated under reduced pressure, and the resulting residue was purified by silica gel column chromatography with eluent system A to give the title product **Se** (465 mg, yield: 34.6%).

MS m/z (ESI): 282.0 [M+1].

### Step 6

### 4-((1H-pyrazol-1-yl)methyl)-2,2-difluoro-[1,3]dioxolo[4',5':5,6]benzo[1,2-d]isoxazol-8-amine 5f

Compound **Se** (1 g, 3.55 mmol) and acetohydroxamic acid (800 mg, 10.66 mmol) were dissolved in 20 mL of a mixed solvent of *N,N-*dimethylformamide and water (V:V = 7:1), and potassium carbonate (2.95 g, 21.33 mmol) was added. The reaction mixture was stirred at 70 °C for 30 min. The reaction mixture was cooled to room temperature and filtered, and the filtrate was concentrated under reduced pressure. The resulting residue was purified by silica gel column chromatography with eluent system A to give the title product **Sf** (60 mg, yield: 5.7%).

MS m/z (ESI): 295.0 [M+1].

### Step 7

### N-(4-((1H-pyrazol-1-yl)methyl)-2,2-difluoro-[1,3]dioxolo[4',5':5,6]benzo[1,2-d]isoxazo l-8-yl)-2,6-dimethoxybenzenesulfonamide 5

Compound **5f** (60 mg, 0.20 mmol) and compound **1o** (144 mg, 0.61 mmol) were dissolved in acetonitrile (5 mL), and dimethyl sulfoxide (1 mg, 0.01 mmol) and 3,5-dimethylpyridine (87 mg, 0.81 mmol) were added. The reaction mixture was left to react at 35 °C for 2 d. The reaction mixture was filtered, and the filtrate was concentrated under reduced pressure. The resulting residue was purified by high performance liquid chromatography (welch Prep C18 5 µm 30 × 150 mm; mobile phases: A-aqueous phase (10 mM ammonium bicarbonate): B-acetonitrile = 25%-45% (20 min), flow rate: 30 mL/min) to give the title product 5 (55 mg, yield: 54.5%).

MS m/z (ESI): 495.0 [M+1].

¹H NMR (500 MHz, CDCl₃) δ 7.65-7.41 (m, 2H), 7.29 (m, 2H), 6.90 (s, 1H), 6.65 (t, 2H), 6.35 (s, 1H), 5.48 (s, 2H), 3.99-3.89 (s, 6H).

### Example 6

### N-(5-((1H-pyrazol-1-yl)methyl)-3,4-dihydro-2H-chromeno[8,7-d]isoxazol-9-yl)-6-meth oxy-2,3-dihydro-1H-indene-5-sulfonamide 6

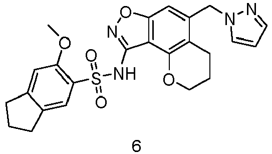

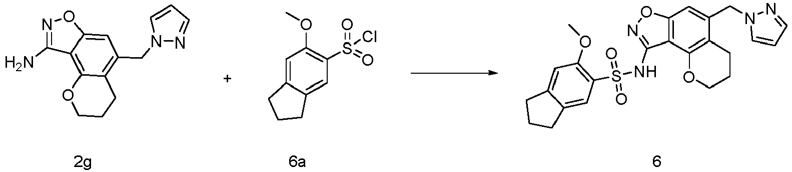

Compound **2g** (50 mg, 0.185 mmol) and 6-methoxy-2,3-dihydro-1*H*-indene-5-sulfonyl chloride **6a** (200 mg, 0.811 mmol, prepared by the method for "intermediate I108 disclosed on page 114 of the specification of the patent application WO2019243491A1") were dissolved in pyridine (2.0 mL), and the solution was purged with nitrogen 3 times. The reaction mixture was microwaved at 120 °C for 3 h. The reaction mixture was cooled to room temperature and concentrated under reduced pressure. The resulting residue was purified by high performance liquid chromatography (Sharpsil-T Prep C18 5 µm 30 × 150 mm; mobile phases: A-aqueous phase (10 mM ammonium bicarbonate): B-acetonitrile = 20%-45% (15 min), flow rate: 30 mL/min) to give the title product **6** (30 mg, yield: 33.7%).

MS m/z (ESI): 480.8 [M+1].

¹H NMR (500 MHz, DMSO-*d*₆) δ 9.59 (s, 1H), 7.78 (d, 1H), 7.63 (s, 1H), 7.51 (d, 1H), 7.08 (s, 1H), 6.44 (s, 1H), 6.31 (d, 1H), 5.41 (s, 2H), 4.24 (t, 2H), 3.79 (s, 3H), 2.89 (t, 2H), 2.83 (t, 2H), 2.69 (t, 2H), 2.04-1.99 (m, 4H).

### Example 7

### N-(5-((1H-pyrazol-1-yl)methyl)-3,4-dihydro-2H-chromeno[8,7-d]isoxazol-9-yl)-2-ethox y-4-methylbenzenesulfonamide 7

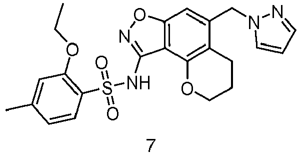

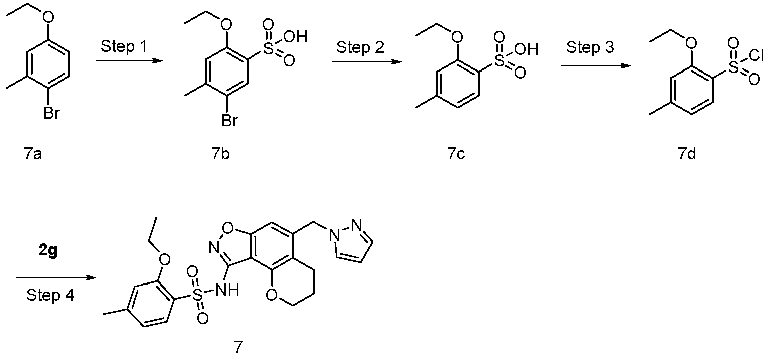

### Step 1

### 5-bromo-2-ethoxy-4-methylbenzenesulfonic acid 7b

The compound 1-bromo-4-ethoxy-2-methylbenzene **7a** (2.0 g, 9.30 mmol) was dissolved in concentrated sulfuric acid (3.6 mL), and the solution was stirred overnight at room temperature. The reaction mixture was poured into ice water (20 mL), concentrated under reduced pressure to remove most of the water, washed with cyclohexane (20 mL), and filtered. The filter cake was washed with ethyl acetate (20 mL) and then ether (20 mL), collected, and dried under vacuum to give the title product **7b** (2.5 g, yield: 91.1%).

MS m/z (ESI): 295.1 [M-1]

### Step 2

### 2-ethoxy-4-methylbenzenesulfonic acid 7c

Compound **7b** (2.5 g, 8.47 mmol) was dissolved in methanol (20 mL), and palladium on carbon (226 mg, 50% water) was added. Hydrogen gas was bubbled into the mixture, and the mixture was heated to 70 °C and left to react for 16 h. The reaction mixture was filtered, and the filtrate was concentrated under reduced pressure to give the title product **7c** (1.8 g, yield: 98.3%).

MS m/z (ESI): 215.1 [M-1]

### Step 3

### 2-ethoxy-4-methylbenzenesulfonyl chloride 7d

Compound **7c** (200 mg, 0.92 mmol) was added to a flask, and thionyl chloride (771 mg, 6.48 mmol) was slowly added dropwise. The mixture was left to react at 85 °C for 3 h. The reaction mixture was concentrated under reduced pressure, and the resulting residue was purified by silica gel column chromatography with eluent system A to give the title product **7d** (200 mg, yield: 92.1%).

### Step 4

### N-(5-((1H-pyrazol-1-yl)methyl)-3,4-dihydro-2H-chromeno[8,7-d]isoxazol-9-yl)-2-ethox y-4-methylbenzenesulfonamide 7

Compound **2g** (50 mg, 0.185 mmol) and compound **7d** (100 mg, 0.426 mmol) were dissolved in pyridine (2.0 mL), and the solution was purged with nitrogen 3 times. The reaction mixture was microwaved at 120 °C for 3 h. The reaction mixture was cooled to room temperature and concentrated under reduced pressure. The resulting residue was purified by high performance liquid chromatography (Sharpsil-T Prep C18 5 µm 30 × 150 mm; mobile phases: A-aqueous phase (10 mM ammonium bicarbonate): B-acetonitrile = 20%-45% (15 min), flow rate: 30 mL/min) to give the title product **7** (30 mg, yield: 34.6%).

MS m/z (ESI): 468.8 [M+1].

¹H NMR (500 MHz, DMSO-*d*₆) δ 9.32 (s, 1H), 7.78 (d, 1H), 7.71 (d, 1H), 7.51 (d, 1H), 7.01 (s, 1H), 6.89 (d, 1H), 6.44 (s, 1H), 6.32 (t, 1H), 5.41 (s, 2H), 4.25-4.19 (m, 2H), 4.10 (q, 2H), 2.70 (t, 2H), 2.34 (s, 3H), 2.02-1.97 (m, 2H), 1.26 (t, 3H).

### Example 8

### N-(5-((1H-pyrazol-1-yl)methyl)-3,4-dihydro-2H-chromeno[8,7-d]isoxazol-9-yl)-4-meth yl-2-(2,2,2-trifluoroethoxy)benzenesulfonamide 8

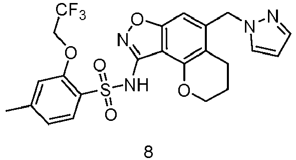

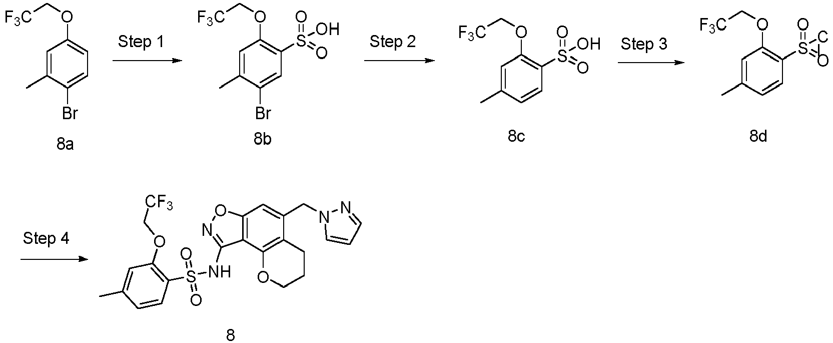

### Step 1

### 5-bromo-4-methyl-2-(2,2,2-trifluoroethoxy)benzenesulfonic acid 8b

1-Bromo-2-methyl-4-(2,2,2-trifluoroethoxy)benzene **8a** (1.2 g, 4.46 mmol, prepared by the method for "intermediate A30 disclosed on page 71 of the specification of the patent application WO2020069322A1") was dissolved in concentrated sulfuric acid (2 mL), and the solution was stirred overnight at room temperature. The reaction mixture was poured into ice water (20 mL), concentrated under reduced pressure to remove most of the water, washed with cyclohexane (20 mL), and filtered. The filter cake was washed with ethyl acetate (20 mL) and then ether (20 mL), collected, and dried under vacuum to give the title product **8b** (1.5 g, yield: 96.3%).

MS m/z (ESI): 349.1 [M-1]

### Step 2

### 4-methyl-2-(2,2,2-trifluoroethoxy)benzenesulfonic acid 8c

Compound **8b** (1.7 g, 4.87 mmol) was dissolved in methanol (10 mL), and palladium on carbon (130 mg, 50% water) was added. Hydrogen gas was bubbled into the mixture, and the mixture was heated to 70 °C and left to react for 16 h. The reaction mixture was filtered, and the filtrate was concentrated under reduced pressure to give the title product **8c** (1.3 g, yield: 98.8%).

MS m/z (ESI): 269.2 [M-1]

### Step 3

### 4-methyl-2-(2,2,2-trifluoroethoxy)benzenesulfonyl chloride 8d

Compound **8c** (500 mg, 1.85 mmol) was added to a flask, and thionyl chloride (1.55g, 13.02 mmol) was slowly added dropwise. The mixture was left to react at 85 °C for 3 h. The reaction mixture was concentrated under reduced pressure, and the resulting residue was purified by silica gel column chromatography with eluent system A to give the title product **8d** (400 mg, yield: 74.9%).

MS m/z (ESI): 287.2 [M-1]

### Step 4

### N-(5-((1H-pyrazol-1-yl)methyl)-3,4-dihydro-2H-chromeno[8,7-d]isoxazol-9-yl)-4-meth yl-2-(2,2,2-trifluoroethoxy)benzenesulfonamide 8

Compound **2g** (50 mg, 0.185 mmol) and compound **8d** (130 mg, 0.450 mmol) were dissolved in pyridine (2.0 mL), and the solution was purged with nitrogen 3 times. The reaction mixture was microwaved at 120 °C for 3 h. The reaction mixture was cooled to room temperature and concentrated under reduced pressure. The resulting residue was purified by high performance liquid chromatography (Sharpsil-T Prep C18 5 µm 30 × 150 mm; mobile phases: A-aqueous phase (10 mM ammonium bicarbonate): B-acetonitrile = 20%-45% (15 min), flow rate: 30 mL/min) to give the title product **8** (10 mg, yield: 10.3%).

MS m/z (ESI): 522.8 [M+1].

1H NMR (500 MHz, DMSO-*d*₆) δ 9.39 (s, 1H), 7.79-7.77 (m, 2H), 7.51 (d, 1H), 7.20 (s, 1H), 7.04 (d, 1H), 6.44 (s, 1H), 6.32 (t, 1H), 5.41 (s, 2H), 4.89 (d, 2H), 4.21 (t, 2H), 2.69 (t, 2H), 2.37 (s, 3H), 2.01-1.98 (m, 2H).

### Example 9

### N-(5-((1H-pyrazol-1-yl)methyl)-3,4-dihydro-2H-chromeno[8,7-d]isoxazol-9-yl)-5-fluor o-2-methoxybenzenesulfonamide 9

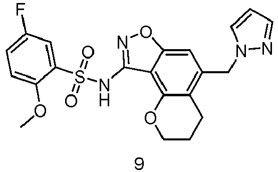

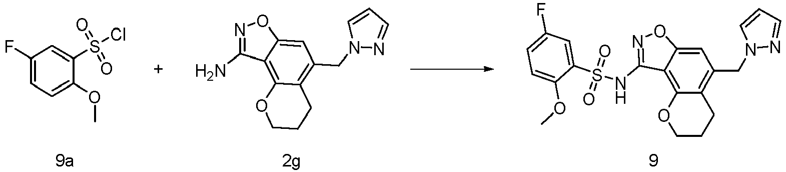

Compound **2g** (50 mg, 0.185 mmol) and 5-fluoro-2-methoxybenzenesulfonyl chloride **9a** (200 mg, 0.890 mmol) were dissolved in pyridine (2.0 mL), and the solution was purged with nitrogen 3 times. The reaction mixture was microwaved at 120 °C for 3 h. The reaction mixture was cooled to room temperature and concentrated under reduced pressure. The resulting residue was purified by high performance liquid chromatography (Sharpsil-T Prep C18 5 µm 30 × 150 mm; mobile phases: A-aqueous phase (10 mM ammonium bicarbonate): B-acetonitrile = 30%-45% (15 min), flow rate: 30 mL/min) to give the title product 9 (15 mg, yield: 17.6%).

MS m/z (ESI): 458.9 [M+1].

¹H NMR (500 MHz, DMSO-*d*₆) δ 10.45 (s, 1H), 7.79 (d, 1H), 7.57 (dd, 1H), 7.58-7.56 (m, 2H), 7.27 (m, 1H), 6.47 (s, 1H), 6.32 (t, 1H), 5.42 (s, 2H), 4.17 (t, 2H), 3.79 (s, 3H), 2.69 (t, 2H), 2.10-1.94 (m, 2H).

### Example 10

### N-(5-((1H-pyrazol-1-yl)methyl)-3,4-dihydro-2H-chromeno[8,7-d]isoxazol-9-yl)-2-meth oxy-6-(trifluoromethoxy)benzenesulfonamide 10

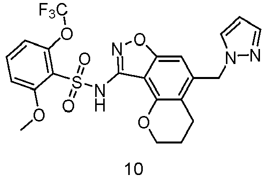

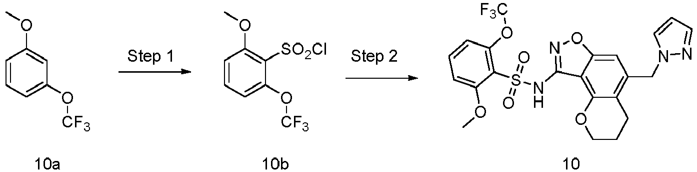

### Step 1

### 2-methoxy-6-(trifluoromethoxy)benzenesulfonyl chloride 10b

To a 100 mL three-necked flask were added 1-methoxy-3-(trifluoromethoxy)benzene **10a** (500 mg, 2.55 mmol), anhydrous tetrahydrofuran (10 mL) and tetramethylethylenediamine (616 mg, 5.30 mmol). The mixture was cooled to -78 °C in a nitrogen atmosphere, and n-butyllithium (1.3 mL, 2.5 M, 3.25 mmol) was added dropwise. After the addition, the reaction was stirred at -78 °C for another 1 h, and sulfuryl chloride (0.3 mL, 3.71 mmol) was added. After the addition, the reaction was warmed to room temperature and stirred for 1 h. Water (20 mL) was added, and the mixture was extracted with ethyl acetate (20 mL). The organic phase was concentrated, and the resulting residue was purified by silica gel column chromatography with eluent system A to give the title product **10b** (200 mg, yield: 26.9%).

¹H NMR (500 MHz, CDCl₃) δ 7.70 (t, 1H), 7.12 (d, 1H), 7.05 (dt, 1H), 4.10 (s, 3H).

### Step 2

### N-(5-((1H-pyrazol-1-yl)methyl)-3,4-dihydro-2H-chromeno[8,7-d]isoxazol-9-yl)-2-meth oxy-6-(trifluoromethoxy)benzenesulfonamide 10

Compound **2g** (50 mg, 0.185 mmol) and compound **10b** (200 mg, 0.688 mmol) were dissolved in pyridine (2.0 mL), and the solution was purged with nitrogen 3 times. The reaction mixture was microwaved at 120 °C for 3 h. The reaction mixture was cooled to room temperature and concentrated under reduced pressure. The resulting residue was purified by high performance liquid chromatography (Sharpsil-T Prep C18 5 µm 30 × 150 mm; mobile phases: A-aqueous phase (10 mM ammonium bicarbonate): B-acetonitrile = 20%-45% (15 min), flow rate: 30 mL/min) to give the title product 10 (3.0 mg, yield: 3.09%).

MS m/z (ESI): 524.8 [M+1].

¹H NMR (500 MHz, CD₃OD) δ 7.66 (d, 1H), 7.60-7.56 (m, 2H), 7.15 (d, 1H), 7.02 (d, 1H), 6.40-6.38 (m, 2H), 5.43 (s, 2H), 4.28-4.26 (m, 2H), 3.85 (s, 3H), 2.69 (t, 2H), 2.12-2.07 (m, 2H).

### Example 11

### N-(5-((1H-pyrazol-1-yl)methyl)-3,4-dihydro-2H-chromeno[8,7-d]isoxazol-9-yl)-4-(dim ethylamino)-2-methoxybenzenesulfonamide 11

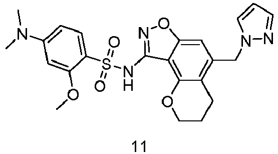

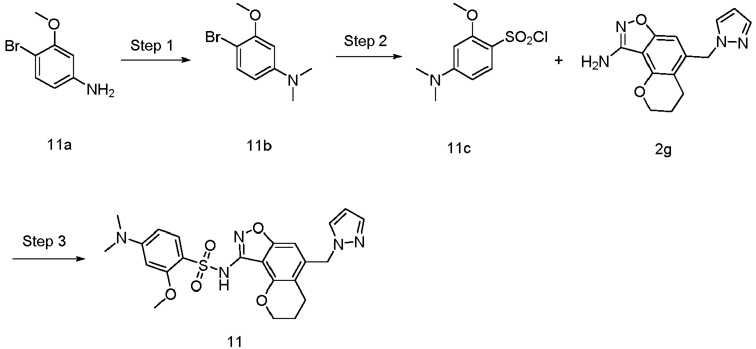

### Step 1

### 4-bromo-3-methoxy-N,N-dimethylaniline 11b

To a 100 mL flask were added 4-bromo-3-methoxyaniline **11a** (2.0 g, 9.90 mmol), 37% aqueous formaldehyde (9.0 g, 97.8 mmol), acetic acid (9.0 g, 150 mmol) and acetonitrile (30 mL). The mixture was stirred at room temperature for 30 min, and sodium cyanoborohydride (800 mg, 12.7 mmol) was added under ice-bath conditions. The mixture was stirred at room temperature for 16 h. Water (100 mL) was added, and the mixture was extracted with ethyl acetate (50 mL). The organic phase was concentrated, and the resulting residue was purified by silica gel column chromatography with eluent system A to give the title product **11b** (930 mg, yield: 40.8%).

MS m/z (ESI): 230.0 [M+1].

¹H NMR (500 MHz, CDCl₃) δ 7.33 (d, 1H), 6.28 (d, 1H), 6.24 (dd, 1H), 3.91 (s, 3H), 2.97 (s, 6H).

### Step 2

### 4-(dimethylamino)-2-methoxybenzenesulfonyl chloride 11c To a 100 mL three-necked flask were added 11b (900 mg, 3.91 mmol) and anhydrous tetrahydrofuran (15 mL). The mixture was cooled to -70 °C in a nitrogen atmosphere, and n-butyllithium (2.0 mL, 2.5 M, 5.0 mmol) was added dropwise. After the addition, the mixture was stirred at -70 °C for another 1 h. Sulfur dioxide prepared in-house was bubbled into the mixture for 10 min, and the mixture was then stirred for 30 min. Then N-chlorosuccinimide (700 mg, 5.24 mmol) was added. After the addition, the mixture was warmed to room temperature and left to react for 30 min. Water (20 mL) was added, and the mixture was extracted with ethyl acetate (20 mL). The organic phase was concentrated, and the resulting residue was purified by silica gel column chromatography with eluent system A to give the title product 11c (50 mg, yield: 5.11%).

MS m/z (ESI): 232.0 [M-Cl+OH+1].

### Step 3

### N-(5-((1H-pyrazol-1-yl)methyl)-3,4-dihydro-2H-chromeno[8,7-d]isoxazol-9-yl)-4-(dim ethylamino)-2-methoxybenzenesulfonamide 11

Compound **2g** (40 mg, 0.148 mmol) and compound **11c** (50 mg, 0.200 mmol) were dissolved in pyridine (2.0 mL), and the solution was purged with nitrogen 3 times. The reaction mixture was microwaved at 120 °C for 3 h. The reaction mixture was cooled to room temperature and concentrated under reduced pressure. The resulting residue was purified by high performance liquid chromatography (Sharpsil-T Prep C18 5 µm 30 × 150 mm; mobile phases: A-aqueous phase (10 mM ammonium bicarbonate): B-acetonitrile = 10%-45% (15 min), flow rate: 30 mL/min) to give the title product **11** (2.0 mg, yield: 2.79%).

MS m/z (ESI): 483.9 [M+1].

¹H NMR (500 MHz, CD₃OD) δ 7.72 (d, 1H), 7.66 (d, 1H), 7.57 (d, 1H), 6.43-6.38 (m, 2H), 6.33 (dd, 1H), 6.19 (d, 1H), 5.42 (s, 2H), 4.38 (t, 2H), 3.87 (s, 3H), 3.03 (s, 6H), 2.70 (t, 2H), 2.18-2.12 (m, 2H).

### Example 12

### N-(5-((1H-pyrazol-1-yl)methyl)-3,4-dihydro-2H-chromeno[8,7-d]isoxazol-9-yl)-2,3-dih ydrobenzofuran-7-sulfonamide 12

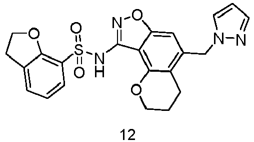

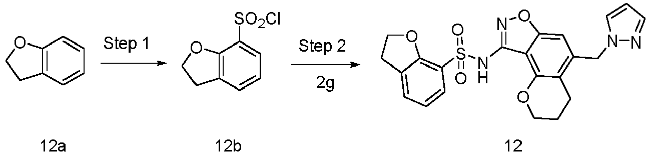

### Step 1

### 2,3-dihydrobenzofuran-7-sulfonyl chloride 12b

To a 100 mL flask were added 2,3-dihydrobenzofuran 12a (2.0 g, 16.6 mmol), sulfur trioxide *N*,*N*-dimethylformamide complex (3.0 g, 19.6 mmol) and 1,2-dichloroethane (10 mL). The reaction was stirred at 80 °C for 1 h. The reaction mixture was cooled to room temperature, and thionyl chloride (2.2 g, 18.5 mmol) was added dropwise. After the addition, the mixture was heated to 70 °C and left to react for 2 h. Then the reaction mixture was cooled to room temperature, poured into ice water (50 mL), and extracted with ethyl acetate (50 mL). The organic phase was concentrated, and the resulting residue was purified by silica gel column chromatography with eluent system A to give the title product **12b** (3.2 g, yield: 87.9%).

1H NMR (500 MHz, CDCl₃) δ 7.89-7.86 (m, 2H), 6.93 (d, 1H), 4.78 (t, 2H), 3.35 (t, 2H).

### Step 2

### N-(5-((1H-pyrazol-1-yl)methyl)-3,4-dihydro-2H-chromeno[8,7-d]isoxazol-9-yl)-2,3-dih ydrobenzofuran-7-sulfonamide 12

Compound **2g** (50 mg, 0.185 mmol) and compound **12b** (200 mg, 0.915 mmol) were dissolved in pyridine (2.0 mL), and the solution was purged with nitrogen 3 times. The reaction mixture was microwaved at 120 °C for 3 h. The reaction mixture was cooled to room temperature and concentrated under reduced pressure. The resulting residue was purified by high performance liquid chromatography (Sharpsil-T Prep C18 5 µm 30 × 150 mm; mobile phases: A-aqueous phase (10 mM ammonium bicarbonate): B-acetonitrile = 30%-45% (15 min), flow rate: 30 mL/min) to give the title product **12** (20.0 mg, yield: 23.9%).

MS m/z (ESI): 452.9 [M+1].

¹H NMR (500 MHz, DMSO-*d*₆) δ 10.56 (s, 1H), 7.83 (d, 1H), 7.78 (d, 1H), 7.76 (d, 1H), 7.52 (d, 1H), 6.93 (d, 1H), 6.44 (s, 1H), 6.32 (t, 1H), 5.42 (s, 2H), 4.64 (t, 2H), 4.30-4.20 (m, 2H), 3.25 (t, 2H), 2.69 (t, 2H), 2.17-1.95 (m, 2H).

### Example 13

### N-(5-((1H-pyrazol-1-yl)methyl)-3,4-dihydro-2H-chromeno[8,7-d]isoxazol-9-yl)-4-ethyl -2-methoxybenzenesulfonamide 13

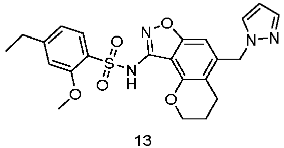

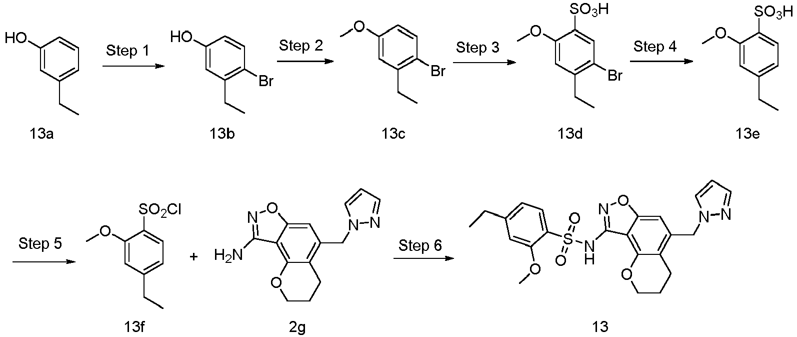

### Step 1

### 4-bromo-3-ethylphenol 13b

The compound 3-ethylphenol **13a** (4.0 g, 32.7 mmol, adamas) was dissolved in dichloromethane (30 mL), and tetrabutylammonium tribromide (16.0 g, 33.2 mmol) was added. The mixture was stirred at room temperature for 2 h. The reaction mixture was washed with 1 M dilute hydrochloric acid (50 mL), water and then a saturated aqueous sodium chloride solution, and concentrated under reduced pressure. The resulting residue was purified by silica gel column chromatography with eluent system A to give the title product **13b** (6.3 g, yield: 95.7%).

¹H NMR (500 MHz, CDCl₃) δ 7.38 (d, 1H), 6.76 (d, 1H), 6.58 (dd, 1H), 4.83 (s, 1H), 2.71 (q, 2H), 1.23 (t, 3H).

### Step 2

### 1-bromo-2-ethyl-4-methoxybenzene 13c

Compound **13b** (3.0 g, 14.9 mmol) was dissolved in *N,N-*dimethylformamide (40 mL), and potassium carbonate (4.1 g, 29.7 mmol) and iodomethane (2.6 g, 18.3 mmol) were added. The mixture was left to react at room temperature for 16 h. The reaction mixture was diluted with ethyl acetate (100 mL), washed with water (100 mL) and then a saturated aqueous sodium chloride solution (100 mL), and concentrated under reduced pressure. The resulting residue was purified by silica gel column chromatography with eluent system A to give the title product **13e** (2.5 g, yield: 77.9%).

¹H NMR (500 MHz, CDCl₃) δ 7.42 (d, 1H), 6.81 (d, 1H), 6.64 (dd, 1H), 3.81 (s, 3H), 2.74 (q, 2H), 1.24 (t, 3H).

### Step 3

### 5-bromo-4-ethyl-2-methoxybenzenesulfonic acid 13d

Compound **13e** (2.5 g, 11.6 mmol) was slowly added to concentrated sulfuric acid (6.0 mL) under ice-bath conditions, and the mixture was stirred at room temperature for 2 h. The reaction mixture was poured into ice water (90 mL), and the pH of the reaction mixture was adjusted to 3 with 20% aqueous sodium hydroxide. The mixture was filtered, and the filter cake was washed with water (100 mL) and dried to give the title product **13d** (1.5 g, yield: 43.7%).

MS m/z (ESI): 293.1 [M-1].

### Step 4

### 4-ethyl-2-methoxybenzenesulfonic acid 13e

Compound **13d** (1.5 g, 5.08 mmol) was dissolved in methanol (20 mL), and palladium on carbon (600 mg, 10%, 50% water) was added. Hydrogen gas was bubbled into the mixture, and the reaction was heated to 60 °C and stirred for 16 h. The reaction mixture was filtered, and the filtrate was concentrated under reduced pressure to give the title product **13e** (1.1 g, yield: 100%).

MS m/z (ESI): 215.2 [M-1].

### Step 5

### 4-ethyl-2-methoxybenzenesulfonyl chloride 13f

Compound **13e** (400 mg, 1.85 mmol) was added to a flask, and thionyl chloride (4.0 mL) was slowly added dropwise. The mixture was left to react at 85 °C for 3 h. The reaction mixture was concentrated under reduced pressure, and the resulting residue was purified by silica gel column chromatography with eluent system A to give the title product **13f** (200 mg, yield: 46.1%).

MS m/z (ESI): 215.2 [M-Cl+OH-1].

### Step 6

### N-(5-((1H-pyrazol-1-yl)methyl)-3,4-dihydro-2H-chromeno[8,7-d]isoxazol-9-yl)-4-ethyl -2-methoxybenzenesulfonamide 13

Compound **13f** (200 mg, 0.852 mmol) and compound **2g** (60 mg, 0.222 mmol) were dissolved in pyridine (2.0 mL), and the solution was purged with nitrogen 3 times. The reaction mixture was microwaved at 120 °C for 3 h. The reaction mixture was cooled to room temperature and concentrated under reduced pressure. The resulting residue was purified by high performance liquid chromatography (Sharpsil-T Prep C18 5 µm 30 × 150 mm; mobile phases: A-aqueous phase (10 mM ammonium bicarbonate): B-acetonitrile = 15%-45% (15 min), flow rate: 30 mL/min) to give the title product **13** (15.0 mg, yield: 14.4%).

MS m/z (ESI): 468.9 [M+1].

¹H NMR (500 MHz, DMSO-*d*₆) δ 9.77 (s, 1H), 7.78 (d, 1H), 7.70 (d, 1H), 7.51 (d, 1H), 7.03 (s, 1H), 6.92 (d, 1H), 6.43 (s, 1H), 6.31 (t, 1H), 5.40 (s, 2H), 4.24-4.18 (m, 2H), 3.81 (s, 3H), 2.69 (t, 2H), 2.64 (t, 2H), 2.01-1.97 (m, 2H), 1.19 (t, 3H).

### Example 14

### N-(5-((1H-pyrazol-1-yl)methyl)-3,4-dihydro-2H-chromeno[8,7-d]isoxazol-9-yl)-4-isopr opyl-2-methoxybenzenesulfonamide 14

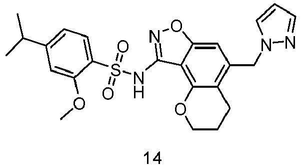

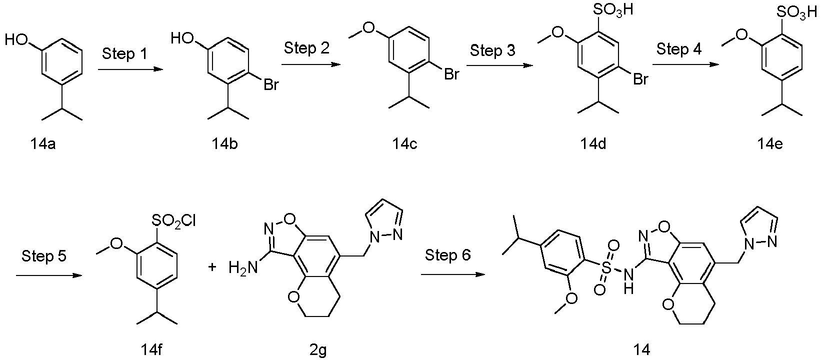

### Step 1

### 4-bromo-3-isopropylphenol 14b

The compound 3-isopropylphenol **14a** (2.0 g, 14.7 mmol) was dissolved in dichloromethane (30 mL) and methanol (10 mL), and tetrabutylammonium tribromide (7.1 g, 14.7 mmol) was added. The mixture was stirred at room temperature for 2 h. The reaction mixture was washed with 1 M dilute hydrochloric acid (50 mL), water and then a saturated aqueous sodium chloride solution, and concentrated under reduced pressure. The resulting residue was purified by silica gel column chromatography with eluent system A to give the title product **14b** (2.9 g; ¹NMR analysis showed that about 20% of the product was an isomer with Br at the para position of the isopropyl group; overall yield: 95.7%; directly used in the next step).

¹H NMR (500 MHz, CDCl₃) δ 7.38 (d, 1H), 6.79 (d, 1H), 6.57 (dd, 1H), 4.99 (s, 1H), 3.31 (m, 1H), 1.24 (d, 6H).

### Step 2

### 1-bromo-2-isopropyl-4-methoxybenzene 14c

Compound **14b** (2.9 g, 13.5 mmol) was dissolved in *N,N*-dimethylformamide (30 mL), and potassium carbonate (3.7 g, 26.8 mmol) and iodomethane (2.28 g, 16.1 mmol) were added. The mixture was left to react at room temperature for 16 h. The reaction mixture was diluted with ethyl acetate (100 mL), washed with water (100 mL) and then a saturated aqueous sodium chloride solution (100 mL), and concentrated under reduced pressure. The resulting residue was purified by silica gel column chromatography with eluent system A to give the title product **14e** (2.4 g; ¹NMR analysis showed that about 10% of the product was an isomer with Br at the para position of the isopropyl group; overall yield: 77.7%; directly used in the next step).

¹H NMR (500 MHz, CDCl₃) δ 7.44 (d, 1H), 6.86 (d, 1H), 6.64 (dd, 1H), 3.81 (s, 3H), 3.33 (m, 1H), 1.25 (d, 6H).

### Step 3

### 5-bromo-4-isopropyl-2-methoxybenzenesulfonic acid 14d

Compound **14e** (2.4 g, 10.5 mmol) was slowly added to concentrated sulfuric acid (6.0 mL) under ice-bath conditions, and the mixture was stirred at room temperature for 2 h. The reaction mixture was poured into ice water (90 mL), and the pH of the reaction mixture was adjusted to 3 with 20% aqueous sodium hydroxide. The mixture was filtered, and the filter cake was washed with water (100 mL) and dried to give the title product **14d** (2.4 g, yield: 74.1%).

MS m/z (ESI): 307.1 [M-1].

### Step 4

### 4-isopropyl-2-methoxybenzenesulfonic acid 14e

Compound **14d** (2.4 g, 7.76 mmol) was dissolved in methanol (30 mL), and palladium on carbon (1.0 g, 10%, 50% water) was added. Hydrogen gas was bubbled into the mixture, and the mixture was heated to 60 °C and left to react for 16 h. The reaction mixture was filtered, and the filtrate was concentrated under reduced pressure to give the title product **14e** (1.3 g, yield: 72.7%).

MS m/z (ESI): 229.2 [M-1].

### Step 5

### 4-isopropyl-2-methoxybenzenesulfonyl chloride 14f

Compound **14e** (360 mg, 1.56 mmol) was added to a flask, and thionyl chloride (4.0 mL) was slowly added dropwise. The mixture was left to react at 85 °C for 3 h. The reaction mixture was concentrated under reduced pressure, and the resulting residue was purified by silica gel column chromatography with eluent system A to give the title product **14f** (250 mg, yield: 64.3%).

MS m/z (ESI): 229.2 [M-Cl+OH-1].

### Step 6

### N-(5-((1H-pyrazol-1-yl)methyl)-3,4-dihydro-2H-chromeno[8,7-d]isoxazol-9-yl)-4-isopr opyl-2-methoxybenzenesulfonamide 14

Compound **14f** (250 mg, 1.01 mmol) and compound **2g** (60 mg, 0.222 mmol) were dissolved in pyridine (2.0 mL), and the solution was purged with nitrogen 3 times. The reaction mixture was microwaved at 120 °C for 3 h. The reaction mixture was cooled to room temperature and concentrated under reduced pressure. The resulting residue was purified by high performance liquid chromatography (Sharpsil-T Prep C18 5 µm 30 × 150 mm; mobile phases: A-aqueous phase (10 mM ammonium bicarbonate): B-acetonitrile = 15%-45% (15 min), flow rate: 30 mL/min) to give the title product **14** (10.0 mg, yield: 9.34%).

MS m/z (ESI): 482.9 [M+1].

1H NMR (500 MHz, DMSO-*d*₆) δ 9.94 (s, 1H), 7.75 (d, 1H), 7.68 (d, 1H), 7.51 (d, 1H), 6.94 (s, 1H), 6.87 (d, 1H), 6.31 (d, 2H), 5.37 (s, 2H), 4.24-4.16 (m, 2H), 3.77 (s, 3H), 2.91 (m, 1H), 2.67 (t, 2H), 2.06-1.94 (m, 2H), 1.21 (d, 6H).

### Example 15

### N-(4-((1H-pyrazol-1-yl)methyl)-2,3-dihydrobenzofuro[7,6-d]isoxazol-8-yl)-6-methoxy-2,3-dihydro-1H-indene-5-sulfonamide 15

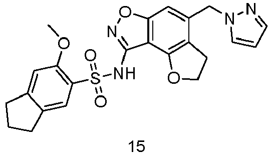

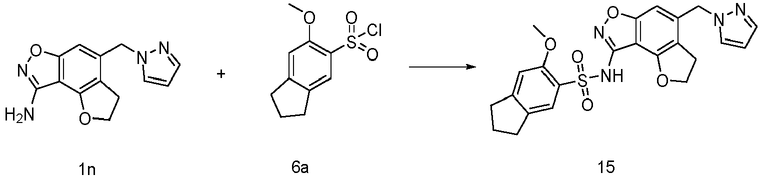

Compound **6a** (289 mg, 1.17 mmol) and compound **1n** (150 mg, 0.59 mmol) were dissolved in pyridine (5.0 mL), and 4-dimethylaminopyridine (15 mg, 0.12 mmol) was added. The mixture was purged with nitrogen 3 times. The reaction mixture was microwaved at 120 °C for 3 h. The reaction mixture was cooled to room temperature and concentrated under reduced pressure. The resulting residue was purified by high performance liquid chromatography (Xtimate phenyl-hexyl Prep C18 5 µm, 30 × 150 mm; mobile phases: A-aqueous phase (0.1% ammonia water): B-acetonitrile = 5%-45% (20 min), flow rate: 30 mL/min) to give the title product **15** (30 mg, yield: 11.0%). MS m/z (ESI): 467.5 [M+1].

¹H NMR (400 MHz, CDCl₃): δ 7.90 (s, 1H), 7.57-7.56 (d, 1H), 7.42-7.41 (d, 1H), 6.84 (s, 1H), 6.64 (s, 1H), 6.33-6.32 (m, 1H), 5.33 (s, 2H), 4.83-4.80 (m, 2H), 3.94 (s, 3H), 3.09-3.05 (m, 2H), 2.94-2.88 (m, 4H), 2.13-2.07 (m, 2H).

### Example 16

### N-(4-((1H-pyrazol-1-yl)methyl)-2,3-dihydrobenzofuro[7,6-d]isoxazol-8-yl)-2-ethoxy-4-methylbenzenesulfonamide 16

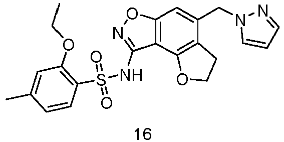

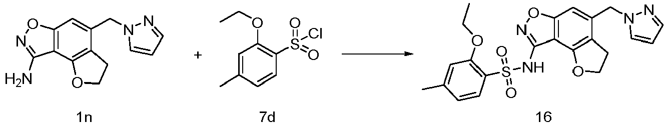

Compound **7d** (92 mg, 0.39 mmol) and compound **1n** (50 mg, 0.20 mmol) were dissolved in pyridine (5.0 mL), and 4-dimethylaminopyridine (3 mg, 0.02 mmol) was added. The mixture was purged with nitrogen 3 times. The reaction mixture was microwaved at 120 °C for 3 h. The reaction mixture was cooled to room temperature and concentrated under reduced pressure. The resulting residue was purified by high performance liquid chromatography (Xtimate phenyl-hexyl Prep C18 5 µm, 30 × 150 mm; mobile phases: A-aqueous phase (0.1% ammonia water): B-acetonitrile = 5%-45% (20 min), flow rate: 30 mL/min) to give the title product **16** (10 mg, yield: 11.3%).

MS m/z (ESI): 455.5 [M+1].

¹H NMR (400 MHz, CDCl₃): δ 7.97-7.96 (d, 1H), 7.56-7.55 (d, 1H), 7.42-7.41 (d, 1H), 6.89-6.87 (d, 1H), 6.75 (s, 1H), 6.66 (s, 1H), 6.33-6.32 (m, 1H), 5.33 (s, 2H), 4.83-4.79 (m, 2H), 4.19-4.15 (m, 2H), 3.09-3.06 (m, 2H), 2.37 (s, 3H), 1.53-1.50 (m, 3H).

### Example 17

### N-(4-((1H-pyrazol-1-yl)methyl)-2,3-dihydrobenzofuro[7,6-d]isoxazol-8-yl)-2-methoxy-6-methylbenzenesulfonamide 17

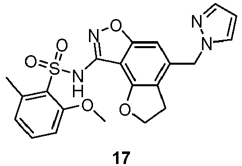

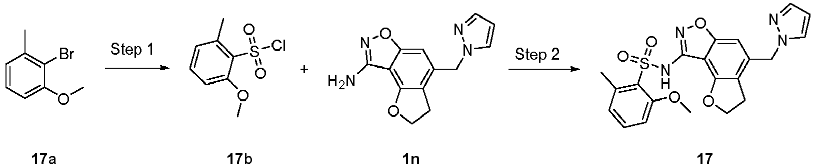

### Step 1

### 2-methoxy-6-methylbenzenesulfonyl chloride 17b

n-Butyllithium (1.0 mL, 2.5 mmol, 2.5 M in n-hexane) was added dropwise to a solution of 2-bromo-1-methoxy-3-methylbenzene **17a** (500.0 mg, 2.5 mmol) in anhydrous diethyl ether (10 mL) at -70 °C. The reaction was stirred at -70 °C for 1 h in a nitrogen atmosphere. Sulfur dioxide gas was bubbled into the reaction mixture at -70 °C for 30 min. A-Chlorosuccinimide (496.0 g, 3.8 mmol) was added, and the mixture was slowly warmed to room temperature and then left to react at room temperature for 2 h. The reaction mixture was washed with a saturated sodium bisulfite solution (20 mL) and a saturated sodium chloride solution (20 mL), dried over anhydrous sodium sulfate, and filtered. The filtrate was concentrated under reduced pressure, and the resulting residue was purified by silica gel column chromatography with eluent system A to give the title product **17b** (390.0 mg, yield: 78.0%).

### Step 2

### N-(4-((1H-pyrazol-1-yl)methyl)-2,3-dihydrobenzofuro[7,6-d]isoxazol-8-yl)-2-methoxy-6-methylbenzenesulfonamide 17

Lithium bis(trimethylsilyl)amide (0.4 mL, 0.4 mmol, 1 M in tetrahydrofuran) was added dropwise to a solution of compound **1n** (60.0 mg, 0.24 mmol) in anhydrous tetrahydrofuran (3 mL) at -70 °C. The mixture was stirred at -70 °C for 1 h in a nitrogen atmosphere. A solution of compound **17b** (78.0 mg, 0.35 mmol) in tetrahydrofuran (0.5 mL) was added dropwise at -70 °C. After the reaction system was slowly warmed to room temperature, the reaction was stirred at room temperature for 16 h. A saturated ammonium chloride solution (5 mL) was added to the reaction mixture, and the mixture was extracted with ethyl acetate (20 mL × 3). The organic phases were combined, dried over anhydrous sodium sulfate, and filtered. The filtrate was concentrated under reduced pressure and purified by high performance liquid chromatography (Welch Xtimate C18, 5 µm, 30 mm × 150 mm; elution system: water (10 mM ammonium bicarbonate) and acetonitrile, from 20% (*v*/*v*) acetonitrile to 34% (*v*/*v*) acetonitrile within 14 min; detection wavelengths: 214&254 nm) to give the title product **17** (25.0 mg, yield: 23.7%).

MS m/z (ESI): 440.9 [M+1].

¹H NMR (500 MHz, CD₃OD): δ 7.70 (d, 1H), 7.54 (d, 1H), 7.36 (dd, 1H), 6.95 (d, 1H), 6.87 (d, 1H), 6.53 (s, 1H), 6.36 (t, 1H), 5.40 (s, 2H), 4.77 (t, 2H), 3.82 (s, 3H), 3.07 (t, 2H), 2.65 (s, 3H).

### Example 18

### N-(4-((1H-pyrazol-1-yl)methyl)-2,3-dihydrobenzofuro[7,6-d]isoxazol-8-yl)-2-methoxy-5-methylbenzenesulfonamide 18

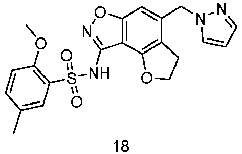

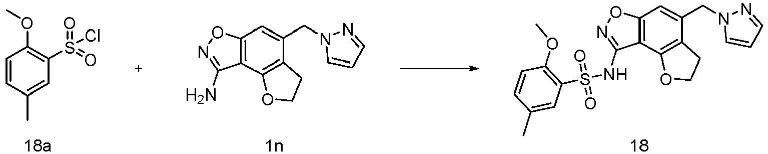

The compound 2-methoxy-5-methylbenzenesulfonyl chloride **18a** (130 mg, 0.59 mmol) and compound **1n** (50 mg, 0.20 mmol) were dissolved in pyridine (5.0 mL), and 4-dimethylaminopyridine (5 mg, 0.04 mmol) was added. The mixture was purged with nitrogen 3 times. The reaction mixture was microwaved at 120 °C for 3 h. The reaction mixture was cooled to room temperature and concentrated under reduced pressure. The resulting residue was purified by high performance liquid chromatography (Xtimate phenyl-hexyl Prep C18 5 µm, 30 × 150 mm; mobile phases: A-aqueous phase (0.1% ammonia water): B-acetonitrile = 5%-45% (20 min), flow rate: 30 mL/min) to give the title product **18** (3 mg, yield: 3.5%).

MS m/z (ESI): 441.3 [M+1].

¹H NMR (400 MHz, CDCl₃): δ 7.80 (s, 1H), 7.57-7.56 (m, 2H), 7.49-7.48 (m, 2H), 6.30-6.29 (m, 2H), 5.37 (s, 2H), 4.70-4.67 (m, 2H), 3.71 (s, 3H), 3.09-3.06 (m, 2H), 2.25 (s, 3H).

### Example 19

### N-(4-((1H-pyrazol-1-yl)methyl)-2,3-dihydrobenzofuro[7,6-d]isoxazol-8-yl)-5-methoxy-2,3-dihydrobenzofuran-6-sulfonamide 19

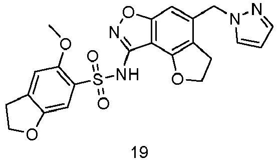

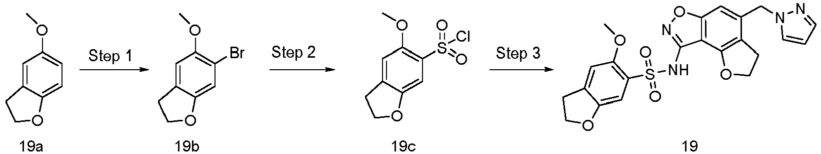

### Step 1

### 6-bromo-5-methoxy-2,3-dihydrobenzofuran 19b

5-Methoxy-2,3-dihydrobenzofuran **19a** (250 mg, 1.66 mmol, prepared by the method for "intermediate 1D disclosed on page 65 of the specification of the patent application WO2017218960A1") was added to dichloromethane (5 mL), and 1,3-dibromo-5,5-dimethylimidazolidin-2,4-dione (291 mg, 0.67 mmol) was slowly added at 0 °C. The mixture was left to react at 0 °C for 2 h. The reaction mixture was concentrated under reduced pressure, and the resulting residue was purified by silica gel column chromatography with eluent system A to give the title product **19b** (330 mg, yield: 86.5%).

MS m/z (ESI): 229.1 [M+1].

### Step 2

### 5-methoxy-2,3-dihydrobenzofuran-6-sulfonyl chloride 19c

Compound **19b** (390 mg, 1.70 mmol) was dissolved in diethyl ether (10 mL), and n-butyllithium (0.69 mL, 1.72 mmol, 2.5 M in tetrahydrofuran) was added dropwise at -78 °C. The mixture was stirred at -78 °C for 1 h. Sulfur dioxide gas was bubbled into the reaction mixture for 30 min, and N-chlorosuccinimide (342 mg, 2.56 mmol) was added. The mixture was stirred at room temperature for 2 h. A saturated ammonium chloride solution (5 mL) was added, and the mixture was extracted with ethyl acetate (20 mL × 3). The organic phases were combined, washed with a saturated sodium chloride solution (20 mL × 2), dried over anhydrous sodium sulfate, and filtered. The filtrate was concentrated under reduced pressure, and the resulting residue was purified by silica gel column chromatography with eluent system A to give the title product **19c** (200 mg, yield: 47.2%).

### Step 3

### N-(4-((1H-pyrazol-1-yl)methyl)-2,3-dihydrobenzofuro[7,6-d]isoxazol-8-yl)-5-methoxy-2,3-dihydrobenzofuran-6-sulfonamide 19

Compound **19c** (194 mg, 0.78 mmol) and compound **1n** (100 mg, 0.39 mmol), 3,5-dimethylpyridine (168 mg, 1.57 mmol) and dimethyl sulfoxide (2 mg, 0.03 mmol) were dissolved in acetonitrile (10 mL), and the reaction mixture was stirred overnight at room temperature. The reaction mixture was concentrated under reduced pressure, and the resulting residue was purified by high performance liquid chromatography (Xtimate phenyl-hexyl Prep C18 5 µm, 30 × 150 mm; mobile phases: A-aqueous phase (0.1% ammonia water): B-acetonitrile = 5%-45% (20 min), flow rate: 30 mL/min) to give the title product **19** (50 mg, yield: 27.3%).

MS m/z (ESI): 469.1 [M+1].

¹H NMR (400 MHz, DMSO-*d₆*): δ 7.80 (s, 1H), 7.49-7.48 (m, 1H), 7.09-7.06 (m, 3H), 6.29-6.28 (m, 1H), 5.36 (s, 2H), 4.70-4.67 (m, 2H), 4.52-4.48 (m, 2H), 3.68 (s, 3H), 3.08-3.07 (m, 2H), 3.06-3.05 (m, 2H).

### Example 20

### N-(4-((1H-pyrazol-1-yl)methyl)-2,3-dihydrobenzofuro[7,6-d]isoxazol-8-yl)-2-methoxyb enzenesulfonamide 20

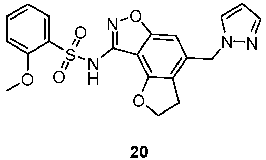

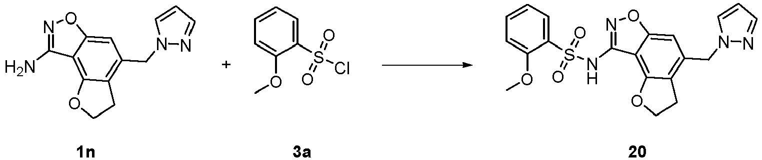

Compound **1n** (100 mg, 0.39 mmol) and compound **3a** (120 mg, 0.58 mmol) were dissolved in pyridine (5.0 mL), and the solution was purged with nitrogen 3 times. The reaction mixture was microwaved at 120 °C for 3 h. The reaction mixture was cooled to room temperature and concentrated under reduced pressure. The resulting residue was purified by high performance liquid chromatography (Sharpsil-T Prep C18 5 µm 30 × 150 mm; mobile phases: A-aqueous phase (10 mM ammonium bicarbonate): B-acetonitrile = 20%-45% (15 min), flow rate: 30 mL/min) to give the title product **20** (19 mg, yield: 11.4%).

MS m/z (ESI): 427.1 [M+1].

¹H NMR (500 MHz, CD₃OD) δ7.91 (dd, 1H), 7.72 (d, 1H), 7.62-7.57 (m, 1H), 7.56 (d, 1H), 7.14 (d, 1H), 7.07 (t, 1H), 6.59 (s, 1H), 6.38 (t, 1H), 5.42 (s, 2H), 4.78 (t, 2H), 3.87 (s, 3H), 3.09 (t, 2H).

### Example 21

### N-(4-((1H-pyrazol-1-yl)methyl)-2,3-dihydrobenzofuro[7,6-d]isoxazol-8-yl)-4-methyl-2-(2,2,2-trifluoroethoxy)benzenesulfonamide 21

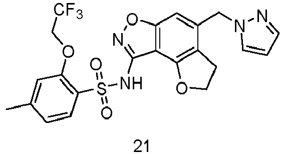

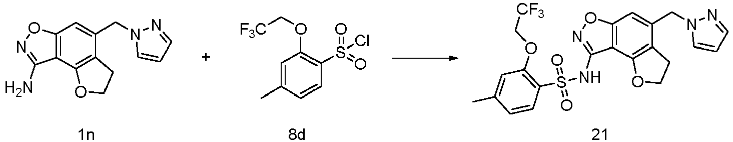

Compound **8d** (113 mg, 0.39 mmol) and compound **1n** (50 mg, 0.20 mmol) were dissolved in pyridine (5.0 mL), and 4-dimethylaminopyridine (5 mg, 0.04 mmol) was added. The mixture was purged with nitrogen 3 times. The reaction mixture was microwaved at 120 °C for 3 h. The reaction mixture was cooled to room temperature and concentrated under reduced pressure. The resulting residue was purified by high performance liquid chromatography (Xtimate phenyl-hexyl Prep C18 5 µm, 30 × 150 mm; mobile phases: A-aqueous phase (0.1% ammonia water): B-acetonitrile = 5%-45% (20 min), flow rate: 30 mL/min) to give the title product **21** (35 mg, yield: 35.3%). MS m/z (ESI): 509.3 [M+1].

¹H NMR (400 MHz, CDCl₃): δ 8.07-8.05 (d, 1H), 7.56-7.55 (d, 1H), 7.41-7.40 (d, 1H), 7.06-7.04 (d, 1H), 6.78 (s, 1H), 6.64 (s, 1H), 6.33-6.32 (m, 1H), 5.33 (s, 2H), 4.82-4.78 (m, 2H), 4.52-4.48 (m, 2H), 3.08-3.04 (m, 2H), 2.42 (s, 3H).

### Example 22

### N-(4-((1H-pyrazol-1-yl)methyl)-2,3-dihydrobenzofuro[7,6-d]isoxazol-8-yl)-2-methoxy-4-methylbenzenesulfonamide 22

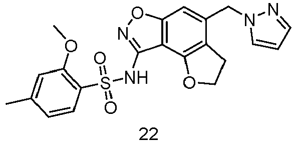

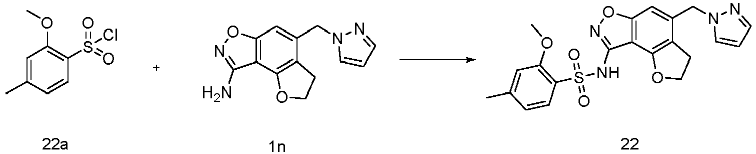

2-Methoxy-4-methylbenzenesulfonyl chloride **22a** (78 mg, 35 mmol) and compound **1n** (30 mg, 0.12 mmol) were dissolved in pyridine (5.0 mL), and 4-dimethylaminopyridine (3 mg, 0.02 mmol) was added. The mixture was purged with nitrogen 3 times. The reaction mixture was microwaved at 120 °C for 3 h. The reaction mixture was cooled to room temperature and concentrated under reduced pressure. The resulting residue was purified by high performance liquid chromatography (Xtimate phenyl-hexyl Prep C18 5 µm, 30 × 150 mm; mobile phases: A-aqueous phase (0.1% ammonia water): B-acetonitrile = 5%-45% (20 min), flow rate: 30 mL/min) to give the title product **22** (5 mg, yield: 9.7%).

MS m/z (ESI): 441.5 [M+1].

¹H NMR (400 MHz, DMSO-*d₆*): δ 7.82 (s, 1H), 7.62-7.60 (m,1H), 7.50-7.49 (m, 1H), 6.99-6.97 (m, 1H), 6.84-6.82 (m, 1H), 6.65-6.63 (m, 1H), 6.30 (s, 1H), 5.40 (s, 2H), 4.73-4.69 (m, 2H), 3.76 (s, 3H), 3.11-3.08 (m, 2H), 2.35 (s, 3H).

### Example 23

### N-(4-((1H-pyrazol-1-yl)methyl)-2,3-dihydrobenzofuro[7,6-d]isoxazol-8-yl)-2-methoxy-6-(trifluoromethoxy)benzenesulfonamide 23

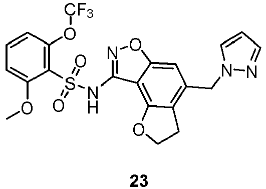

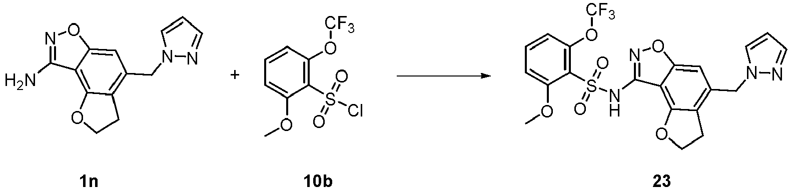

Compound **1n** (80 mg, 0.31 mmol) and compound **10b** (185 mg, 0.63 mmol) were dissolved in acetonitrile (3 mL), and dimethyl sulfoxide (3 mg, 0.04 mmol) and 3,5-dimethylpyridine (135 mg, 1.26 mmol) were added. The reaction mixture was left to react at room temperature for 16 h. The reaction mixture was filtered, and the filtrate was concentrated under reduced pressure. The resulting residue was purified by high performance liquid chromatography (Sharpsil-T Prep C18 5 µm 30 × 150 mm; mobile phases: A-aqueous phase (10 mM ammonium bicarbonate): B-acetonitrile = 20%-40% (10 min), flow rate: 30 mL/min) to give the title product **23** (16 mg, yield: 10.0%). MS m/z (ESI): 511.0 [M+1].

¹H NMR (500 MHz, DMSO-*d*₆) δ 7.79 (s, 1H), 7.48 (s, 1H), 7.38 (s, 1H), 7.10 (s, 2H), 6.86 (s, 1H), 6.37 (s, 1H), 6.29 (m, 1H), 5.34 (s, 2H), 4.65 (t, 2H), 3.68 (s, 3H), 3.05 (t, 2H).

### Example 24

### 2,6-dimethoxy-N-(4-(pyridin-2-ylmethyl)-2,3-dihydrobenzofuro[7,6-d]isoxazol-8-yl)be nzenesulfonamide 24

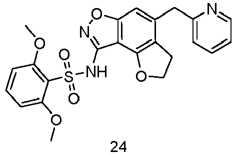

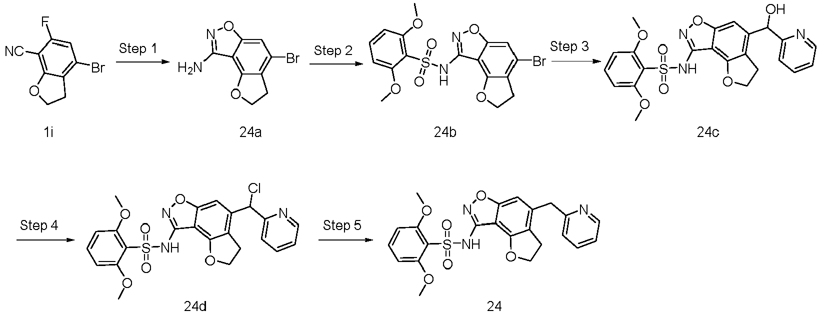

### Step 1

### 4-bromo-2,3-dihydrobenzofuro[7,6-d]isoxazol-8-amine 24a

Compound **1i** (2.3 g, 9.59 mmol) and acetohydroxamic acid (2.2 g, 28.85 mmol) were dissolved in a mixture of *N,N*-dimethylformamide (25 mL) and water (2.5 mL), and potassium carbonate (7.9 g, 57.54 mmol) was added. The reaction mixture was stirred at 65 °C for 16 h. The reaction mixture was filtered to give the title product **24a** (1.7 g, yield: 67.5%).

MS m/z (ESI): 255.0 [M+1].

### Step 2

### N-(4-bromo-2,3-dihydrobenzofuro[7,6-d]isoxazol-8-yl)-2,6-dimethoxybenzenesulfonam ide 24b

Compound **24a** (300 mg, 1.18 mmol) and compound **1o** (835 mg, 3.53 mmol) were dissolved in pyridine (8 mL), and 4-dimethylaminopyridine (29 mg, 0.24 mmol) was added. The mixture was purged with nitrogen 3 times. The reaction mixture was microwaved at 120 °C for 2 h. The reaction mixture was cooled to room temperature and concentrated under reduced pressure. The resulting residue was purified by high performance liquid chromatography (Welch Xtimate C18 5 µm 30 × 150 mm; mobile phases: A-aqueous phase (0.1% formic acid): B-acetonitrile = 30%-45% (15 min), flow rate: 30 mL/min) to give the title product **24b** (130 mg, yield: 24.3%).

MS m/z (ESI): 455.1 [M+1].

### Step 3

### (±)-N-(4-(hydroxy(pyridin-2-yl)methyl)-2,3-dihydrobenzofuro[7,6-d]isoxazol-8-yl)-2,6-dimethoxybenzenesulfonamide 24c

Compound **24b** (30 mg, 0.065 mmol) was dissolved in tetrahydrofuran (5 mL), and n-butyllithium (0.06 mL, 0.16 mmol, 2.5 M in tetrahydrofuran) was added dropwise at -78 °C. The mixture was stirred at -78 °C for 1 h. Pyridine-2-carbaldehyde (9 mg, 0.084 mmol) was added to the reaction mixture, and the reaction mixture was stirred overnight at room temperature. A saturated ammonium chloride solution (5 mL) was added to the reaction mixture, and the mixture was extracted with ethyl acetate (20 mL × 3). The organic phases were combined, washed with a saturated sodium chloride solution (20 mL × 2), dried over anhydrous sodium sulfate, and filtered. The filtrate was concentrated under reduced pressure, and the resulting residue was purified by silica gel column chromatography with eluent system A to give the title product **24c** (5 mg, yield: 15.7%).

MS m/z (ESI): 484.3 [M+1].

### Step 4

### (±)-N-(4-(chloro(pyridin-2-yl)methyl)-2,3-dihydrobenzofuro[7,6-d]isoxazol-8-yl)-2,6-di methoxybenzenesulfonamide 24d

Compound **24c** (5 mg, 0.01 mmol) was added to dichloromethane (5 mL), and thionyl chloride (13 mg, 0.1 mmol) was slowly added dropwise. The mixture was left to react at room temperature for 3 h. The reaction mixture was concentrated under reduced pressure and dissolved in ethyl acetate (20 mL). The solution was washed with a saturated sodium bicarbonate solution (20 mL), dried over anhydrous sodium sulfate, and filtered. The filtrate was concentrated under reduced pressure to give the title product **24d** (5 mg, yield: 96.3%).

MS m/z (ESI): 502.3 [M+1].

### Step 5

### 2,6-dimethoxy-N-(4-(pyridin-2-ylmethyl)-2,3-dihydrobenzofuro[7,6-d]isoxazol-8-yl)be nzenesulfonamide 24

Compound **24d** (5 mg, 0.01 mmol) was added to acetic acid (1 mL), and zinc powder (1 mg, 0.015 mmol) was added. The mixture was left to react at 60 °C for 3 h. The reaction mixture was concentrated under reduced pressure, and the resulting residue was purified by high performance liquid chromatography (Xtimate phenyl-hexyl Prep C18 5 µm, 30 × 150 mm; mobile phases: A-aqueous phase (0.1% ammonia water): B-acetonitrile = 5%-45% (20 min), flow rate: 30 mL/min) to give the title product **24** (2.5 mg, yield: 53.7%). MS m/z (ESI): 468.4 [M+1].

¹H NMR (400 MHz, DMSO-*d₆*): δ 8.49-8.48 (m, 1H), 7.80-7.77 (m,2H), 7.42 (s, 1H), 7.32-7.28 (m, 2H), 6.72-6.71 (m, 2H), 4.77-4.76 (m, 2H), 4.60 (s, 3H), 4.20 (s, 2H), 3.78 (s, 3H), 3.13-3.10 (m, 2H).

### Example 25

### N-(4-(furan-2-yl)-2,3-dihydrobenzofuro[7,6-d]isoxazol-8-yl)-2-methoxybenzenesulfona mide 25

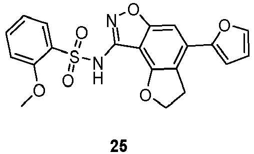

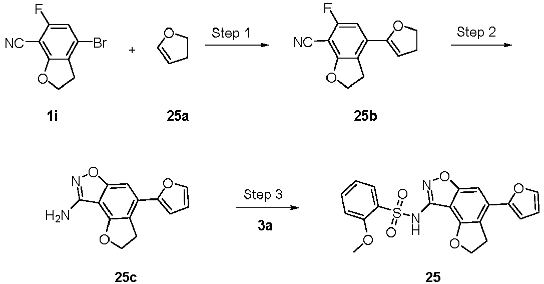

### Step 1

### 4-(4,5-dihydrofuran-2-yl)-6-fluoro-2,3-dihydrobenzofuran-7-carbonitrile 25b

Compound **1i** (300 mg, 1.24 mmol) and 2,3-dihydrofuran **25a** (435 mg, 6.20 mmol) were dissolved in *N,N*-dimethylformamide (10 mL), and the solution was purged with nitrogen 3 times. Palladium acetate (28 mg, 0.12 mmol), triphenylphosphine (65 mg, 0.24 mmol) and potassium carbonate (345 mg, 2.49 mmol) were added, and the reaction mixture was left to react at 110 °C for 16 h. The reaction mixture was cooled to room temperature and filtered, and the filtrate was concentrated under reduced pressure. The resulting residue was purified by silica gel column chromatography with eluent system A to give the title product **25b** (100 mg, yield: 34.9%).

MS m/z (ESI): 232.0 [M+1].

### Step 2

### 4-(furan-2-yl)-2,3-dihydrobenzofuro[7,6-d]isoxazol-8-amine 25c

Compound **25b** (100 mg, 0.43 mmol) and acetohydroxamic acid (100 mg, 1.33 mmol) were dissolved in 4 mL of a mixed solvent of *N*,*N*-dimethylformamide and water (V:V = 7:1), and potassium carbonate (360 mg, 2.60 mmol) was added. The reaction mixture was stirred at 70 °C for 30 min. The reaction mixture was cooled to room temperature and filtered, and the filtrate was concentrated under reduced pressure. The resulting residue was purified by silica gel column chromatography with eluent system A to give the title product **25c** (22 mg, yield: 21.0%).

MS m/z (ESI): 243.0 [M+1].

¹H NMR (500 MHz, CDCl₃) δ 7.56 (dd, 1H), 7.27 (s, 1H), 6.69 (dd, 1H), 6.56 (dd, 1H), 4.86 (t, 2H), 4.52 (s, 2H), 3.49 (t, 2H).

### Step 3

### N-(4-(furan-2-yl)-2,3-dihydrobenzofuro[7,6-d]isoxazol-8-yl)-2-methoxybenzenesulfona mide 25

Compound **25c** (22 mg, 0.09 mmol) and compound **3a** (95 mg, 0.46 mmol) were dissolved in pyridine (1.0 mL), and the solution was purged with nitrogen 3 times. The reaction mixture was microwaved at 120 °C for 3 h. The reaction mixture was cooled to room temperature and concentrated under reduced pressure. The resulting residue was purified by high performance liquid chromatography (welch Prep C18 5 µm 30 × 150 mm; mobile phases: A-aqueous phase (10 mM ammonium bicarbonate): B-acetonitrile = 25%-45% (20 min), flow rate: 30 mL/min) to give the title product 25 (5 mg, yield: 13.3%).

MS m/z (ESI): 413.0 [M+1].

¹H NMR (500 MHz, DMSO-*d*₆) δ 10.85 (s, 1H), 7.88 (s, 1H), 7.78 (dd, 1H), 7.61 (s, 1H), 7.37 (s, 1H), 7.19 (s, 1H), 7.06 (s, 1H), 7.01 (s, 1H), 6.70 (dd, 1H), 4.79 (t, 2H), 3.79 (s, 3H), 3.44 (t, 2H).

### Example 26

### 2,6-dimethoxy-N-(4-(tetrahydrofuran-2-yl)-2,3-dihydrobenzofuro[7,6-d]isoxazol-8-yl)b enzenesulfonamide 26

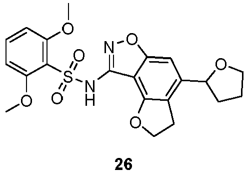

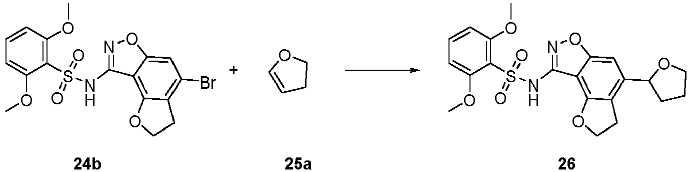

Compound **24b** (500 mg, 1.09 mmol) and compound **25a** (430 mg, 6.13 mmol) were dissolved in *N*,*N*-dimethylformamide (10 mL), and the solution was purged with nitrogen 3 times. Palladium acetate (30 mg, 0.13 mmol), triphenylphosphine (60 mg, 0.22 mmol) and potassium carbonate (320 mg, 2.31 mmol) were added, and the reaction mixture was left to react at 110 °C for 16 h. The reaction mixture was cooled to room temperature, purged with hydrogen 3 times, and left to react at room temperature for another 6 h. The reaction mixture was filtered, and the filtrate was concentrated under reduced pressure. The resulting residue was purified by high performance liquid chromatography (Sharpsil-T Prep C18 8 µm 50 × 250 mm; mobile phases: A-aqueous phase (10 mM ammonium bicarbonate): B-acetonitrile = 18%-38% (20 min), flow rate: 80 mL/min) to give the title product **26** (203 mg, yield: 41.4%).

MS m/z (ESI): 447.0 [M+1].

¹H NMR (500 MHz, CDCl₃) δ 7.40 (t, 1H), 6.99 (s, 1H), 6.61 (d, 2H), 4.94 (t, 1H), 4.90-4.78 (m, 2H), 4.12 (dt, 1H), 3.96 (dt, 1H), 3.93 (s, 6H), 3.25 (t, 2H), 2.36 (dq, 1H), 2.09-1.99 (m, 2H), 1.76 (dq, 1H).

### Example 26-1 and Example 26-2

### (R)-2,6-dimethoxy-N-(4-(tetrahydrofuran-2-yl)-2,3-dihydrobenzofuro[7,6-d]isoxazol-8-yl)benzenesulfonamide 26-1

### (S)-2,6-dimethoxy-N-(4-(tetrahydrofuran-2-yl)-2,3-dihydrobenzofuro[7,6-d]isoxazol-8-yl)benzenesulfonamide 26-2

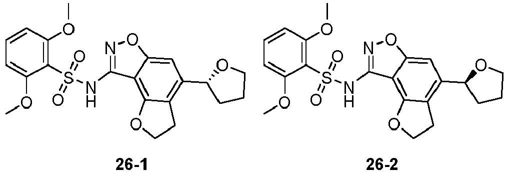

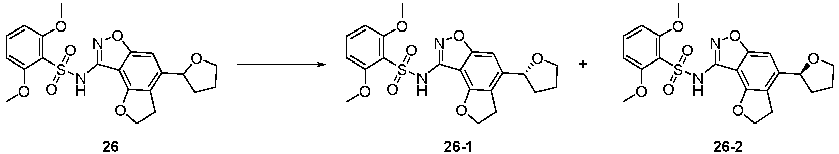

Compound **26** (203 mg, 0.45 mmol) was resolved by preparative chiral chromatography (resolution conditions: CHIRALPAK IE preparative chiral column, 20 mm × 250 mm; mobile phase: n-hexane/ethanol/trifluoroacetic acid = 60/40/0.1 (V/V/V), flow rate: 20 mL/min), and the corresponding fractions were collected and concentrated under reduced pressure to give the title products **26-1** (60 mg) and **26-2** (56 mg).

**26-1** (longer retention time, 60 mg):
MS m/z (ESI): 447.0 [M+1].

Chiral HPLC analysis: retention time 36.6 min, chiral purity: 100% (column: CHIRALPAK IE, 20 mm × 250 mm, 5 µm; mobile phase: n-hexane/ethanol/trifluoroacetic acid = 60/40/0.1 (V/V/V).

¹H NMR (500 MHz, CDCl₃) δ 7.40 (t, 1H), 6.99 (s, 1H), 6.61 (d, 2H), 4.94 (t, 1H), 4.90-4.78 (m, 2H), 4.12 (dt, 1H), 3.96 (dt, 1H), 3.93 (s, 6H), 3.25 (t, 2H), 2.36 (dq, 1H), 2.09-1.99 (m, 2H), 1.76 (dq, 1H).

**26-2** (shorter retention time, 56 mg):
MS m/z (ESI): 447.0 [M+1].

Chiral HPLC analysis: retention time 26.3 min, chiral purity: 100% (column: CHIRALPAK IE, 20 mm × 250 mm, 5 µm; mobile phase: n-hexane/ethanol/trifluoroacetic acid = 60/40/0.1 (V/V/V).

¹H NMR (500 MHz, CDCl₃) δ 7.40 (t, 1H), 6.99 (s, 1H), 6.61 (d, 2H), 4.94 (t, 1H), 4.90-4.78 (m, 2H), 4.12 (dt, 1H), 3.96 (dt, 1H), 3.93 (s, 6H), 3.25 (t, 2H), 2.36 (dq, 1H), 2.09-1.99 (m, 2H), 1.76 (dq, 1H).

### Biological Evaluation

The present disclosure is further described and explained below with reference to test examples. However, these test examples are not intended to limit the scope of the present disclosure.

### Test Example 1: KAT6 Enzyme Activity Assay (AlphaScreen Method)

### I. Reagents and instrument

1. KAT6A (customized by Chempartner)
2. Ovalbumin (Sigma-Aldrich, A5378-5G)
3. 2 M Tris-HCl solution, pH 7.8, sterile (Sangon, B548140-0500)
4. 5 M NaCl solution (Sangon, B548121-0100)
5. EDTA (0.5 M), pH 8.0, RNase-free (Thermofisher, AM9260G)
6. Tween-20 (Sangon, A100777-0500)
7. DTT, 1 M (Invitrogen, P2325)
8. Acetyl coenzyme A (Ac-CoA, CAYMAN, Cat. No. 16160)
9. Recombinant histone H3.1 biotinylated (human) (Active Motif 31696)
10. 384-well plate, light gray (Perkin Elmer, Cat. No. 6005350)
11. Anacardic acid (MCE, Cat. No. HY-N2020)
12. AlphaScreen streptavidin donor beads, 5 mg (PerkinElmer, 6760002)
13. AlphaScreen protein A acceptor beads, 5 mg (PerkinElmer, 6760137M)
14. Acetylated-lysine antibody #9441 (CST 9441S)
15. PHERA star microplate reader (BMG labtech)

### II. Experimental method

1. Reagent preparation
   a. 1× assay buffer: 100 mM Tris-HCl, pH 7.8; 15 mM NaCl; 1 mM EDTA; 0.01% tween-20; 1 mM DTT; 0.01% m/v ovalbumin.
   b. KAT enzyme solution: 1.25 nM (final concentration), prepared in 1× assay buffer.
   c. Mixed substrate of Ac-CoA and H3: a mixed substrate of 1000 nM (final concentration) Ac-CoA and 55 nM (final concentration) H3, prepared in 1× assay buffer.
   d. Compounds: initial concentration of 100 µM, 3-fold dilution, 10 gradient concentrations. All compound concentrations were diluted 83-fold with 1× assay buffer for later use.
   e. Assay reagent: 8 ng/µL (final concentration) AlphaScreen protein A acceptor beads, 8 ng/µL AlphaScreen streptavidin donor beads, 1:1500 diluted acetylated-lysine antibody, and 100 µM anacardic acid; prepared in 1× assay buffer.
2. Experimental procedure
   a. The prepared enzyme solution was added to a 384-well plate at 3 µL/well, and 3 µL of 1× assay buffer was added to each well in columns 23 and 24 (Min).
   b. 3 µL of compound solution was added to each well, and 3 µL of buffer was added to each Min well; 3 µL of DMSO solution was added to each well in columns 1 and 2 (Max) as controls. The plate was centrifuged, shaken for 2 min, and incubated at room temperature for 15 min.
   c. The mixed substrate of Ac-CoA and H3 was added at 6 µL/well, and the plate was centrifuged, shaken for 2 min, and incubated at room temperature for 20 min.
   d. The assay reagent was added at 6 µL/well, and the plate was centrifuged, shaken for 2 min, and incubated in the dark at room temperature for 120 min.
   e. Plate reading was performed on the microplate reader, and AlphaScreen counts were recorded.
   f. Plotting was performed using the Graphpad software, and the IC₅₀ values of the compounds were calculated.

**Table 1. The IC₅₀ values for the inhibition of the enzyme human KAT6A by the compounds of the present disclosure**

| Compound No. | KAT6A/IC₅₀ (nM) |
|---|---|
| 1 | 0.6 |
| 2 | 0.3 |
| 3 | 0.5 |
| 4 | 4.2 |
| 5 | 1.0 |
| 6 | 0.4 |
| 7 | 0.5 |
| 8 | 0.7 |
| 9 | 0.7 |
| 10 | 0.9 |
| 11 | 1.6 |
| 12 | 12.5 |
| 13 | 0.3 |
| 15 | 1.2 |
| 16 | 1.9 |
| 17 | 10.5 |
| 18 | 12.5 |
| 19 | 2.7 |
| 20 | 2.9 |
| 21 | 4.6 |
| 22 | 4.7 |
| 23 | 4.8 |
| 26 | 12.2 |
| 26-1 | 5.2 |

| | |
|---|---|
| Conclusion: The compounds of the present disclosure have good inhibitory effects on KAT6A. | |

### Test Example 2: KAT6B Enzyme Activity Assay (AlphaScreen Method)

### I. Reagents and instrument

1. KAT6B (718-1008) (ActiveMotif, 81224)
2. Bovine serum albumin (Sangon, A500023-0100)
3. 2 M Tris-HCl solution, pH 7.8, sterile (Sangon, B548140-0500)
4. EDTA (0.5 M), pH 8.0, RNase-free (Thermofisher, AM9260G)
5. Tween-20 (Sangon, A100777-0500)
6. DTT, 1 M (Invitrogen, P2325)
7. Acetyl coenzyme A (Ac-CoA, CAYMAN, Cat. No. 16160)
8. Recombinant histone H3.1 biotinylated (human) (Active Motif 31696)
9. 384-well plate, light gray (Perkin Elmer, Cat. No. 6007290)
10. Anacardic acid (MCE, Cat. No. HY-N2020)
11. AlphaScreen streptavidin donor beads, 5 mg (PerkinElmer, 6760002)
12. AlphaScreen protein A acceptor beads, 5 mg (PerkinElmer, 6760137M)
13. Acetylated-lysine antibody #9441 (CST 9441S)
14. DMSO (Tansoole, G7592B)
15. PHERA star microplate reader (BMG labtech)

### II. Experimental method

1. Reagent preparation
   a. 1× buffer 2: 50 mM Tris-HCl, pH 7.8; 0.1 mM EDTA; 0.01% v/v tween-20; 1 mM DTT; 0.01% m/v bovine serum albumin.
   b. KAT6B enzyme solution: 3 nM (final concentration), prepared in buffer 2.
   c. Mixed substrate of Ac-CoA and H3: a mixed substrate of 30 nM (final concentration) Ac-CoA and 30 nM (final concentration) H3, prepared in buffer 2.
   d. Compounds: initial concentration of 10 mM, 4-fold dilution, 10 gradient concentrations. All compound concentrations were diluted 2500-fold with buffer 2 for later use.
   e. Assay reagent: 8 ng/µL (final concentration) AlphaScreen protein A acceptor beads, 8 ng/µL AlphaScreen streptavidin donor beads, 1:1000 diluted acetylated-lysine antibody, and 100 µM anacardic acid; prepared in buffer 2.
2. Experimental procedure
   a. The prepared compound solutions were added to a 384-well plate at 2 µL/well, and 2 µL of buffer 2 (containing 0.04% DMSO) was added to each Min well and each Max well as controls. The plate was centrifuged.
   b. The prepared enzyme solution was added at 2 µL/well, and 2 µL of buffer 2 was added to each Min well. The plate was centrifuged, shaken for 2 min, and incubated at room temperature for 10 min.
   c. The mixed substrate of Ac-CoA and H3 was added at 4 µL/well, and the plate was centrifuged, shaken for 2 min, and incubated at room temperature for 120 min.
   d. The assay reagent was added at 4 µL/well, and the plate was centrifuged, shaken for 2 min, and incubated in the dark at room temperature for 120 min.
   e. Plate reading was performed on the microplate reader, and AlphaScreen counts were recorded.
   f. Dose-response curves were plotted using log(inhibitor) vs. response of the graphpad prism software, with the abscissa representing the logarithm of the compound concentration and the ordinate representing the calculated enzyme activity inhibition rate, and IC₅₀ values were calculated.

**Table 2. The IC₅₀ values for the inhibition of the enzyme human KAT6B by the compounds of the present disclosure**

| Compound No. | KAT6B/IC₅₀ (nM) |
|---|---|
| 1 | 1.1 |
| 2 | 0.7 |
| 3 | 1.4 |
| 5 | 1.5 |

| | |
|---|---|
| Conclusion: The compounds of the present disclosure have good inhibitory effects on KAT6B. | |

### Test Example 3: U2OS Cell H3K23 Acetylation IF Assay (Immunofluorescence)

### I. Reagents and instruments

1. U-2 OS (ATCC HTB-96)
2. Recombinant anti-histone H3 (acetyl K23) antibody (Abcam, ab177275)
3. Goat anti-rabbit IgG (H+L), Superclonal^{™} recombinant secondary antibody, Alexa Fluor 488 (Thermofisher, A27034)
4. Hoechst 33342 (Sigma-Aldrich, B2261-25MG)
5. Assay plate, 96-well, black with clear bottom (Corning, 3603)
6. Bovine serum albumin (BSA) (Sangon Biotech, A500023-0100)
7. Methanol (GENMERAL-REAGENT, G75851D)
8. Tween-20 (Sangon, A100777-0500)
9. Triton X-100 (Solarbio, T8200)
10. PBS (Shanghai BasalMedia Technologies Co., Ltd., B320KJ)
11. 20× PBS buffer (Sangon Biotech, B548117-0500)
12. McCoy's 5A medium (Gibco, 16600082)
13. 0.25% trypsin-EDTA (1×) (Gibco, 25200-072)
14. Pen strep (Gibco, 15140-122)
15. DPBS (1×) (Gibco, 14190-144)
16. FBS (Gibco, 10091148)
17. Automatic cell counter (Countstar, IC1000)
18. Thermostatic incubator (Thermo, I160)
19. ImageXpress^{®} Micro Confocal (Molecular Device)

### II. Experimental method

1. Reagent preparation
   a. Blocking buffer: PBS (Shanghai BasalMedia) + BSA (final concentration of 1%) + Triton X-100 (final concentration of 0.5%).
   b. Wash buffer: PBS (20× PBS was diluted to 1× PBS) + Tween-20 (final concentration of 0.1%).
   c. Primary antibody solution: Recombinant anti-histone H3 (acetyl K23) antibody was diluted in blocking buffer at a ratio of 1:1000.
   d. Secondary antibody solutions: Goat anti-rabbit IgG (H+L), Superclonal^{™} recombinant secondary antibody, Alexa Fluor 488 was diluted in blocking buffer at a ratio of 1:1000, and Hoechst 33342 was diluted in blocking buffer at a ratio of 1:5000.
   e. Compounds: initial concentration of 100 µM, 3-fold dilution, 9 gradient concentrations. All compound concentrations were diluted 500-fold with McCoy's 5A medium for later use.
2. Experimental procedure
2.1. Cell treatment (day one)
   a. The state of U-2 OS cells was observed under a microscope to make sure that the cell confluence was about 90%.
   b. The cell supernatant was discarded. The cells were rinsed with DPBS once, and the DPBS was removed. The cells were digested with a proper amount of trypsin and left to stand at room temperature or 37 °C for 5 min.
   c. The digestion was stopped with an equal volume of medium containing 10% FBS, and the cell suspension was collected and centrifuged at 300 g for 3 min. The cells were suspended in a proper amount of fresh medium.
   d. The resulting cell suspension was collected and cell counting was performed.
   e. The cell suspension was diluted and plated at 9000 cells/50 µL/well.
   f. The wells on the periphery were blocked with 100 µL of PBS.
   g. The cell culture plates were incubated overnight in a 37 °C, 5% carbon dioxide incubator.
2.2. Compound addition (day two)
   a. 50 µL of diluted compound was added to the cell supernatant (50 µL/well) in each cell plate.
   b. After the addition, the cell plates were incubated in a 37 °C, 5% carbon dioxide incubator for 24 h.
2.3. Immunofluorescence staining and measurement (day three to day four)
   a. After the 24-hour incubation, the cell plates were removed from the incubator. The medium was discarded, and plates were fixed with pre-cooled methanol at room temperature for 10 min.
   b. The fixation buffer was discarded, and the plates were quickly washed with wash buffer 3 times and then slowly washed 3 times (5 min/wash).
   c. The wash buffer was discarded, and blocking buffer was added. The plates were incubated at room temperature for 60 min.
   d. The blocking buffer was discarded, and the prepared primary antibody solution was added. The plates were incubated overnight at 4 °C.
   e. The primary antibody solution was discarded, and the prepared secondary antibody solution was added. The plates were incubated at room temperature for 60 min.
   f. The secondary antibody solution was discarded, and the plates were quickly washed with wash buffer 3 times and then slowly washed 5 times (5 min/wash).
   g. The plates were tested on ImageXpress^{®} Micro Confocal.
   h. The mean fluorescence intensity data for cells were plotted using the Graphpad software, and the IC₅₀ values and Imax% of the compounds were calculated.

**Table 3. The IC₅₀ values and maximum inhibition rates for the inhibition of H3K23 acetylation by the compounds of the present disclosure**

| Compound No. | H3K23 **ac**/IC₅₀ (nM) | Maximum inhibition rate/Imax% |
|---|---|---|
| 1 | 1.1 | 101.8 |
| 2 | 0.4 | 98.5 |
| 3 | 0.9 | 106.3 |
| 4 | 1.5 | 105.5 |
| 5 | 0.6 | 104.2 |
| 7 | 1.4 | 97.4 |
| 8 | 2.8 | 99.4 |
| 9 | 0.75 | 99.5 |
| 10 | 1.2 | 106.3 |
| 11 | 2.1 | 103.1 |
| 20 | 4.6 | 99.4 |
| 22 | 2.9 | 104.8 |

| | | |
|---|---|---|
| Conclusion: The compounds of the present disclosure have good inhibitory effects on H3K23 acetylation. | | |

### Test Example 4: ZR-75-1 Proliferation Assay

### I. Reagents and instruments

1. ZR-75-1 (ATCC CRL1500)
2. 1640 medium (Gibco, 22400-089)
3. 0.25% trypsin-EDTA (1×) (Gibco, 25200-072)
4. Penicillin-streptomycin (Gibco, 15140-122)
5. DPBS (1×) (Gibco, 14190-144)
6. FBS (Gibco, 10091148)
7. Assay plate, 96-well, black with clear bottom (Corning, 3603)
8. 96-well non-treated round-bottom plate (JET BIOFIL, TCP-002-096)
9. CellTiter-Glo buffer (Promega, G756B)
10. CellTiter-Glo substrate (Promega, G755B)
11. Automatic cell counter (Countstar, IC1000)
12. Thermostatic incubator (Thermo, I160)
13. PHERAstar FS (BMG labtech, PHERAstar FS)

### II. Experimental method

### 1. Cell plating (day 0)

a. The state of cells was observed under a microscope to make sure that the cell confluence was about 90%.
b. The cell supernatant was discarded. The cells were rinsed with DPBS once, and the DPBS was removed. The cells were digested with a proper amount of trypsin and left to stand at 37 °C for 5 min.
c. The digestion was stopped with an equal volume of 1640 medium containing 10% FBS, and the cell suspension was collected and centrifuged at 300 g for 3 min. The cells were suspended in a proper amount of fresh medium.
d. The resulting cell suspension was collected and cell counting was performed.
e. The cell suspension was diluted to 5×10⁴/mL, 50 µL/well with 1640 medium containing 10% FBS. Each well contained 2500 ZR-75-1 cells.
f. The cell culture plates were incubated overnight in a 37 °C, 5% carbon dioxide incubator.

### 2. Compound addition (day 1)

a. Each compound was serially diluted in DMSO to 9 concentration points (initial concentration of 100 µM, 3-fold dilution; the highest concentration of each compound may be adjusted depending on its IC₅₀). For example, in a 96-well non-treated round-bottom plate, 3 µL of compound was serially diluted in 6 µL of DMSO.
b. Each concentration point of each compound was diluted 500-fold in the corresponding volume of 1640 medium.
c. 50 µL of diluted compound solution was added to the cell supernatant (50 µL/well) in each cell plate.
d. After the addition, the cell plates were incubated in a 37 °C, 5% carbon dioxide incubator.

### 3. The cells were re-digested and plated, and the compounds were added (day 7).

a. Six days after the addition, the compound-containing medium was discarded. Then the cells were rinsed with DPBS (150 µL/well), and the DPBS was immediately pipetted off.
b. The cells were digested with 50 µL of trypsin and left to stand at 37 °C for 3 min, and then the digestion was stopped with 1640 medium containing 10% FBS (150 µL/well).
c. The cells were well mixed using a pipette and re-plated at a ratio of 1:8; that is, 25 µL of cell suspension was pipetted into a new 96-well plate (25 µL of 1640 medium containing 10% FBS had been added to the new plate in advance).
d. Compound solution preparation and addition (50 µL/well) were performed according to steps a to c in **2.**
e. After the addition, the cell plates were incubated in a 37 °C, 5% carbon dioxide incubator.

### 4. CTG assay (day 14)

a. CellTiter-Glo buffer and lyophilized CellTiter-Glo substrate were equilibrated to room temperature before use and were well mixed to prepare a 100-mL CellTiter-Glo reagent (or a prepared CellTiter-Glo reagent was removed from a -20 °C freezer and equilibrated to room temperature).
b. The plates to be tested were removed from the incubator and equilibrated to room temperature, and the CellTiter-Glo reagent was added at 50 µL/well.
c. The plates were shaken for 2 min so that the cells were completely lysed.
d. After the plates were left to stand at room temperature for 28 min and signals were stable, the plates were tested on PHERAstar FS.

**Table 4. The IC₅₀ values and maximum inhibition rates for the inhibition of ZR-75-1 proliferation by the compounds of the present disclosure**

| Compound No. | ZR-75-1/IC₅₀ (nM) | Maximum inhibition rate/Imax% |
|---|---|---|
| 1 | 1.4 | 96 |
| 2 | 0.4 | 96 |
| 3 | 1.9 | 95 |
| 5 | 0.9 | 98 |
| 6 | 0.6 | 95.4 |
| 7 | 1.6 | 95.6 |
| 8 | 2.6 | 96.3 |
| 9 | 1.6 | 96.2 |
| 10 | 2.95 | 94.1 |
| 20 | 8.6 | 94.9 |
| 23 | 10.9 | 90.8 |

| | | |
|---|---|---|
| Conclusion: The compounds of the present disclosure have good inhibitory effects on ZR-75-1 proliferation. | | |

### Test Example 5: Pharmacokinetic Evaluation

### I. Testing in SD rats

### 1. Abstract

SD rats were used as test animals. After intragastric (i.g.) administration of the compounds of the present disclosure to SD rats, the plasma concentrations at different time points were measured by the LC/MS/MS method. The pharmacokinetic behavior of the compounds of the present disclosure in SD rats was studied, and their pharmacokinetic profiles were evaluated.

### 2. Method

### 2.1. Test compounds

Compound 2, compound 7, compound 21, and compound 23.

### 2.2. Test animals

16 SD rats, of which half were male and half female, were evenly divided into 4 groups. The rats were provided by Vital River Laboratory Animal Technology Co., Ltd. The rats were fasted overnight and intragastrically administered the compounds.

### 2.3. Compound solution preparation

A certain amount of test compound was weighed out and dissolved in 5% DMSO + 5% tween 80 + 90% normal saline to prepare a 0.2 mg/mL colorless clear solution.

### 2.4. Administration

The dose administered was 2.0 mg/kg, and the volume was 10.0 mL/kg.

### 3. Procedure

0.1 mL blood samples were collected from the orbit before the administration and 0.25 h, 0.5 h, 1.0 h, 2.0 h, 4.0 h, 6.0 h, 8.0 h, 11.0 h and 24.0 h post-dose. The blood samples were placed into EDTA-K2 anticoagulation tubes and centrifuged at 10,000 rpm for 1 min (4 °C), and plasma was separated within 1 h and stored at -20 °C before analysis. The process from the blood collection to the centrifugation was performed in an ice bath. Access to food was given 2 h post-dose.

Measurement of plasma concentrations in SD rats after compounds were administered in different concentrations: 25 µL samples of the SD rat plasma collected at various time points post-dose were taken, and to each of the samples was added 25 µL of camptothecin (an internal standard for compound 2, 100 ng/mL) or 50 µL of tolbutamide (an internal standard for compound 7, 100 ng/mL) or 50 µL of verapamil (an internal standard for compounds 21 and 23, 100 ng/mL). Protein was precipitated with 200 µL of acetonitrile, and after 5 min of vortexing, the mixtures were centrifuged at 3700 rpm for 10 min. 120 µL of supernatant was taken and vortexed with 30 µL of water for 5 min, and a 5 µL sample was taken for LC/MS/MS analysis.

### 4. Pharmacokinetic parameters

**Table 5. The pharmacokinetic parameters of the compounds of the present disclosure**

| No. | Route of administration/dose (mg/kg) | Plasma concentration Cmax (ng/mL) | Area under curve AUC₀₋ₜ (h*ng/mL) | Half-life T1/2 (h) | Clearance rate CL/F (mL/min/kg) | Apparent distribution volume Vz/F (mL/kg) |
|---|---|---|---|---|---|---|
| Compound 2 | i.g. /2.0 | 8210 | 43324 | 2.96 | 0.8 | 202 |
| Compound 7 | i.g. /2.0 | 9198 | 81329 | 6.71 | 0.614 | 250 |
| Compound 21 | i.g. /2.0 | 4306 | 43693 | 9.58 | 0.788 | 496 |
| Compound 23 | i.g. /2.0 | 6245 | 61158 | 13.7 | 0.431 | 470 |

| | | | | | | |
|---|---|---|---|---|---|---|
| Conclusion: The compounds of the present disclosure demonstrated high plasma concentrations, substantial exposure, and low clearance rates in the SD rats, indicating pharmacokinetic advantages. | | | | | | |

### II. Testing in C57 mice

### 1. Abstract

C57 mice were used as test animals. After intragastric (i.g.)/intravenous (i.v.) administration of the compounds of the present disclosure to C57 mice, the plasma concentrations at different time points were measured by the LC/MS/MS method. The pharmacokinetic behavior of the compounds of the present disclosure in C57 mice was studied and their pharmacokinetic profiles were evaluated.

### 2. Method

### 2.1. Test compounds

Compound 2 and compound 3.

### 2.2. Test animals

36 C57 mice, of which half were male and half female, were evenly divided into 4 groups of 9, 3 mice per time point per group. The mice were provided by Vital River Laboratory Animal Technology Co., Ltd. The mice were intragastrically and intravenously administered the compounds.

### 2.3. Compound solution preparation

A certain amount of test compound was weighed out and dissolved in 5% DMSO + 5% tween 80 + 90% normal saline to prepare a 0.1 mg/mL colorless clear solution (for the intragastric administration groups) and a 0.1 mg/mL colorless clear solution (for the intravenous administration groups).

### 2.4. Administration

Intragastric administration groups: The dose was 2.0 mg/kg, and the volume was 0.2 mL/10 g.

Intravenous administration groups: The dose was 1.0 mg/kg, and the volume was 0.1 mL/10 g.

### 3. Procedure

Intragastric administration groups: 0.1 mL blood samples were collected before the administration and 0.25 h, 0.5 h, 1.0 h, 2.0 h, 4.0 h, 6.0 h, 8.0 h, 11.0 h and 24.0 h post-dose. The blood samples were placed into EDTA-K2 anticoagulation tubes and centrifuged at 10,000 rpm for 1 min (4 °C), and plasma was separated within 1 h and stored at -80 °C before analysis. The process from the blood collection to the centrifugation was performed in an ice bath.

Intravenous administration groups: Blood samples were collected before administration and 5 min, 0.25 h, 0.5 h, 1.0 h, 2.0 h, 4.0 h, 8.0 h, 11.0 h and 24 h post-dose and were treated in the same way as those collected from the intragastric administration groups. Measurement of plasma concentrations in C57 mice after compounds were administered in different concentrations: 25 µL samples of the C57 mouse plasma collected at various time points post-dose were taken, and to each of the samples was added 50 µL of diclofenac (an internal standard for compound 2, 100 ng/mL) or 25 µL of tolbutamide (an internal standard for compound 3, 10 µg/mL, purchased from LGC Ltd., UK). Protein was precipitated with 200 µL of acetonitrile, and after 5 min of vortexing, the mixtures were centrifuged at 3700 rpm for 10 min. 120 µL of supernatant was taken and vortexed with 30 µL of water for 5 min, and a 5 µL sample was taken for LC/MS/MS analysis.

### 4. Pharmacokinetic parameters

**Table 6. The pharmacokinetic parameters of the compounds of the present disclosure**

| No. | Route of administration/dose (mg/kg) | Plasma concentration Cmax (ng/mL) | Area under curve AUC₀₋ₜ (h*ng/mL) | Half-1 ife T1/2 (h) | Clearance rate CL/F (mL/min/kg) | Apparent distribution volume Vss (mL/kg) | Bioavailability F (%) |
|---|---|---|---|---|---|---|---|
| Compound 2 | i.g./2.0 | 40100 | 404968 | 29.7 | 0.0330 | - | 108 |
| | i.v./1.0 | 17600 | 188231 | 18.4 | 0.0504 | 93 | |
| Compound 3 | i.g./2.0 | 32994 | 393064 | 58.3 | 0.0225 | - | 94.1 |
| | i.v./1.0 | 22143 | 208818 | 49.0 | 0.0251 | 102 | |

| | | | | | | | |
|---|---|---|---|---|---|---|---|
| Conclusion: The compounds of the present disclosure demonstrated high plasma concentrations, high exposure, low clearance rates, and relatively long half-lives in the C57 mice, indicating pharmacokinetic advantages. | | | | | | | |

## Claims

1. A compound represented by general formula (I) or a pharmaceutically acceptable salt thereof: 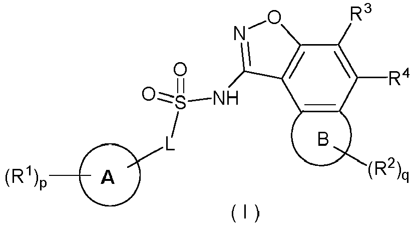
wherein: ring A is selected from the group consisting of cycloalkyl, heterocyclyl, aryl and heteroaryl;
ring B is cycloalkyl or heterocyclyl;
L is a chemical bond, alkylene or heteroalkylene, wherein the alkylene or heteroalkylene is independently optionally substituted with one or more substituents selected from the group consisting of hydroxy, halogen, alkyl, haloalkyl, hydroxyalkyl, alkoxy and haloalkoxy;
each R¹, each R², and R³ are identical or different and are each independently selected from the group consisting of a hydrogen atom, halogen, cyano, nitro, oxo, alkenyl, alkynyl, alkyl, alkoxy, cycloalkyl, heterocyclyl, aryl, heteroaryl, -OR⁵, -C(O)R⁶, -C(O)OR⁶, -OC(O)R⁶, -NHC(O)OR⁶, -NR⁷R⁸, -C(O)NR⁷R⁸, -S(O)ᵣR⁶ and -S(O)ᵣNR⁷R⁸, wherein the alkenyl, alkynyl, alkyl, alkoxy, cycloalkyl, heterocyclyl, aryl and heteroaryl are each independently optionally substituted with one or more substituents selected from the group consisting of hydroxy, halogen, cyano, amino, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy and heterocyclyloxy;
R⁴ is a hydrogen atom or 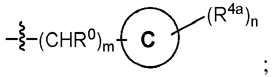
ring C is selected from the group consisting of cycloalkyl, heterocyclyl, aryl and heteroaryl;
R⁰ is selected from the group consisting of a hydrogen atom, hydroxy, halogen, alkyl, haloalkyl, hydroxyalkyl, alkoxy, haloalkoxy and cycloalkyl;
each R^{4a} is identical or different and is independently selected from the group consisting of a hydrogen atom, hydroxy, halogen, cyano, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy, heterocyclyloxy and -NR⁹R¹⁰;
R⁵ and R⁶ are identical or different and are each independently selected from the group consisting of a hydrogen atom, alkenyl, alkynyl, alkyl, cycloalkyl, heterocyclyl, aryl and heteroaryl, wherein the alkenyl, alkynyl, alkyl, cycloalkyl, heterocyclyl, aryl and heteroaryl are each independently optionally substituted with one or more substituents selected from the group consisting of hydroxy, halogen, cyano, amino, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy and heterocyclyloxy;
R⁷, R⁸, R⁹ and R¹⁰ are identical or different and are each independently selected from the group consisting of a hydrogen atom, alkyl, cycloalkyl, heterocyclyl, aryl and heteroaryl, wherein the alkyl, cycloalkyl, heterocyclyl, aryl and heteroaryl are each independently optionally substituted with one or more substituents selected from the group consisting of hydroxy, halogen, cyano, amino, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy and heterocyclyloxy;
or R⁷ and R⁸, together with the N atom to which they are attached, form one heterocyclyl group; or R⁹ and R¹⁰, together with the N atom to which they are attached, form one heterocyclyl group; the heterocyclyl group is optionally substituted with one or more substituents selected from the group consisting of hydroxy, halogen, cyano, amino, nitro, oxo, alkenyl, alkynyl, alkyl, haloalkyl, hydroxyalkyl, alkoxy, haloalkoxy, cycloalkyl, heterocyclyl, aryl, heteroaryl, cycloalkylalkyl, heterocyclylalkyl, cycloalkyloxy and heterocyclyloxy;
p is 0, 1, 2, 3 or 4;
q is 0, 1, 2, 3 or 4;
m is 0, 1, 2, 3 or 4;
n is 0, 1, 2, 3 or 4; and
r is 0, 1 or 2.

2. The compound represented by general formula (I) or the pharmaceutically acceptable salt thereof according to claim 1, wherein ring C is aryl or heteroaryl.

3. The compound represented by general formula (I) or the pharmaceutically acceptable salt thereof according to claim 1 or 2, wherein R³ is a hydrogen atom.

4. The compound represented by general formula (I) or the pharmaceutically acceptable salt thereof according to any of claims 1 to 3, wherein R⁴ is 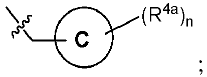 ring C is 5- to 10-membered heteroaryl, preferably 5- or 6-membered heteroaryl; R^{4a} and n are as defined in claim 1.

5. The compound represented by general formula (I) or the pharmaceutically acceptable salt thereof according to any of claims 1 to 4, being a compound represented by general formula (II) or a pharmaceutically acceptable salt thereof: 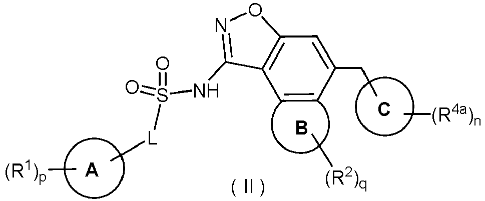 wherein:
ring C is 5- to 10-membered heteroaryl, preferably 5- or 6-membered heteroaryl;
ring A, ring B, L, R¹, R², R^{4a}, p, q and n are as defined in claim 1.

6. The compound represented by general formula (I) or the pharmaceutically acceptable salt thereof according to any of claims 1 to 5, wherein ring A is 6- to 10-membered aryl, and is preferably selected from the group consisting of phenyl, 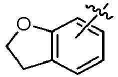 and

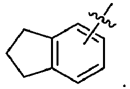

7. The compound represented by general formula (I) or the pharmaceutically acceptable salt thereof according to any of claims 1 to 6, wherein ring B is 4- to 7-membered heterocyclyl.

8. The compound represented by general formula (I) or the pharmaceutically acceptable salt thereof according to any of claims 1 to 7, being a compound represented by general formula (III) or a pharmaceutically acceptable salt thereof: 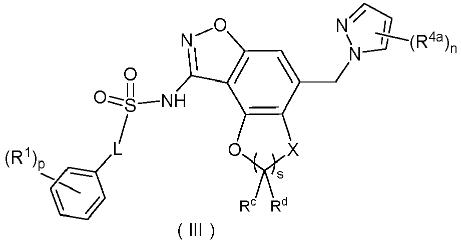 wherein:
X is selected from the group consisting of O, CR^{a}R^{b} and C=O;
each R^{a}, R^{b}, R^{c} and R^{d} is identical or different and is independently selected from the group consisting of a hydrogen atom, hydroxy, halogen, cyano, amino, alkenyl, alkynyl, alkyl, haloalkyl, hydroxyalkyl, alkoxy, haloalkoxy, cycloalkyl, heterocyclyl, aryl, heteroaryl, cycloalkyloxy and heterocyclyloxy;
or R^{c} and R^{d}, together with the carbon atom to which they are attached, form one C=O; s is 0, 1, 2 or 3;
L, R¹, R^{4a}, p and n are as defined in claim 1.

9. The compound represented by general formula (I) or the pharmaceutically acceptable salt thereof according to claim 8, wherein R^{c} and R^{d} are identical or different and are each independently hydrogen atoms or halogens; and/or X is O or CH₂; and/or s is 1 or 2.

10. The compound represented by general formula (I) or the pharmaceutically acceptable salt thereof according to any of claims 1 to 9, wherein each R¹ is identical or different and is independently selected from the group consisting of a hydrogen atom, hydroxy, halogen, C₁₋₆ alkyl, C₁₋₆ haloalkyl, C₁₋₆ hydroxyalkyl, C₁₋₆ alkoxy, C₁₋₆ haloalkoxy, C₁₋₆ alkoxy C₁₋₆ alkyl, and -C(O)OCH₃ and -NR⁷R⁸; R⁷ and R⁸ are identical or different and are each independently hydrogen atoms or C₁₋₆ alkyl; preferably, each R¹ is identical or different and is independently selected from the group consisting of a hydrogen atom, halogen, C₁₋₆ alkyl, C₁₋₆ haloalkyl, C₁₋₆ alkoxy, C₁₋₆ haloalkoxy and -NR⁷R⁸; R⁷ and R⁸ are identical or different and are each independently hydrogen atoms or C₁₋₆ alkyl; more preferably, each R¹ is identical or different and is independently C₁₋₆ alkoxy

11. The compound represented by general formula (I) or the pharmaceutically acceptable salt thereof according to any of claims 1 to 7 and 10, wherein each R² is identical or different and is independently selected from the group consisting of a hydrogen atom, halogen, C₁₋₆ alkyl and C₁₋₆ alkoxy, and is preferably a hydrogen atom or halogen.

12. The compound represented by general formula (I) or the pharmaceutically acceptable salt thereof according to any of claims 1 to 11, wherein each R^{4a} is identical or different and is independently selected from the group consisting of a hydrogen atom, hydroxy, halogen, C₁₋₆ alkyl, C₁₋₆ hydroxyalkyl and C₁₋₆ alkoxy, and is preferably a hydrogen atom.

13. The compound represented by general formula (I) or the pharmaceutically acceptable salt thereof according to any of claims 1 to 12, wherein L is a chemical bond or -CH₂-, preferably a chemical bond.

14. The compound represented by general formula (I) or the pharmaceutically acceptable salt thereof according to any of claims 1 to 13, being selected from the group consisting of the following compounds:

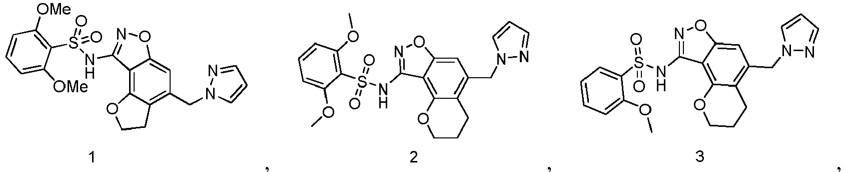

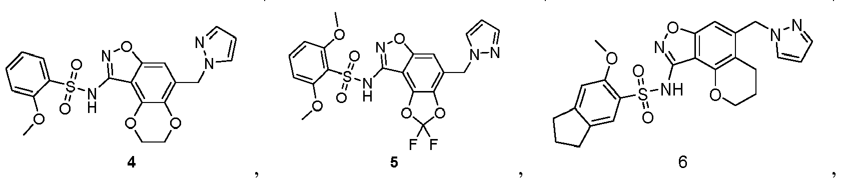

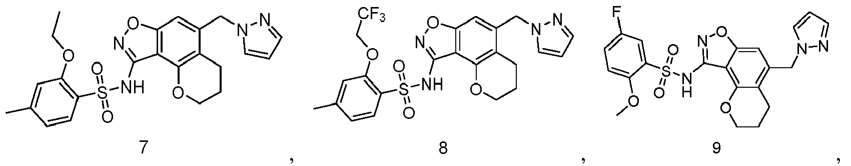

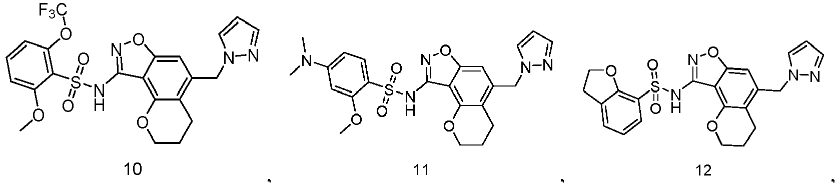

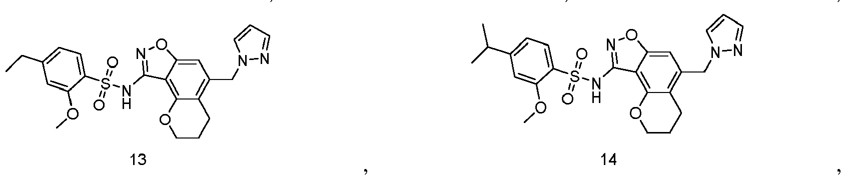

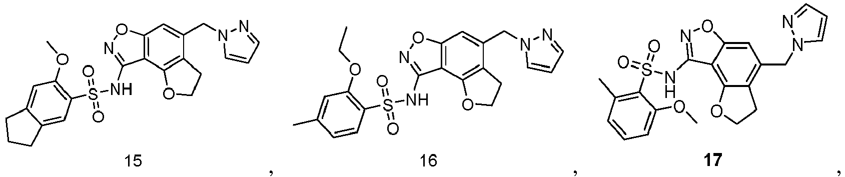

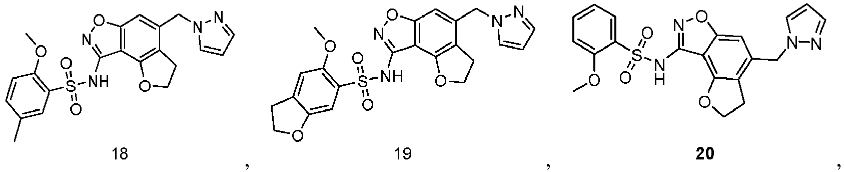

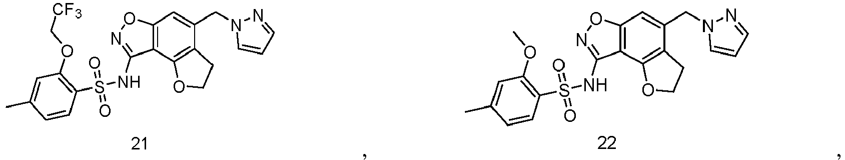

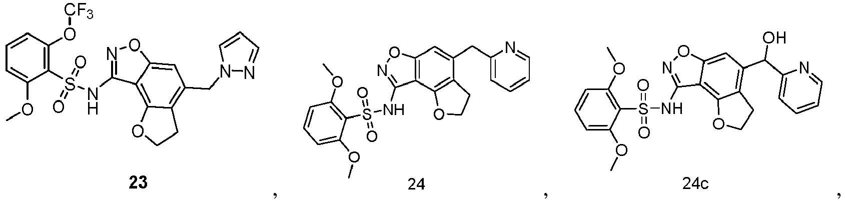

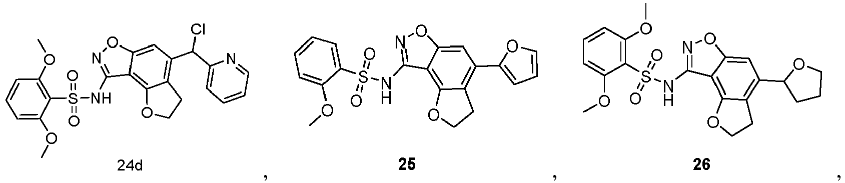

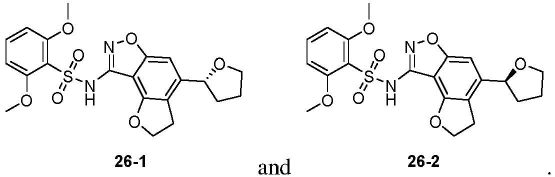

15. A compound represented by general formula (IA) or a salt thereof: 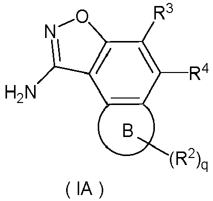 wherein:
ring B, R², R³, R⁴ and q are as defined in claim 1.

16. A compound or a salt thereof, selected from the group consisting of the following compounds or salts thereof:

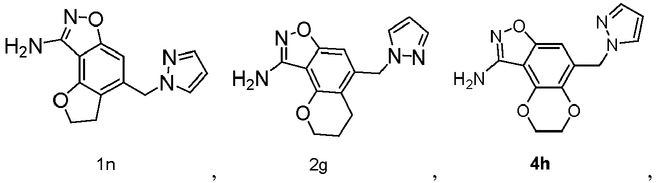

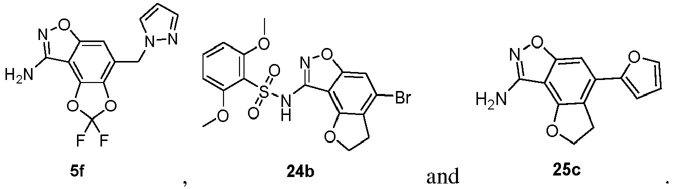

17. A method for preparing the compound represented by general formula (I) or the pharmaceutically acceptable salt thereof according to claim 1, wherein the method comprises: 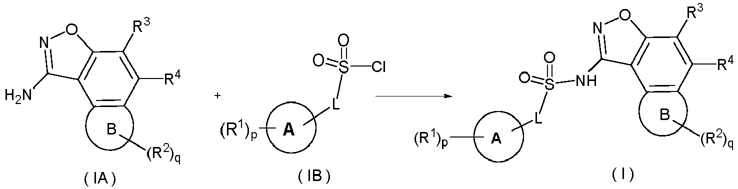 reacting a compound represented by general formula (IA) or a salt thereof with a compound represented by general formula (IB) or a salt thereof to give the compound represented by general formula (I) or the pharmaceutically acceptable salt thereof, wherein:
ring A, ring B, L, R¹ to R⁴, p and q are as defined in claim 1.

18. A pharmaceutical composition, wherein the pharmaceutical composition comprises the compound represented by general formula (I) or the pharmaceutically acceptable salt thereof according to any of claims 1 to 14, and one or more pharmaceutically acceptable carriers, diluents or excipients.

19. Use of the compound represented by general formula (I) or the pharmaceutically acceptable salt thereof according to any of claims 1 to 14 or the pharmaceutical composition according to claim 18 in the preparation of a medicament for inhibiting a KAT, wherein the KAT is preferably KAT6, and is more preferably KAT6A and/or KAT6B.

20. Use of the compound represented by general formula (I) or the pharmaceutically acceptable salt thereof according to any of claims 1 to 14 or the pharmaceutical composition according to claim 18 in the preparation of a medicament for treating and/or preventing a cancer, wherein the cancer is preferably selected from the group consisting of lung cancer, mesothelioma, bone cancer, pancreatic cancer, skin cancer, head and neck cancer, brain cancer, melanoma, anal cancer, liver cancer, breast cancer, fallopian tube cancer, endometrial cancer, cervical cancer, ovarian cancer, vaginal cancer, vulvar cancer, Hodgkin's lymphoma, esophageal cancer, colorectal cancer, small intestine cancer, stomach cancer, thyroid cancer, parathyroid cancer, adrenal cancer, soft tissue sarcoma, penile cancer, testicular cancer, prostate cancer, leukemia, B-cell lymphoma, bladder cancer, urethral cancer, ureter cancer, renal cell carcinoma, renal pelvis cancer, central nervous system tumor (CNS), primary CNS lymphoma, spinal cord tumor, glioblastoma, cerebral glioma, pituitary adenoma and squamous cell carcinoma; and is more preferably selected from the group consisting of breast cancer, prostate cancer, lung cancer, pancreatic cancer, ovarian cancer, cervical cancer, endometrial cancer, bladder cancer, cerebral glioma, B-cell lymphoma, liver cancer and leukemia, wherein the breast cancer is preferably ER⁺ breast cancer or ER⁺/HER2⁻ breast cancer; the lung cancer is preferably non-small cell lung cancer; the prostate cancer is preferably castration-resistant prostate cancer.
